# EUROPEAN PATENT SPECIFICATION

(11) **EP 3 823 958 B1**
(45) Date of publication and mention of the grant of the patent: **23.08.2023**
(21) Application number: 19742554.9
(22) Date of filing: 17.07.2019
(51) Int. Cl.: C07D 221/12, C07D 401/04, C07D 401/14, C07D 471/04, C07D 471/14, C07D 513/04, C09K 11/06

(54) **MATERIALS FOR ORGANIC ELECTROLUMINESCENT DEVICES**
MATERIALIEN FÜR ORGANISCHE ELEKTROLUMINESZENTE VORRICHTUNGEN
MATÉRIAUX POUR DISPOSITIFS ÉLECTROLUMINESCENTS ORGANIQUES

(30) Priority: 20.07.2018 EP 18184673
(43) Date of publication of application: 26.05.2021
(73) Proprietor: Merck Patent GmbH, 64293 Darmstadt (DE)
(72) Inventor: PARHAM, Amir Hossain, 60486 FRANKFURT AM MAIN (DE); KROEBER, Jonas Valentin, 60311 FRANKFURT AM MAIN (DE); ENGELHART, Jens, 64285 DARMSTADT (DE); JATSCH, Anja, 60489 FRANKFURT AM MAIN (DE); EICKHOFF, Christian, 68259 MANNHEIM (DE); EHRENREICH, Christian, 64285 DARMSTADT (DE)
(86) International application number: PCT/EP2019/069178
(87) International publication number: WO 2020/016264

(56) References cited:
- WO-A1-2018/099846
- WO-A1-2018/189134

## Description

The present invention relates to a compound of the formula (1), to the use of the compound in an electronic device, and to an electronic device comprising a compound of the formula (1). The present invention furthermore relates to a process for the preparation of a compound of the formula (1) and to a formulation comprising one or more compounds of the formula (1).

The structure of organic electroluminescent devices (OLEDs) in which organic semiconductors are employed as functional materials is described, for example in US 4539507. The emitting materials employed here are very often organometallic complexes which exhibit phosphorescence. For quantum-mechanical reasons, an up to four-fold increase in efficiency is possible using phosphorescent instead of fluorescent emitters. In general, however, there is still a need for improvement in the case of OLEDs, in particular also in the case of OLEDs which exhibit triplet emission (phosphorescence), for example with respect to efficiency, operating voltage and lifetime.

The properties of phosphorescent OLEDs are not only determined by the triplet emitters but also by the other materials used together with triplet emitters in OLEDs, such as matrix materials, also called host materials. Improvements in these materials and their charge-transport properties can thus also result in significant improvements in the OLED properties.

Thus, the choice of the matrix material in an emission layer comprising a phosphorescent emitter has a great influence on OLEDs properties, especially in terms of efficiency. The matrix material limits the quenching of excited states of emitter molecules by energy transfer.

Compounds comprising lactam derivatives and their use in OLEDs are known from the prior art (for example in WO 2011/137951 and WO 2013/064206).

Certain compounds containing lactam derivatives combined with triphenylene derivatives, as described in greater detail below, exhibit excellent properties when they are employed in OLEDs, particularly when employed as matrix material for phosphorescent emitters. Indeed, these compounds lead to OLEDs exhibiting better properties in terms of lifetime and/or efficiency and/or electroluminescent emission. In addition, these compounds have a high glass transition temperature and a good thermal stability, which is an important property for OLED materials, especially when the materials are vapor-deposited via a vacuum process. It has now been found that specific mixtures of compounds containing lactam derivatives combined with triphenylene derivatives and compounds as described below lead to particularly suitable mixtures for the fabrication of OLEDs.

The present invention therefore relates to electronic devices, in particular organic electroluminescent devices, which comprise specific mixtures of compounds containing lactam derivatives combined with triphenylene derivatives and compounds as described below.

The present invention relates to an organic electroluminescent device comprising a mixture comprising a compound of the formula (1), 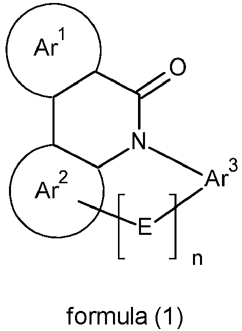
where the following applies to the symbols and indices used:
   - Ar¹: stands on each occurrence, identically or differently, for an aromatic or heteroaromatic ring system having 5 to 60 aromatic ring atoms, which may in each case be substituted by one or more radicals R¹;
   - Ar²: stands on each occurrence, identically or differently, for an aromatic or heteroaromatic ring system having 5 to 60 aromatic ring atoms, which may in each case be substituted by one or more radicals R²;
   - Ar³: stands on each occurrence, identically or differently, for an aromatic or heteroaromatic ring system having 5 to 60 aromatic ring atoms, which may in each case be substituted by one or more radicals R³;
   - -E-: is selected from a single bond, -B(R⁰)-, -C(R⁰)₂-, -Si(R⁰)₂-, -C(=O)-,-C(=NR⁰)-, -C(=C(R⁰)₂)-, -O-, -S-, -S(=O)-, -S(O₂)-, -N(R⁰)-, -P(R⁰)- and-P((=O)R⁰)-;
   - R⁰, R², R³: R¹, stand on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 40 aromatic ring atoms, which may be substituted by one or more radicals R, where two radicals R°, two radicals R¹, two radicals R² and/or two radicals R³ may form a monocyclic or polycyclic, aliphatic, aromatic or heteroaromatic ring system with one another, which may be substituted by one or more radicals R;
   - R: stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', a straight-chain alkyl, alkoxy or thioalkyl group having 1 to 40 C atoms or branched or cyclic alkyl, alkoxy or thioalkyl group having 3 to 40 C atoms, each of which may be substituted by one or more radicals R', where in each case one or more non-adjacent CH₂ groups may be replaced by R'C=CR' , C=C, Si(R')₂, Ge(R')₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring systems having 5 to 60 aromatic ring atoms, which may in each case be substituted by one or more radicals R , or an aryloxy group having 5 to 40 aromatic ring atoms, which may be substituted by one or more radicals R', where two radicals R may form a ring system with one another, which may be substituted by one or more radicals R';
   - Ar: is an aromatic or heteroaromatic ring system having 5 to 24 aromatic ring atoms, which may in each case also be substituted by one or more radicals R';
   - R': stands on each occurrence, identically or differently, for H, D, F, Cl, Br, I, CN, a straight-chain alkyl, alkoxy or thioalkyl group having 1 to 20 C atoms or branched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 C atoms;
   - n: is 0 or 1; wherein when n is 0, the group -E- is absent;
where the compound of formula (1) comprises at least
one group Ar³, R¹, R² or R³, which stands for a triphenylene derivative of the formula (T), 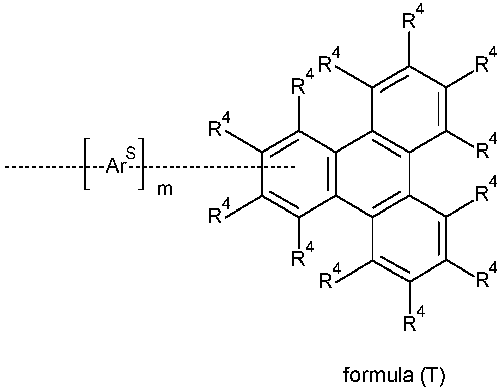
where the dashed bond indicates the bonding to the structure of formula (1); and where:
   - R⁴: is C at the position of the bonding to Ar^{s} or, R⁴ is C at the position of the bonding to the structure of formula (1) if m is 0; and at other positions, R⁴ stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 40 aromatic ring atoms, which may be substituted by one or more radicals R, where two radicals R⁴ may form a monocyclic or polycyclic, aliphatic, aromatic or heteroaromatic ring system, which may be substituted by one or more radicals R;
   - Ar^{s}: is an aromatic or heteroaromatic ring system having 5 to 60 aromatic ring atoms, which may in each case also be substituted by one or more radicals R;
   - m: is an integer selected from 0, 1, 2, 3 or 4,
and a second compound selected from compounds of formulae (11) and (12), 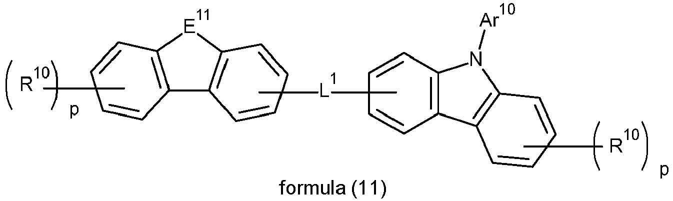 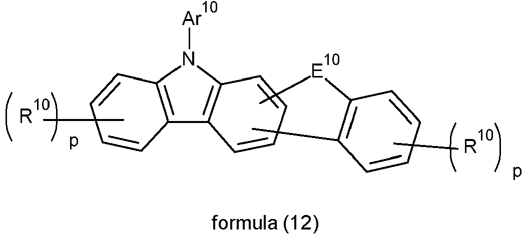
where:
   - R¹⁰: stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 40 aromatic ring atoms, which may be substituted by one or more radicals R, where two radicals R¹⁰ may form a monocyclic or polycyclic, aliphatic, aromatic or heteroaromatic ring system with one another, which may be substituted by one or more radicals R;
   - Ar¹⁰: is an aromatic or heteroaromatic ring system having 5 to 60 aromatic ring atoms, which may in each case also be substituted by one or more radicals R;
   - p: is an integer selected from 0, 1, 2, 3 and 4;
   - E¹⁰: stands for -C(R⁰)₂-, -Si(R⁰)₂-, -C(=O)-, -C(=NR⁰)-, -C(=C(R⁰)₂)-, -O-, -S-,-S(=O)-, -S(O₂)-, -N(Ar¹⁰)-, -P(R⁰)- or -P((=O)R⁰)-;
   - E¹¹: stands for-C(R⁰)₂-, -Si(R⁰)₂-, -C(=O)-, -C(=NR⁰)-, -C(=C(R⁰)₂)-, -O-, -S-,-S(=O)-, -S(O₂)-, -N(Ar¹⁰)-, -P(R⁰)- or -P((=O)R⁰)-; and
   - L¹: is a single bond or an aromatic or heteroaromatic ring system having 5 to 60 aromatic ring atoms, which may in each case also be substituted by one or more radicals R,
where the mixture is employed as a matrix material for phosphorescent emitters in a light-emitting layer; and
where the following compounds are excluded from the compounds of formula (1):

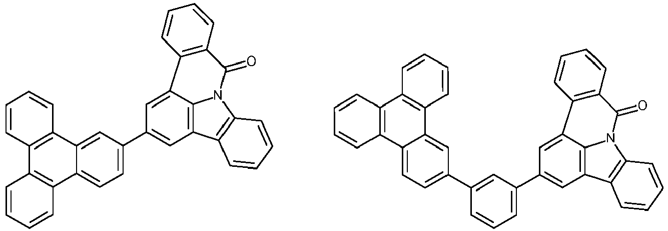

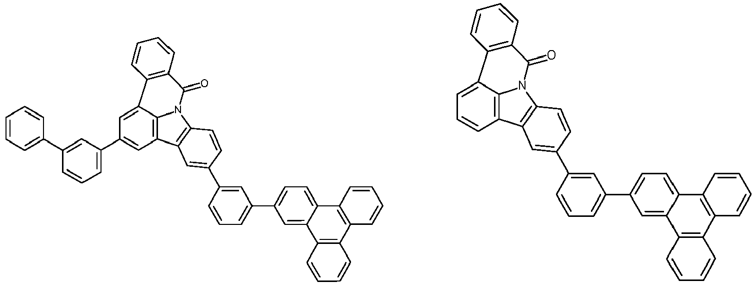

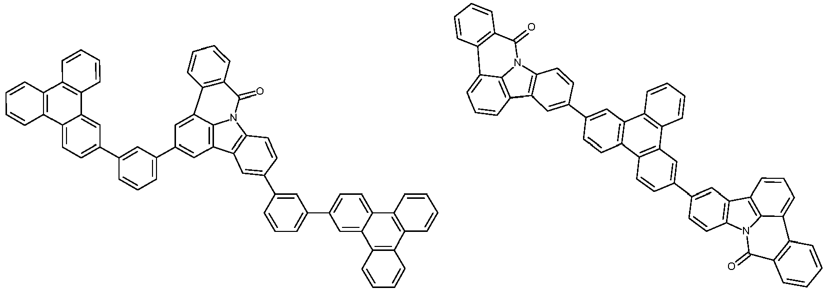

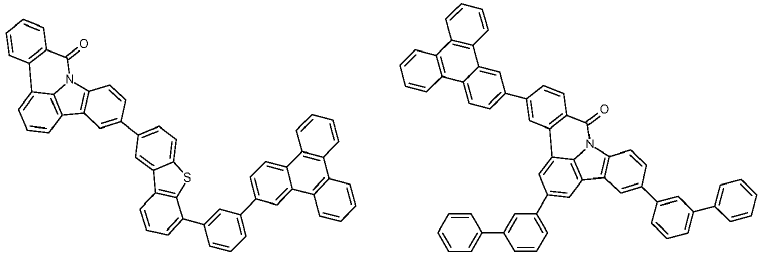

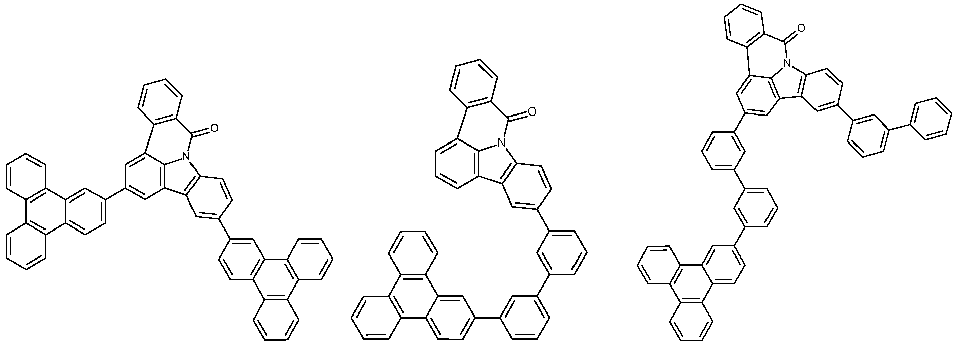

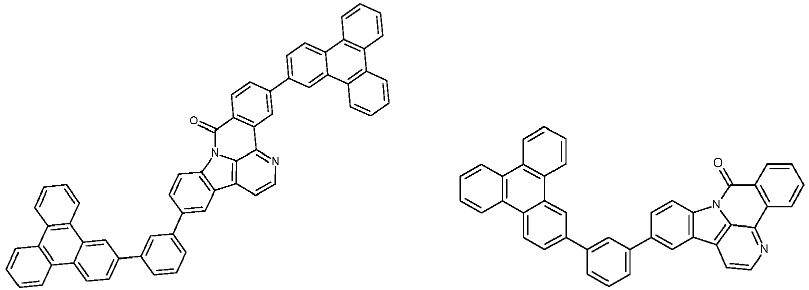

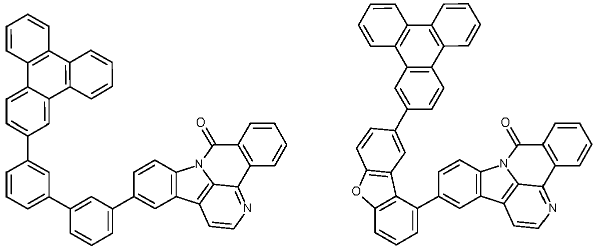

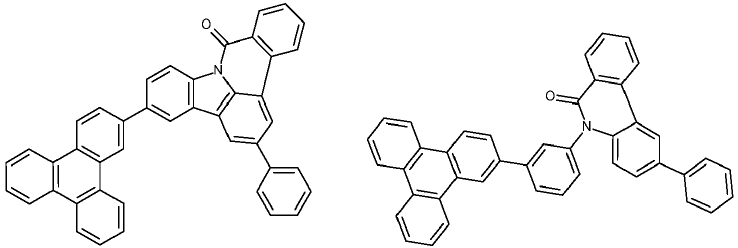

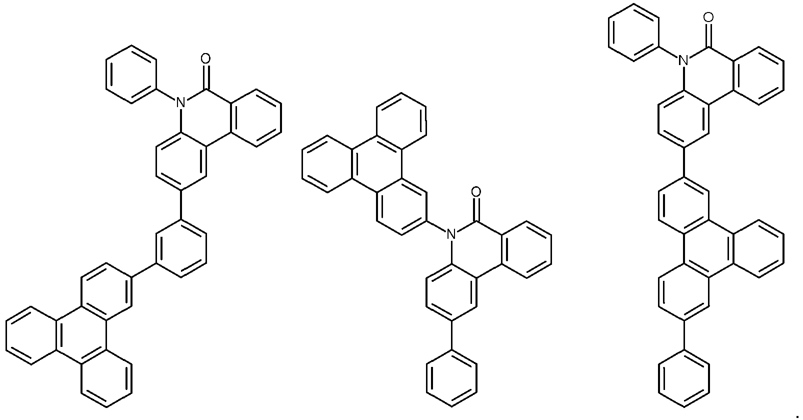

Adjacent substituents in the sense of the present invention are substituents which are bonded to carbon atoms which are linked directly to one another or which are bonded to the same carbon atom.

Furthermore, the following definitions of chemical groups apply for the purposes of the present application:
An aryl group in the sense of this invention contains 6 to 60 aromatic ring atoms; a heteroaryl group in the sense of this invention contains 5 to 60 aromatic ring atoms, at least one of which is a heteroatom. The hetero atoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups.

An aromatic ring system in the sense of this invention contains 6 to 60 C atoms in the ring system. A heteroaromatic ring system in the sense of this invention contains 5 to 60 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5 - 60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two radicals may form a ring with one another is, for the purposes of the present application, intended to be taken to mean, inter alia, that the two radicals are linked to one another by a chemical bond. This is illustrated by the following schemes:

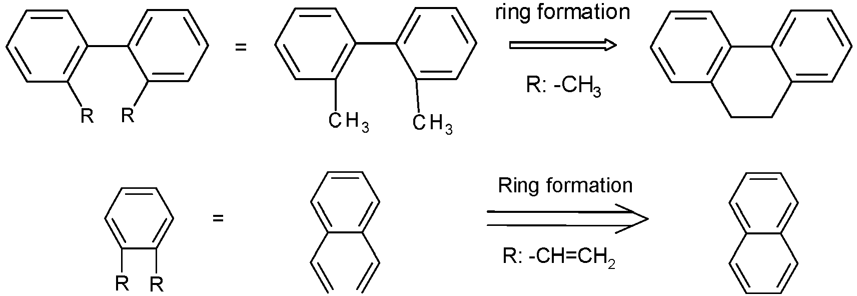

Furthermore, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

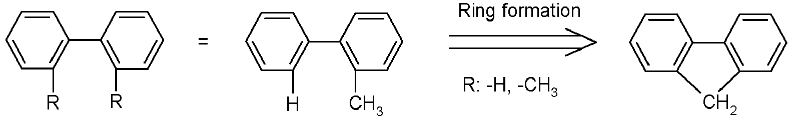

Preferably, the compounds of formula (1) are selected from the compounds of formulae (1A) or (1B), 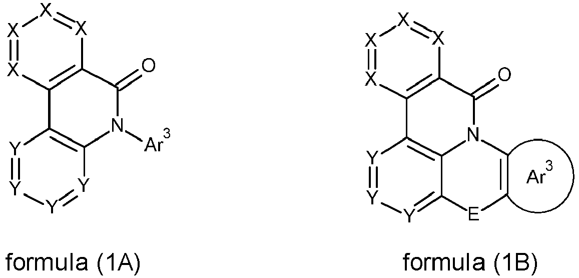 where
the symbol -E- has the same meaning as above;
X is CR¹ or N;
Y is CR² or N;
where the symbols R¹, R² have the same meaning as above; and
Ar³ has the same meaning as above, with the proviso that Ar³ in formula (1B) is connected to the group -E- and the atom N via two adjacent C atoms as represented in formula (1B);
characterized in that, in the compounds of formula (1A), at least one group Ar³, R¹ or R² stands for a triphenylene derivative of the formula (T) and, in the compounds of formula (1B), at least one group R¹ or R² stands for a triphenylene derivative of the formula (T).

In accordance with the invention, when the group -E- is a single bond, then the compounds of formula (1B) correspond to compounds of formula (1B-E), 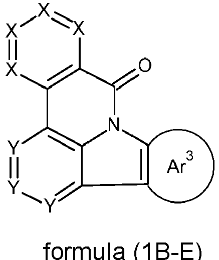 where the symbols have the same meaning as above.

More preferably, the compounds of formula (1) are selected from the compounds of formulae (2), (3), (4), (5), (6) or (7), 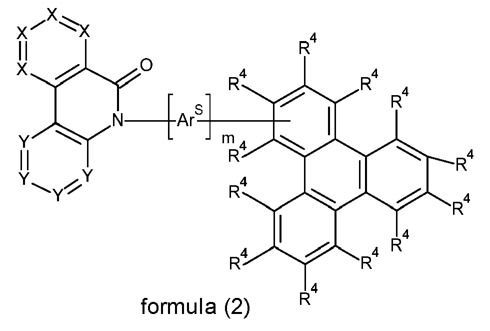 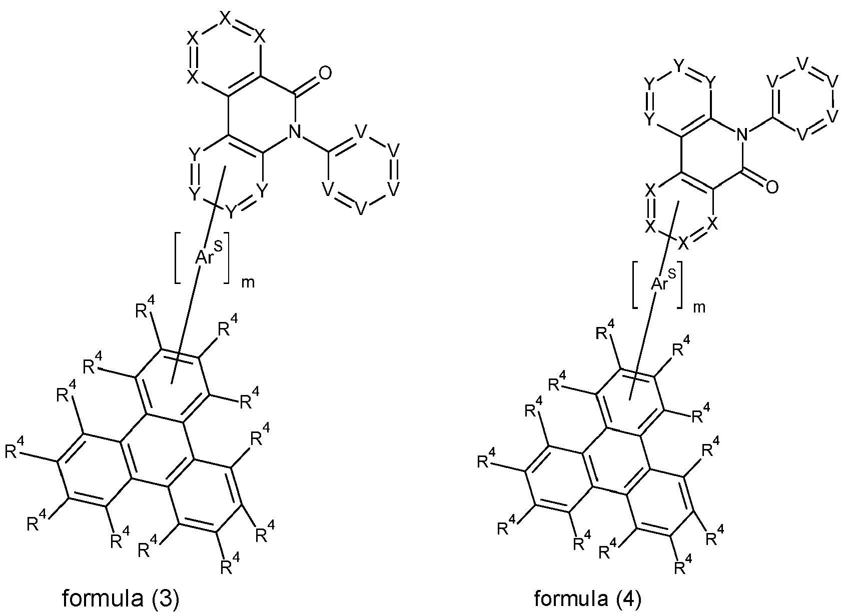 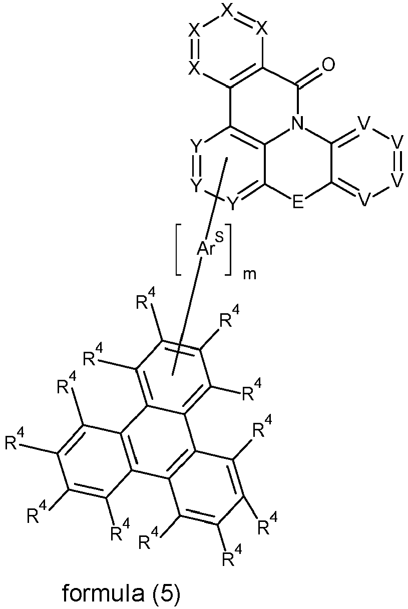 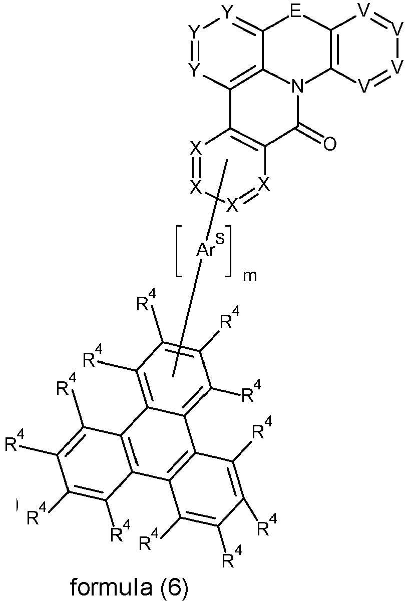 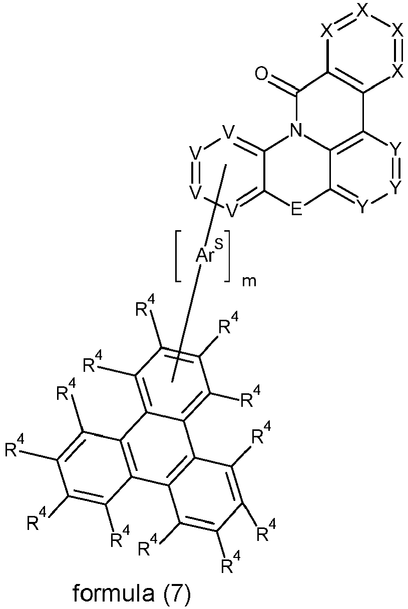 where the symbols and indices E, R⁴, Ar^{S} and m have the same meaning as above; and where
- X: is CR¹ or N, with the proviso that X is C at the position of the bonding to Ar^{s} or, if m is 0 and Ar^{s} is absent, to the triphenylene group in formulae (4) and (6);
- Y: is CR² or N, with the proviso that Y is C at the position of the bonding to Ar^{s} or, if m is 0 and Ar^{s} is absent, to the triphenylene group in formulae (3) and (5);
- V: is CR³ or N, with the proviso that V is C at the position of the bonding to Ar^{S} or, if m is 0 and Ar^{S} is absent, to the triphenylene group in formula (7); or two adjacent groups V stand for a group of formula (V-1) or (V-2), 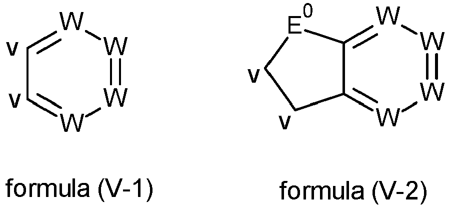 where the symbols ^{v} indicate the corresponding adjacent groups V in formulae (2) to (7);
- E°: stands for-C(R⁰)₂-, -Si(R⁰)₂-, -C(=O)-, -C(=NR⁰)-, -C(=C(R⁰)₂)-, -O-, -S-,-S(=O)-, -S(O₂)-, -N(R⁰)-, -P(R⁰)- or -P((=O)R⁰)-, preferably for -C(R⁰)₂-,-O-, -S-, or -N(R⁰)-; where R⁰ has the same meaning as above;
- W: stands for CR or N;

In accordance with a preferred embodiment, the index m is equal to 0 or 1. When m is 0, then the group Ar^{s} is absent and the group -(Ar^{S})₀- corresponds to a single bond.

In accordance with a preferred embodiment, there is 0 or 1 group X standing for N in the six-membered ring comprising the groups X, there is 0 or 1 group Y standing for N in the six-membered ring comprising the groups Y, and there is 0 or 1 group V in the six-membered ring comprising the groups V.

Particularly preferably, the compounds of formula (1) are selected from the compounds of formulae (2A) to (7B), 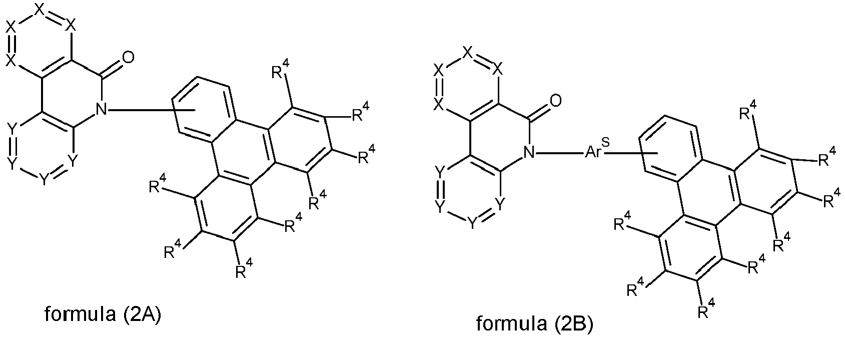 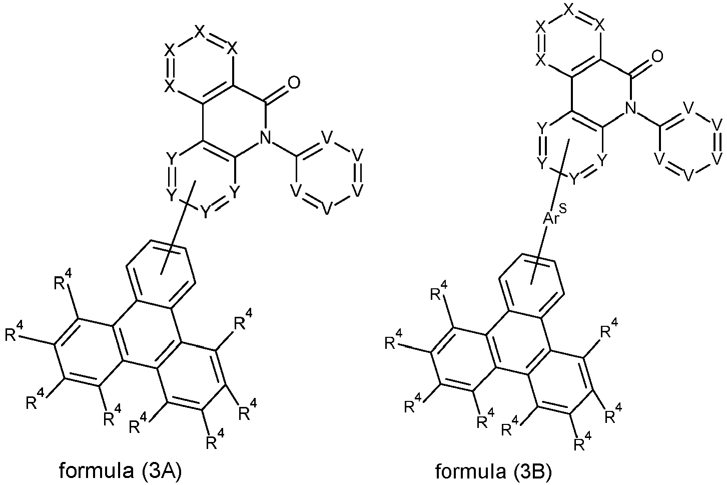 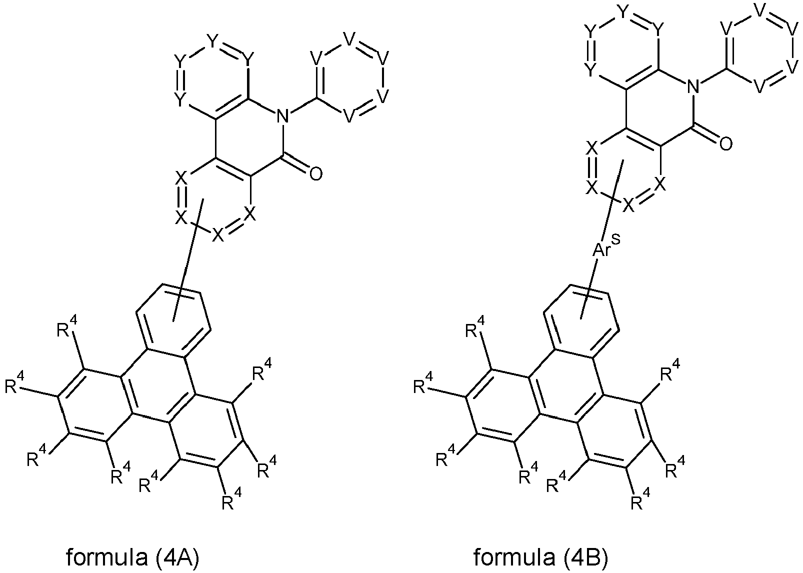 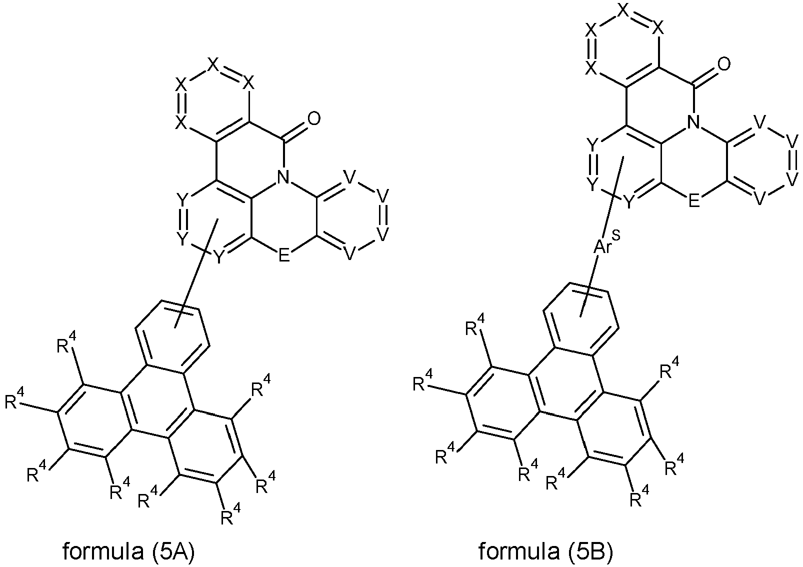 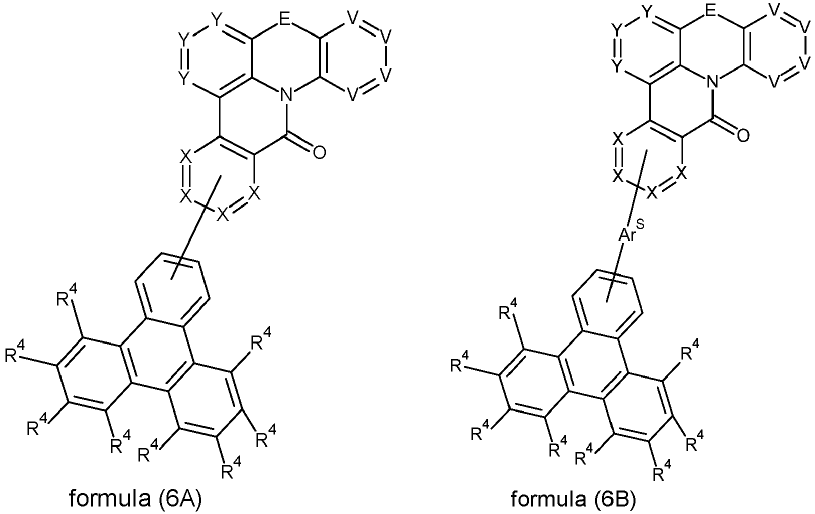 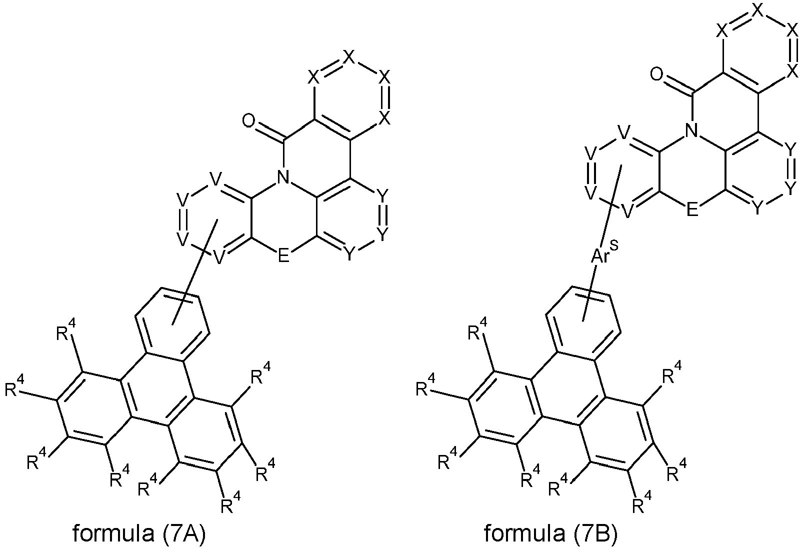 where the symbols -E-, Ar^{S}, R⁴, X, Y and V have the same meaning as above.

Very particularly preferably, the compounds of formula (1) are selected from the compounds of formulae (2A-1) to (7B-1), 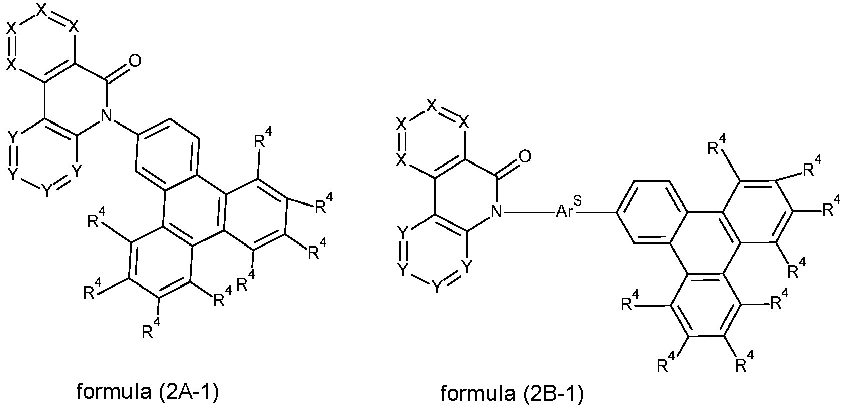 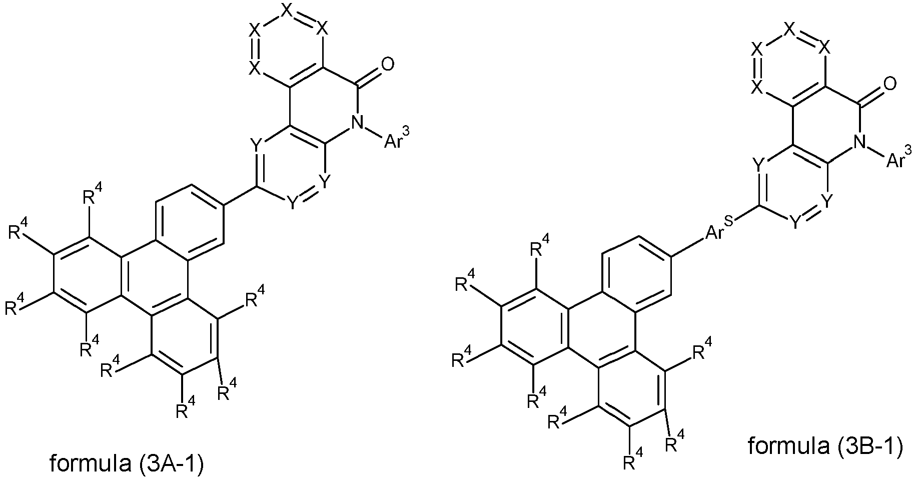 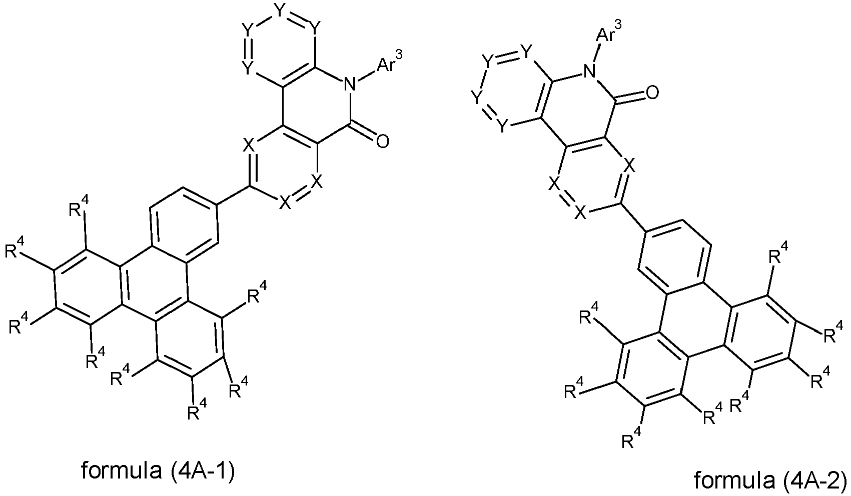 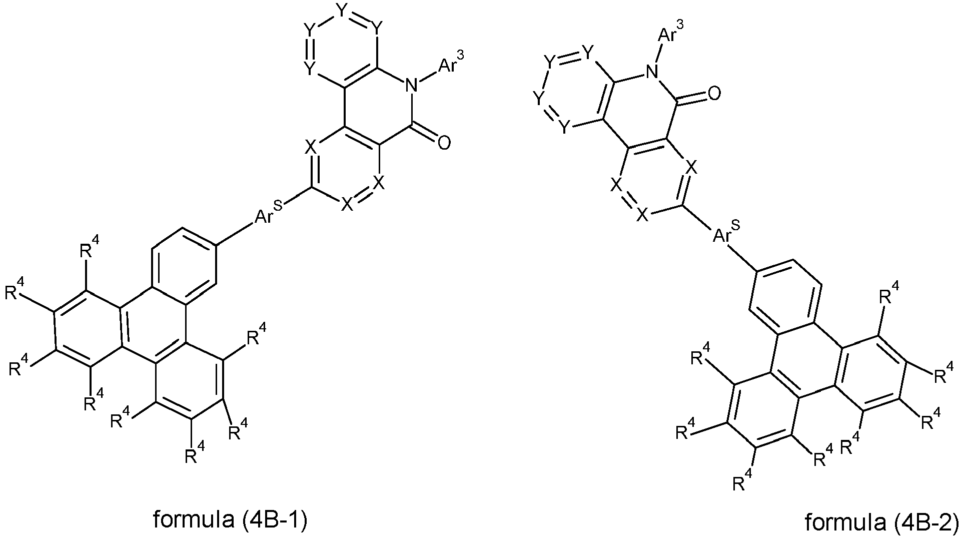 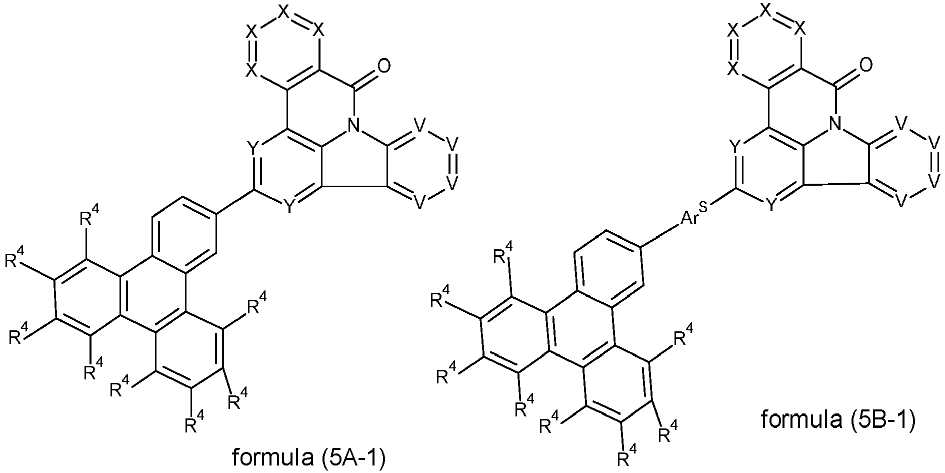 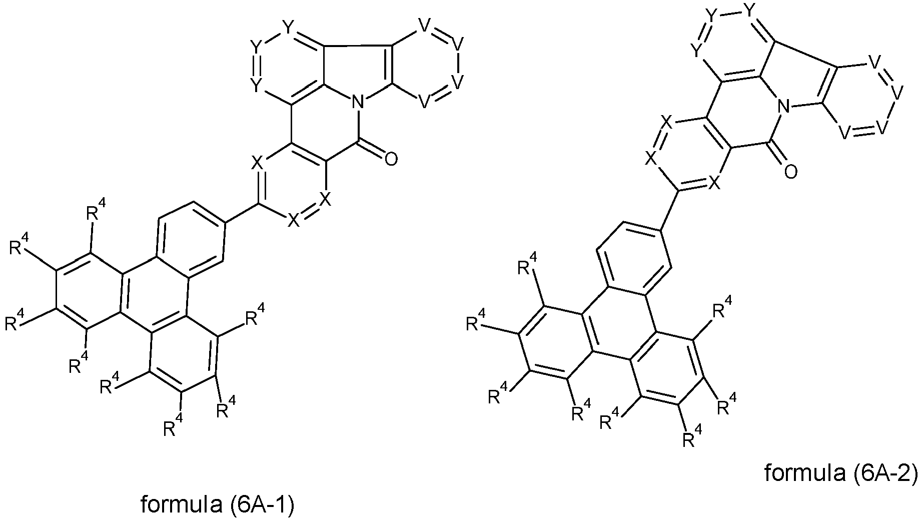 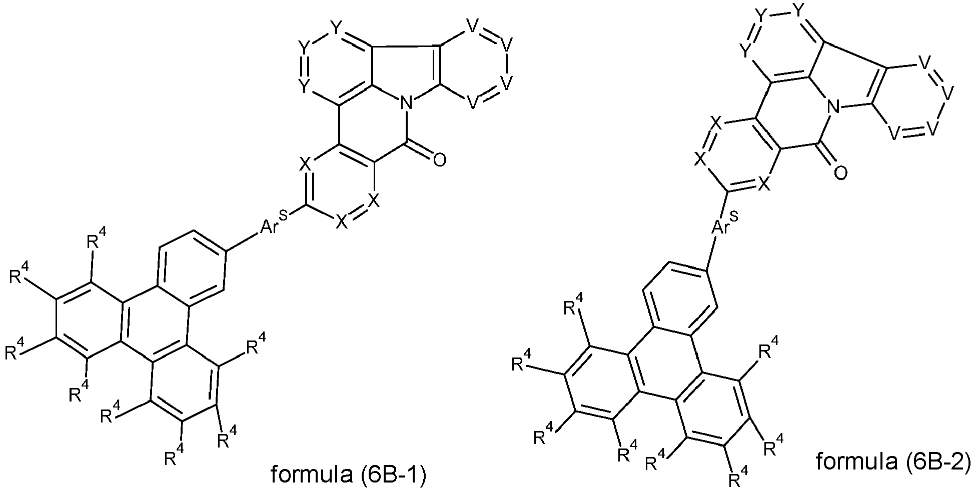 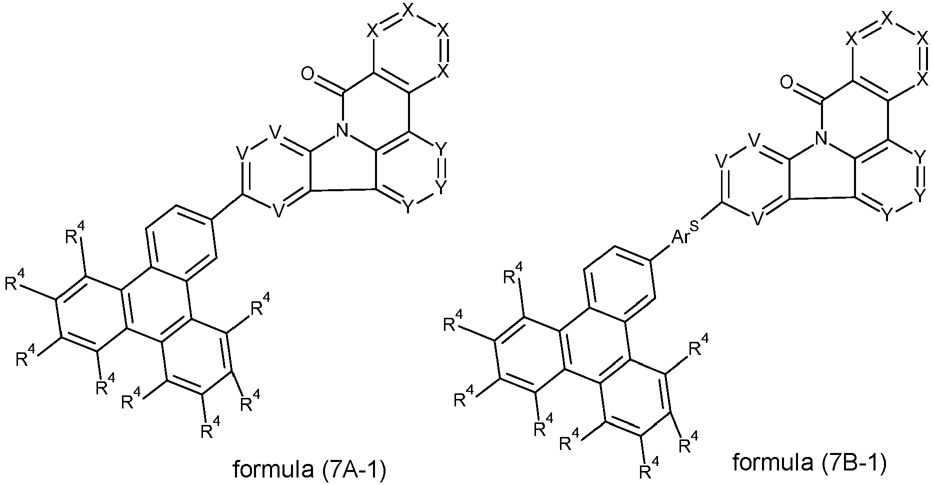 where the symbols E, X, Y and V have the same meaning as above.

In accordance with a preferred embodiment, -E- is selected from a single bond, -C(R⁰)₂-, -O-, -S- and -N(R⁰)-. More preferably, -E- is selected from a single bond, -O- or -S-. Particularly preferably, -E- is a single bond.

Preferably, Ar^{S} is an aromatic or heteroaromatic ring system having 5 to 40, more preferably 5 to 30, particularly preferably 5 to 18 aromatic ring atoms, which may in each case also be substituted by one or more radicals R; and

More preferably, the group Ar^{S} stands on each occurrence, identically or differently, for phenyl, biphenyl, fluorene, spirobifluorene, naphthalene, phenanthrene, anthracene, dibenzofuran, dibenzothiophene, carbazole, pyridine, pyrimidine, pyrazine, pyridazine, triazine, benzopyridine, benzopyridazine, benzopyrimidine and quinazoline, each of which may be substituted by one or more radicals R.

In accordance with a very preferred embodiment, the group Ar^{S} stands on each occurrence, identically or differently, for phenyl, biphenyl, fluorene, naphthalene, dibenzofuran, dibenzothiophene and carbazole, each of which may be substituted by one or more radicals R.

Examples of suitable groups Ar^{S} are the groups (Ar^{S}-1) to (Ar^{S}-22) depicted in the table below:

| | | |
|---|---|---|
| 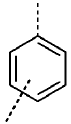 | 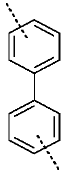 | 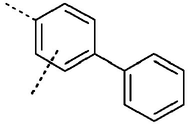 |
| Ar^{S}-1 | Ar^{S}-2 | Ar^{S}-3 |
| 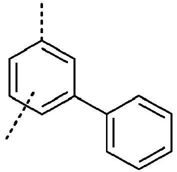 | 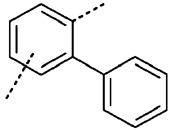 | 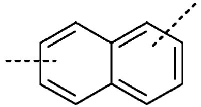 |
| Ar^{S}-4 | Ar^{S}-5 | Ar^{S}-6 |
| 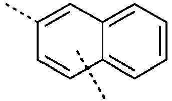 | 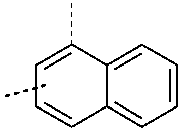 | 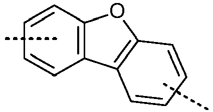 |
| Ar^{S}-7 | Ar^{S}-8 | Ar^{S}-9 |
| 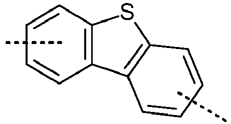 | 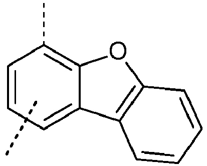 | 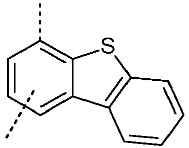 |
| Ar^{S}-10 | Ar^{s}-11 | Ar^{S}-12 |
| 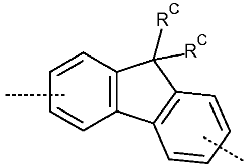 | 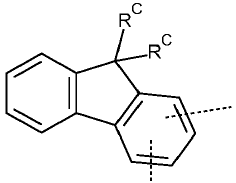 | 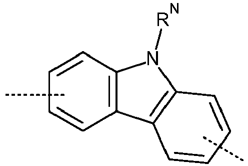 |
| Ar^{S}-13 | Ar^{S}-14 | Ar^{S}-15 |
| 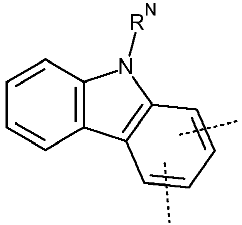 | 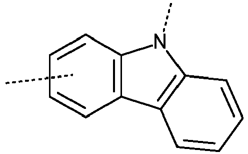 | 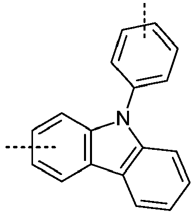 |
| Ar^{S}-16 | Ar^{S}-17 | Ar^{S}-18 |
| 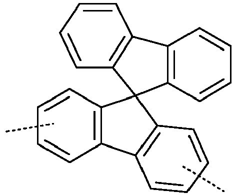 | 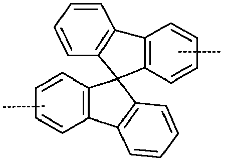 | 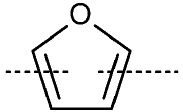 |
| Ar^{S}-19 | Ar^{S}-20 | Ar^{S}-21 |
| 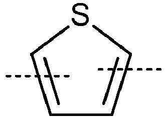 | | |
| Ar^{S}-22 | | |

where the dashed bonds indicate the bonds to the structure of formula (1) and to the triphenylene derivative, and where the groups (Ar^{S}-1) to (Ar^{S}-22) may be substituted at each free position by a group R and where:
- R^{N}, R^{C}: in formulae (Ar^{S}-13) to (Ar^{S}-16), are on each occurrence, identically or differently, H, D, F, Cl, Br, I, CN, a straight-chain alkyl, alkoxy or thioalkoxy group having 1 to 40, preferably 1 to 20, more preferably 1 to 10 C atoms or cyclic alkyl, alkoxy or thioalkoxy group having 3 to 40, preferably 3 to 20, more preferably 3 to 10 C atoms, each of which may be substituted by one or more radicals R, where one or more non-adjacent CH₂ groups may be replaced by (R)C=C(R), C=C, O or S and where one or more H atoms may be replaced by D, F, Cl, Br, I, CN or NO₂, or an aromatic or heteroaromatic ring system having 5 to 60, preferably 5 to 40, more preferably 5 to 30, very more preferably 5 to 18 aromatic ring atoms, which may in each case be substituted by one or more radicals R, where optionally two adjacent substituents R^{C} can form a mono- or polycyclic, aliphatic, aromatic or heteroaromatic ring system with one another.

Examples of very suitable groups Ar^{S} are the groups (Ar^{S}-23) to (Ar^{S}-67) depicted in the table below:

| | | |
|---|---|---|
| 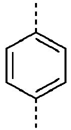 | 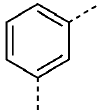 | 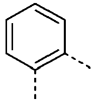 |
| Ar^{S}-23 | Ar^{S}-24 | Ar^{S}-25 |
| 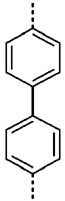 | 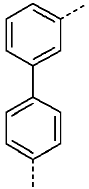 | 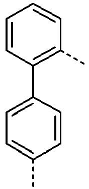 |
| Ar^{S}-26 | Ar^{S}-27 | Ar^{S}-28 |
| 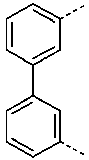 | 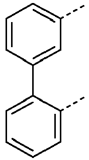 | 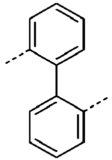 |
| Ar^{S}-29 | Ar^{S}-30 | Ar^{S}-31 |
| 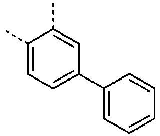 | 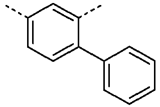 | 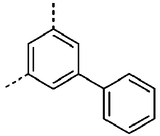 |
| Ar^{S}-32 | Ar^{S}-33 | Ar^{S}-34 |
| 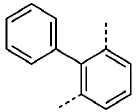 | 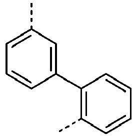 | 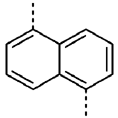 |
| Ar^{S}-35 | Ar^{S}-36 | Ar^{S}-37 |
| 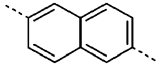 | 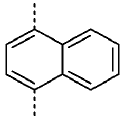 | 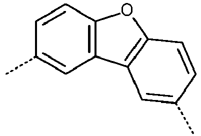 |
| Ar^{S}-38 | Ar^{S}-39 | Ar^{S}-40 |
| 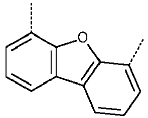 | 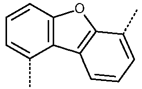 | 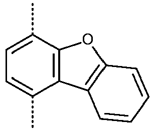 |
| Ar^{s}41 | Ar^{S}-42 | Ar^{S}-43 |
| 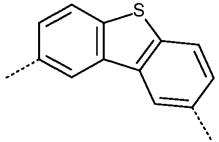 | 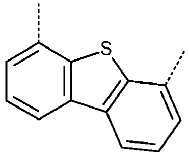 | 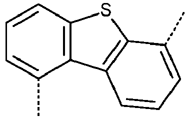 |
| Ar^{S}-44 | Ar^{S}-45 | Ar^{S}-46 |
| 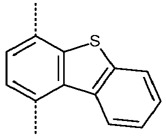 | 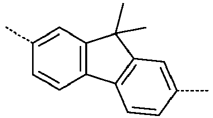 | 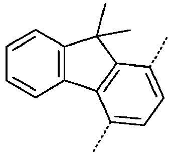 |
| Ar^{S}-47 | Ar^{S}-48 | Ar^{S}-49 |
| 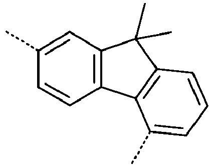 | 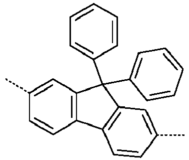 | 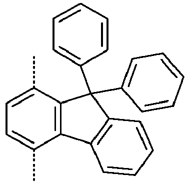 |
| Ar^{s}-50 | Ar^{s}-51 | Ar^{s}-52 |
| 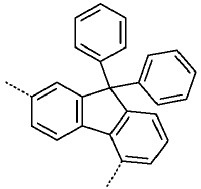 | 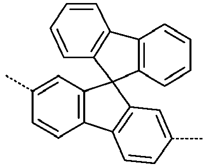 | 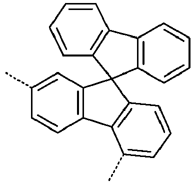 |
| Ar^{S}-53 | Ar^{S}-54 | Ar^{S}-55 |
| 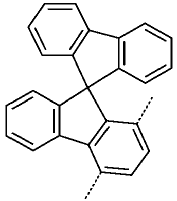 | 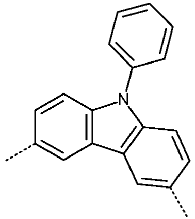 | 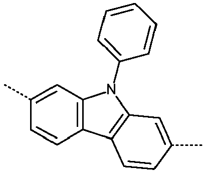 |
| Ar^{S}-56 | Ar^{S}-57 | Ar^{S}-58 |
| 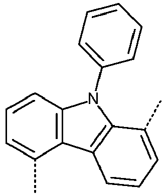 | 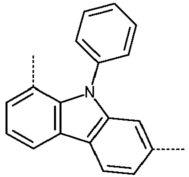 | 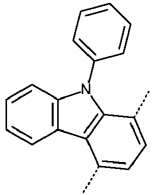 |
| Ar^{S}-59 | Ar^{S}-60 | Ar^{S}-61 |
| 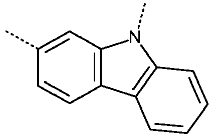 | 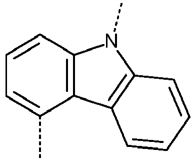 | 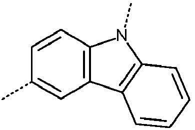 |
| Ar^{S}-62 | Ar^{S}-63 | Ar^{S}-64 |
| 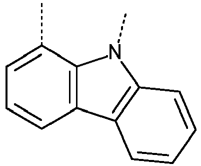 | 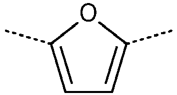 | 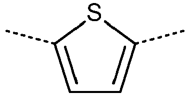 |
| Ar^{S}-65 | Ar^{S}-66 | Ar^{S}-67 |

where the dashed bonds indicate the bonds to the structure of formula (1) and to the triphenylene derivative and where the groups (Ar^{S}-23) to (Ar^{S}-67) may be substituted at each free position by a group R.

Preferably, R⁰ stands on each occurrence, identically or differently, for H, D, F, CN, a straight-chain alkyl group having 1 to 20 C atoms or branched or a cyclic alkyl group having 3 to 20 C atoms, each of which may be substituted by one or more radicals R, where in each case one or more non-adjacent CH₂ groups may be replaced by RC=CR, C=C, O or S and where one or more H atoms may be replaced by D, F, Cl, Br, I, CN or NO₂, an aromatic or heteroaromatic ring systems having 5 to 40, preferably 5 to 30, more preferably 5 to 18 aromatic ring atoms, which may in each case be substituted by one or more radicals R, or an aryloxy group having 5 to 40, preferably 5 to 30, more preferably 5 to 18 aromatic ring atoms, which may be substituted by one or more radicals R, where two radicals R° may form a monocyclic or polycyclic, aliphatic, aromatic or heteroaromatic ring system with one another, which may be substituted by one or more radicals R. More preferably, R° stands on each occurrence, identically or differently, for H, D, a straight-chain alkyl group having 1 to 10 C atoms or branched or a cyclic alkyl group having 3 to 10 C atoms, each of which may be substituted by one or more radicals R, an aromatic or heteroaromatic ring systems having 5 to 18 aromatic ring atoms, which may in each case be substituted by one or more radicals R, where two radicals R° may form a monocyclic or polycyclic, aliphatic, aromatic or heteroaromatic ring system with one another, which may be substituted by one or more radicals R.

Preferably, R¹, R², R³ stand on each occurrence, identically or differently, for H, D, F, CN, Si(R)₃, a straight-chain alkyl or alkoxy group having 1 to 20 C atoms or branched or a cyclic alkyl or alkoxy group having 3 to 20 C atoms, each of which may be substituted by one or more radicals R, where in each case one or more non-adjacent CH₂ groups may be replaced by RC=CR, C=C, O or S and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 40 aromatic ring atoms, which may be substituted by one or more radicals R, where two radicals R¹, two radicals R² and/or two radicals R³ may form a monocyclic or polycyclic, aliphatic, aromatic or heteroaromatic ring system with one another, which may be substituted by one or more radicals R. More preferably, R¹, R², R³ stand on each occurrence, identically or differently, for H, D, F, CN, a straight-chain alkyl group having 1 to 10 C atoms or branched or a cyclic alkyl group having 3 to 10 C atoms, each of which may be substituted by one or more radicals R, or an aromatic or heteroaromatic ring systems having 5 to 60, preferably 5 to 40, more preferably 5 to 30, very more preferably 5 to 18 aromatic ring atoms, which may in each case be substituted by one or more radicals R, where two radicals R¹, two radicals R² and/or two radicals R³ may form a monocyclic or polycyclic, aliphatic, aromatic or heteroaromatic ring system with one another, which may be substituted by one or more radicals R.

When the groups R¹, R² or R³ stands for an aromatic or heteroaromatic ring system having 5 to 60 aromatic ring atoms, then the aromatic or heteroaromatic ring system is preferably selected from benzene, naphthalene, anthracene, benzanthracene, phenanthrene, triphenylene (also called benzophenanthrene), pyrene, chrysene, perylene, fluoranthene, naphthacene, pentacene, benzopyrene, biphenyl, biphenylene, terphenyl, terphenylene, quaterphenyl, fluorene, spirobifluorene, indenofluorene, furan, benzofuran, dibenzofuran, thiophene, benzothiophene, dibenzothiophene, pyrrole, indole, isoindole, carbazole, indolocarbazole, indenocarbazole, pyridine, quinoline, isoquinoline, acridine, phenanthridine, benzoquinoline, phenothiazine, phenoxazine, imidazole, benzimidazole, naphthimidazole, phenanthrimidazole, pyrimidine, benzopyrimidine, quinoxaline, pyrazine, phenazine, phenoxazine, phenothiazine, azacarbazole, triazine, or combinations of these groups. More preferably, when the groups R¹, R² or R³ stands for an aromatic or heteroaromatic ring system having 5 to 60 aromatic ring atoms, then the aromatic or heteroaromatic ring system is selected from benzene, naphthalene, phenanthrene, triphenylene, fluoranthene, biphenyl, terphenyl, quaterphenyl, fluorene, spirobifluorene, indenofluorene, dibenzofuran, dibenzothiophene, carbazole, indolocarbazole, indenocarbazole, pyridine, quinoline, benzoquinoline, pyrimidine, benzopyrimidine, quinoxaline, phenoxazine, phenothiazine, azacarbazole, triazine, or combinations of these groups.

Preferably, R⁴ stands on each occurrence, identically or differently, for H, D, F, Si(R)₃, a straight-chain alkyl or alkoxy group having 1 to 20 C atoms or branched or a cyclic alkyl or alkoxy group having 3 to 20 C atoms, each of which may be substituted by one or more radicals R, where in each case one or more non-adjacent CH₂ groups may be replaced by RC=CR, C=C, O or S and where one or more H atoms may be replaced by D, F, Cl, Br, I, CN or NO₂, an aromatic or heteroaromatic ring systems having 5 to 60, preferably 5 to 40, more preferably 5 to 30, very more preferably 5 to 18 aromatic ring atoms, which may in each case be substituted by one or more radicals R, where two radicals R⁴may form a monocyclic or polycyclic, aliphatic, aromatic or heteroaromatic ring system, which may be substituted by one or more radicals R. More preferably, R⁴ stands on each occurrence, identically or differently, for H, D, F, a straight-chain alkyl group having 1 to 10 C atoms or branched or a cyclic alkyl group having 3 to 10 C atoms, each of which may be substituted by one or more radicals R, an aromatic or heteroaromatic ring systems having 5 to 60, preferably 5 to 40, more preferably 5 to 30, very more preferably 5 to 18 aromatic ring atoms, which may in each case be substituted by one or more radicals R, where two radicals R⁴ may form a monocyclic or polycyclic, aliphatic, aromatic or heteroaromatic ring system, which may be substituted by one or more radicals R. Particularly preferably, R⁴ stands on each occurrence, identically or differently, for H, D, a straight-chain alkyl group having 1 to 10 C atoms or a branched or a cyclic alkyl group having 3 to 10 C atoms, each of which may be substituted by one or more radicals R, or an aromatic or heteroaromatic ring systems having 5 to 18 aromatic ring atoms, which may in each case be substituted by one or more radicals R.

Preferably, R stands on each occurrence, identically or differently, for H, D, F, CN, a straight-chain alkyl or alkoxy group having 1 to 20 C atoms or branched or cyclic alkyl or alkoxy group having 3 to 20 C atoms, each of which may be substituted by one or more radicals R', an aromatic or heteroaromatic ring systems having 5 to 40, preferably 5 to 30, more preferably 5 to 18 aromatic ring atoms, which may in each case be substituted by one or more radicals R', where two radicals R may form a ring system with one another, which may be substituted by one or more radicals R'. More preferably, R stands on each occurrence, identically or differently, for H, D, F, CN, a straight-chain alkyl group having 1 to 10 C atoms or branched or cyclic alkyl group having 3 to 10 C atoms, each of which may be substituted by one or more radicals R', an aromatic or heteroaromatic ring systems having 5 to 18 aromatic ring atoms, which may in each case be substituted by one or more radicals R.

Preferably, Ar is an aromatic or heteroaromatic ring system having 5 to 18 aromatic ring atoms, which may in each case also be substituted by one or more radicals R'.

Preferably, R'stands on each occurrence, identically or differently, for H, D, F, Cl, Br, I, CN, a straight-chain alkyl or alkoxy group having 1 to 20, preferably 1 to 10, more preferably 1 to 5 C atoms or branched or cyclic alkyl or alkoxy group having 3 to 20, preferably 1 to 10, more preferably 1 to 5 C atoms, where one or more H atoms may be replaced by D, F, Cl, Br or I, or an aromatic or heteroaromatic ring system having 5 to 24 C, preferably 5 to 18 C atoms.

In accordance with a preferred embodiment, the compounds of formula (1) comprise at least one substituent R¹ or R², which is selected from aromatic and heteroaromatic ring systems.

Examples of suitable compounds of formula (1) are the structures shown in the table below:

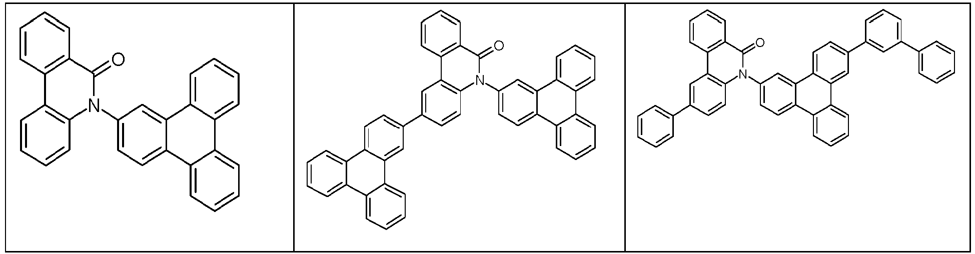

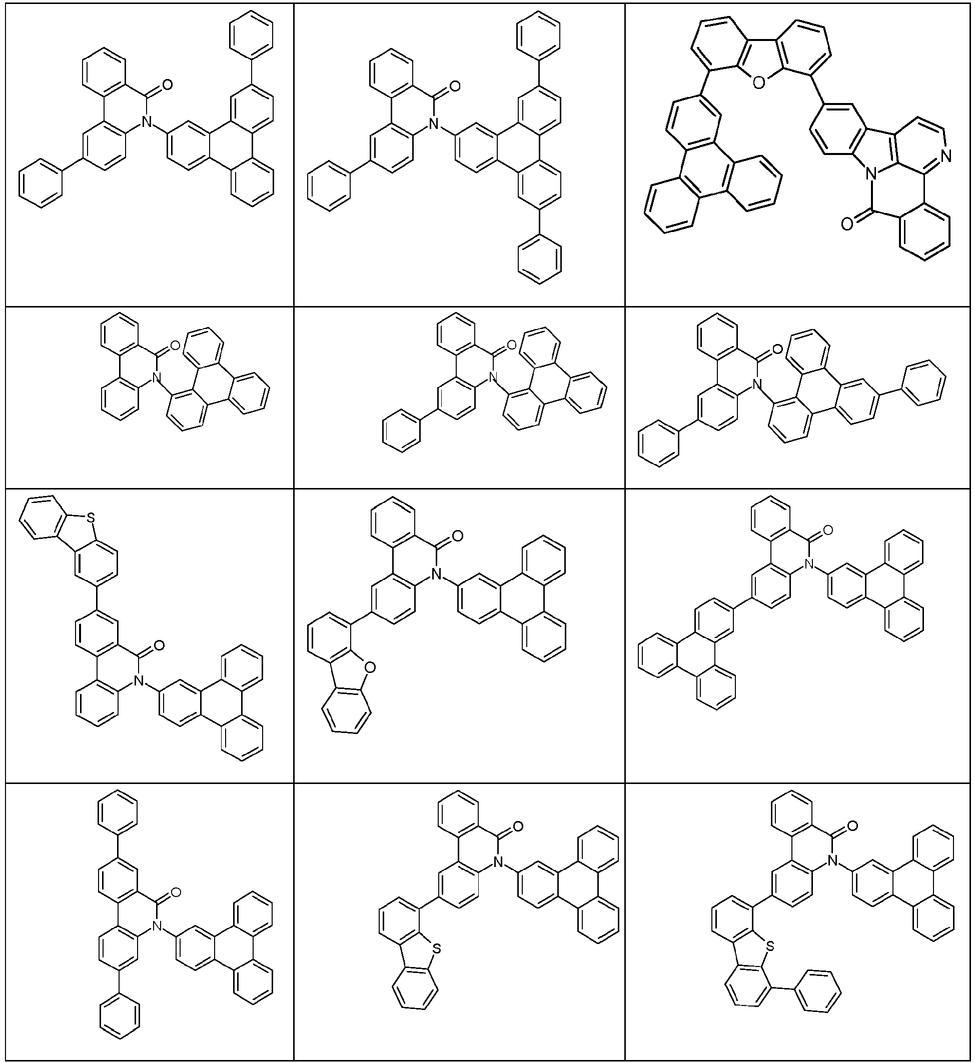

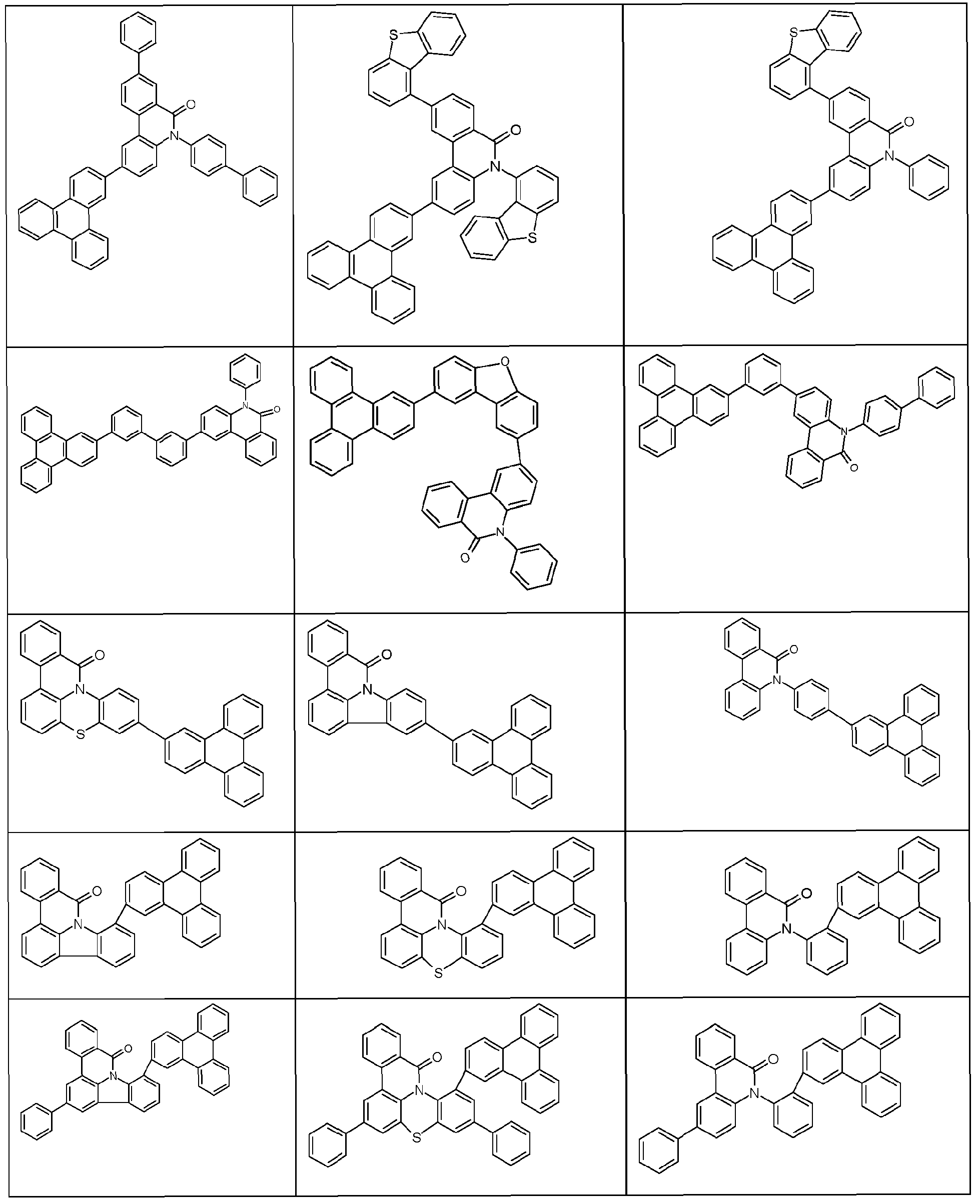

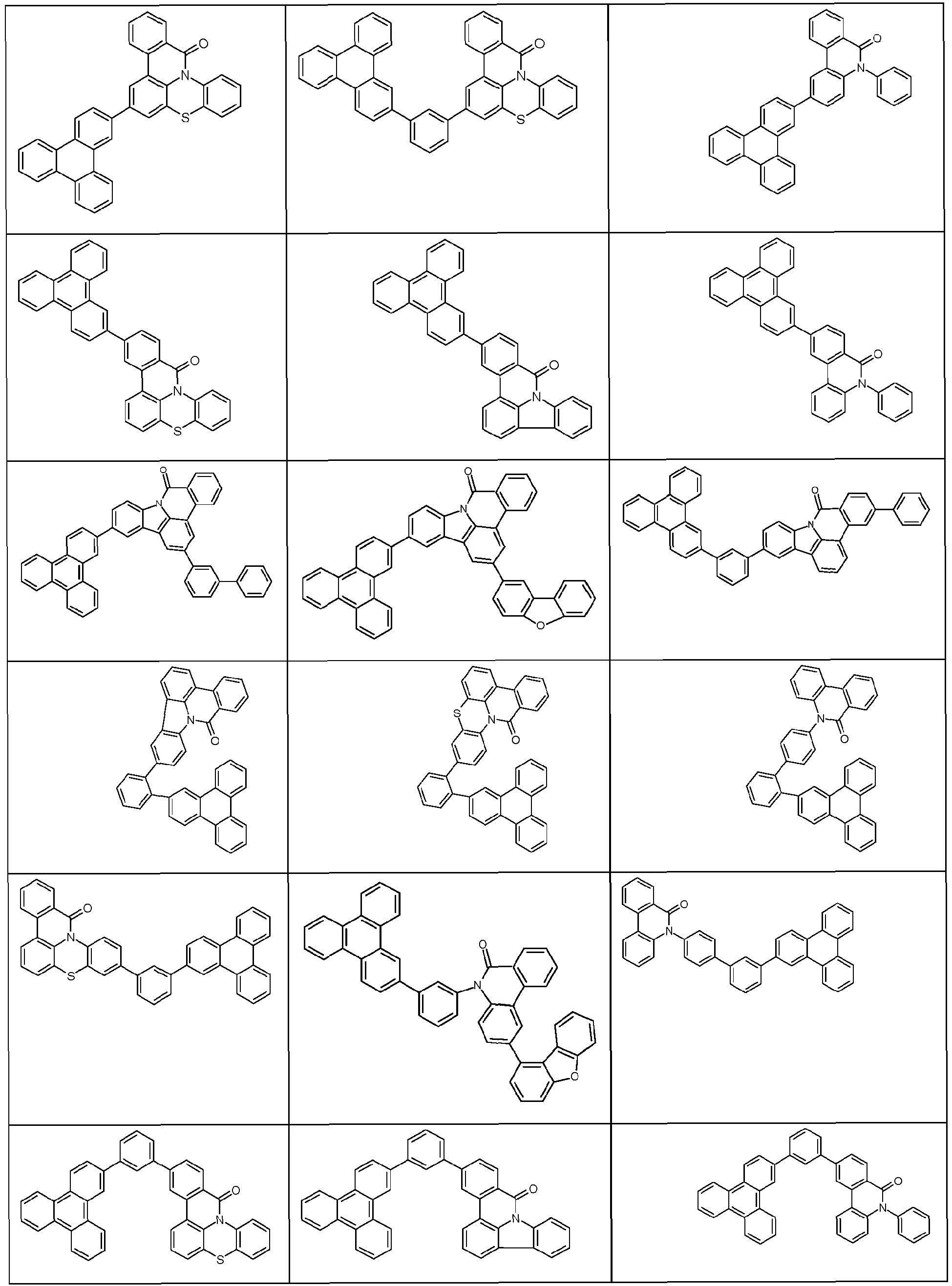

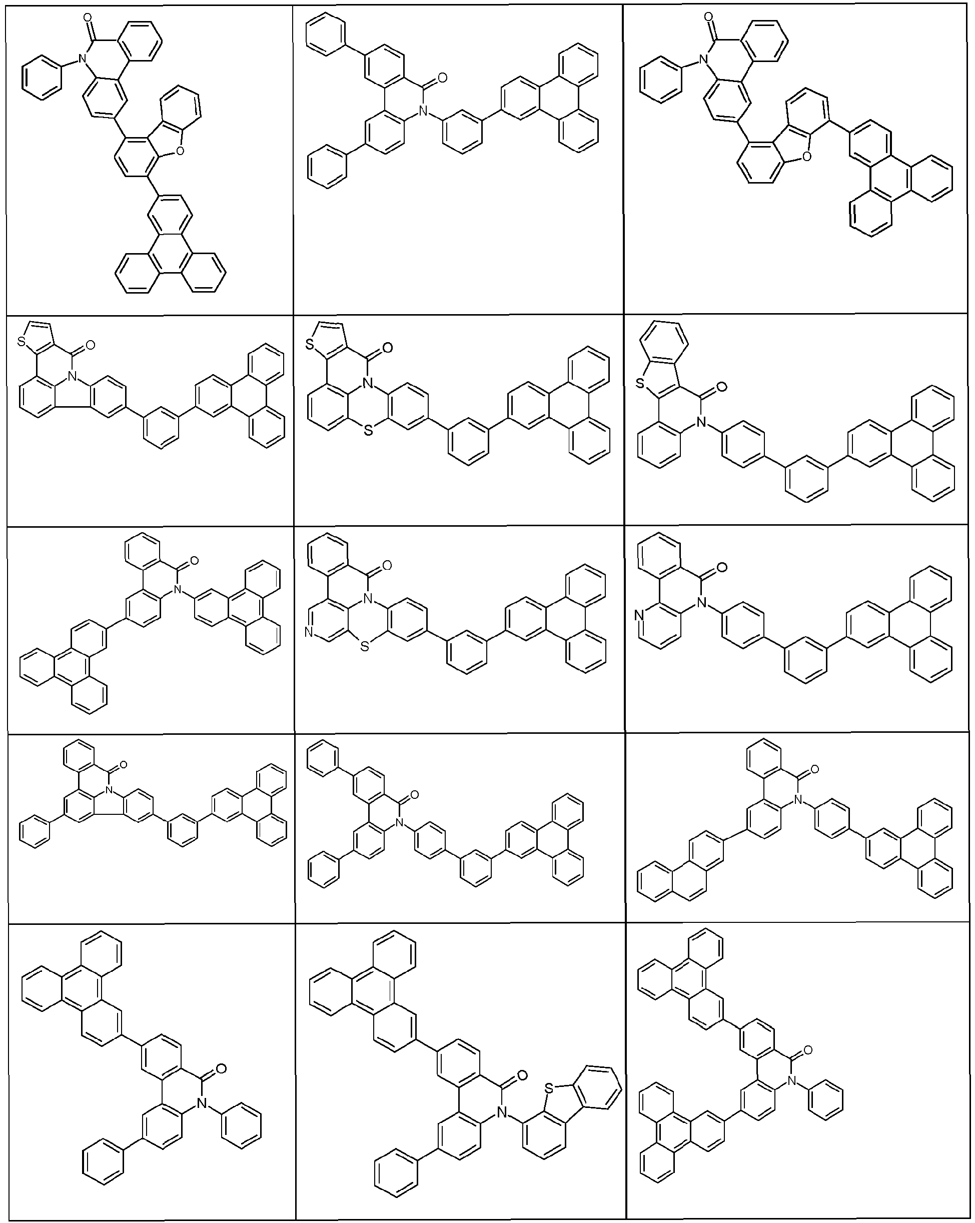

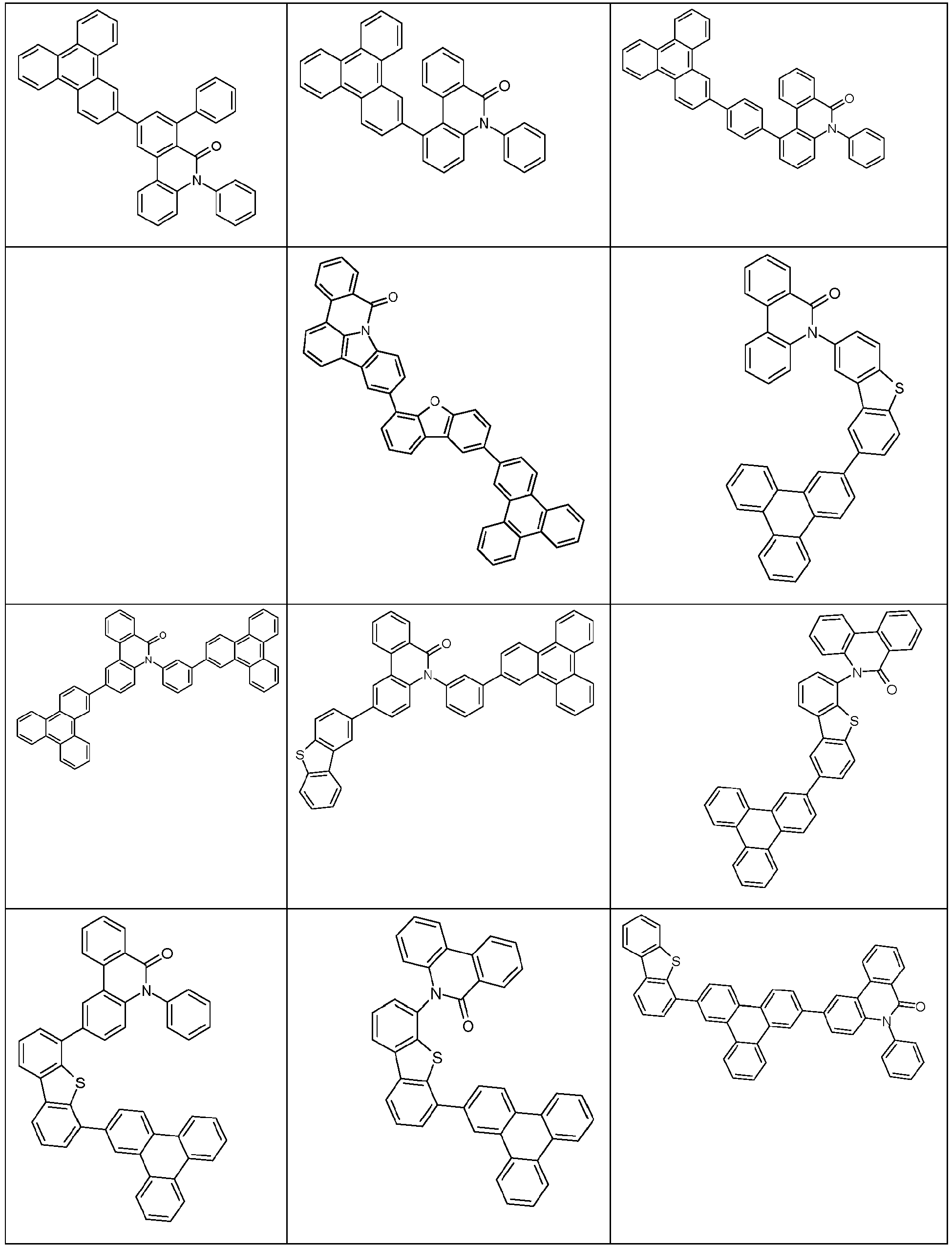

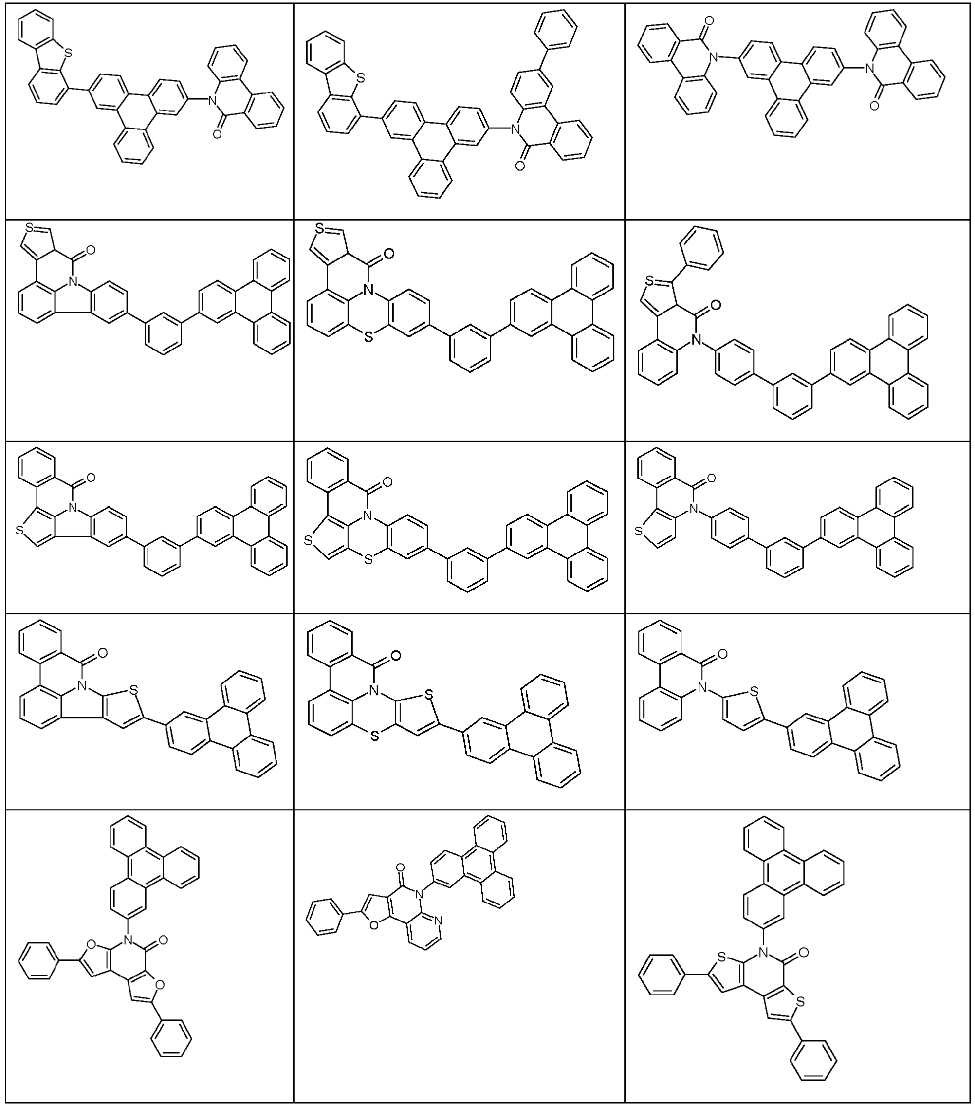

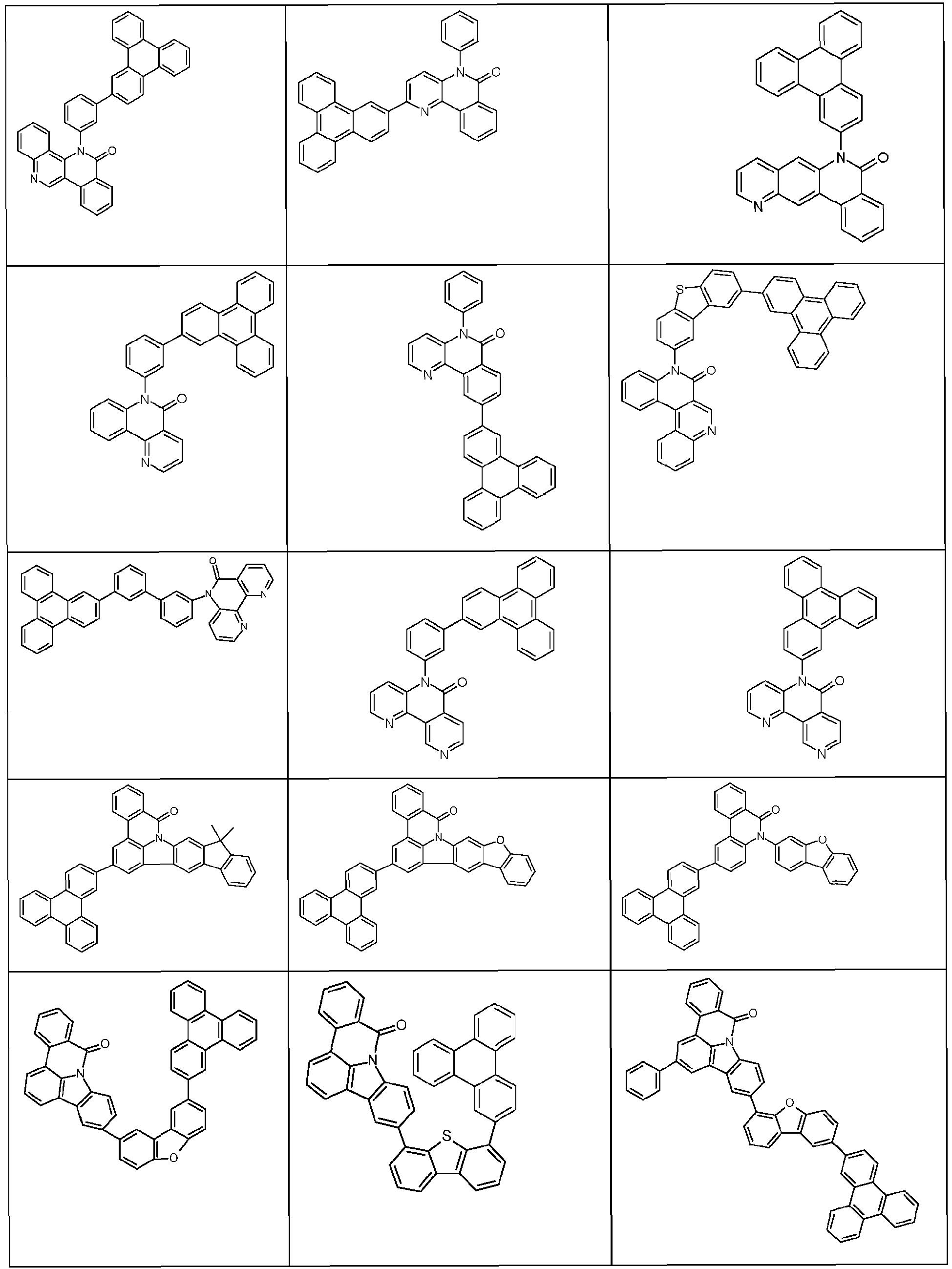

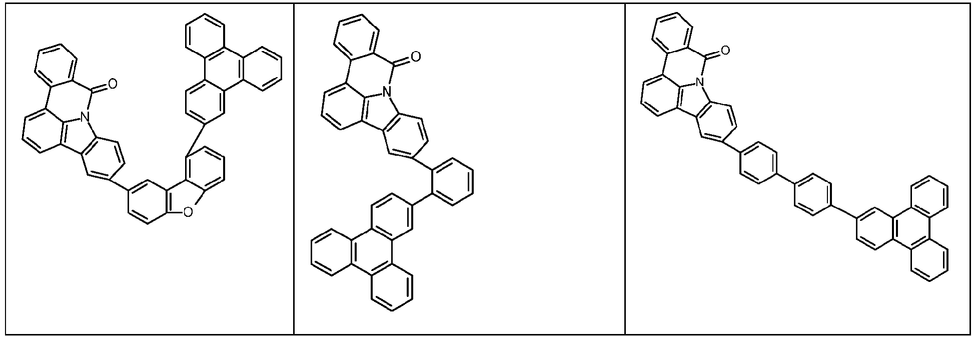

The compounds can be prepared by synthesis steps known to the person skilled in the art, such as, for example, bromination, Suzuki coupling, Ullmann coupling, Hartwig-Buchwald coupling, etc. Examples of suitable synthesis processes are depicted in general terms in Schemes 1 and 2 below. 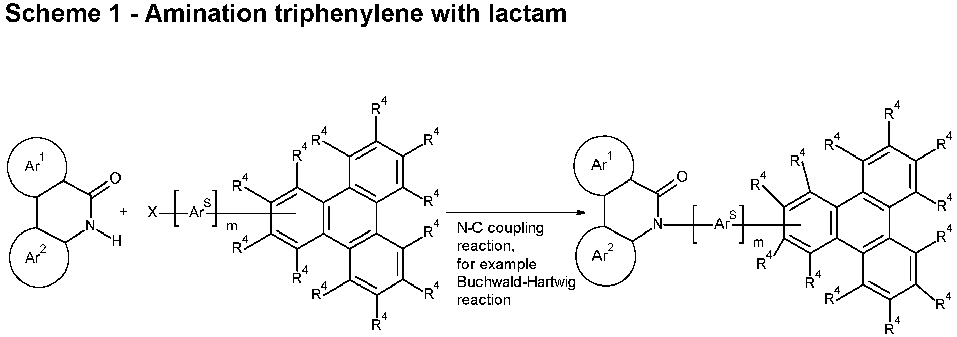 where X in scheme 1 is a leaving group, preferably selected from halogens (like Br, Cl, I), boronic acids and triflates, and where the other symbols and indices have the same meaning as above. 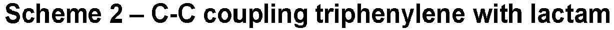 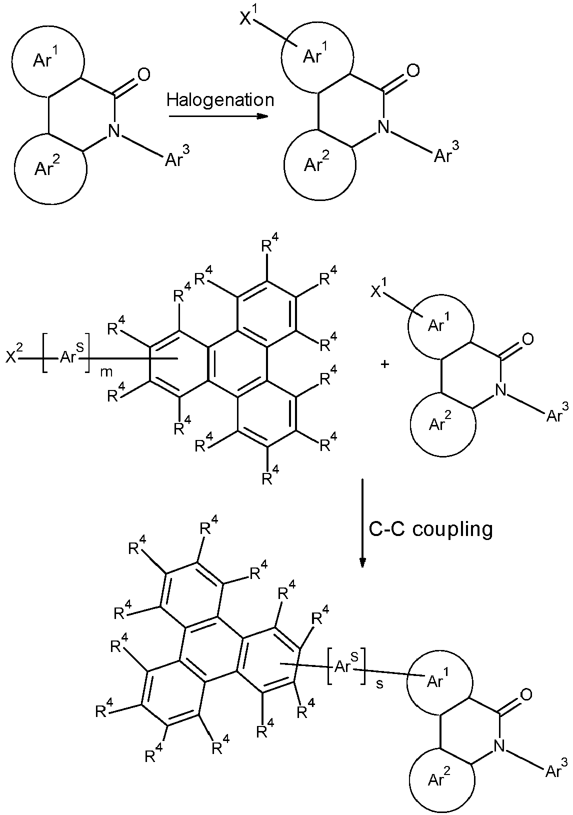 where X¹ and X², in scheme 2, are leaving groups, preferably selected from halogens (like Br, Cl, I), boronic acids and triflates, and where the other symbols and indices have the same meaning as above.

For the processing of the compounds from the liquid phase, for example by spin coating or by printing processes, formulations of the compounds are necessary. These formulations can be, for example, solutions, dispersions or emulsions. It may be preferred to use mixtures of two or more solvents for this purpose. Suitable and preferred solvents are, for example, toluene, anisole, o-, m- or p-xylene, methyl benzoate, mesitylene, tetralin, veratrol, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol - monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

A formulation here comprises a compound of formula (1) and at least one further compound. The further compound may be, for example, a solvent, in particular one of the above-mentioned solvents or a mixture of these solvents. However, the further compound may also be at least one further organic or inorganic compound which is likewise employed in the electronic device, for example an emitting compound, in particular a phosphorescent dopant, and/or a further matrix material. Suitable emitting compounds and further matrix materials are indicated below in connection with the organic electroluminescent device. This further compound may also be polymeric.

The compounds and mixtures are suitable for use in an electronic device. An electronic device here is taken to mean a device which comprises at least one layer which comprises at least one organic compound. However, the component here may also comprise inorganic materials or also layers built up entirely from inorganic materials.

An electronic device is preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic dye-sensitised solar cells, organic optical detectors, organic photoreceptors, organic field-quench devices (O-FQDs), light-emitting electrochemical cells (LECs), organic laser diodes (O-lasers) and "organic plasmon emitting devices" (D. M. Koller et al., Nature Photonics 2008, 1-4), preferably organic electroluminescent devices (OLEDs, PLEDs), in particular phosphorescent OLEDs.

The organic electroluminescent device comprises a cathode, an anode and at least one emitting layer. Apart from these layers, it may also comprise further layers, for example in each case one or more hole-injection layers, hole-transport layers, hole-blocking layers, electron-transport layers, electron-injection layers, exciton-blocking layers, electron-blocking layers and/or charge-generation layers. It is likewise possible for interlayers, which have, for example, an exciton-blocking function, to be introduced between two emitting layers. However, it should be pointed out that each of these layers does not necessarily have to be present. The organic electroluminescent device here may comprise one emitting layer or a plurality of emitting layers. If a plurality of emission layers are present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). These can be fluorescent or phosphorescent emission layers or hybrid systems, in which fluorescent and phosphorescent emission layers are combined with one another.

The organic electroluminescent device here may comprise one emitting layer or a plurality of emitting layers, where at least one emitting layer comprises at least one compound of formula (1) as matrix material.

Phosphorescence in the sense of this invention is taken to mean the luminescence from an excited state having spin multiplicity > 1, in particular from an excited triplet state. For the purposes of this application, all luminescent transition-metal complexes and luminescent lanthanide complexes, in particular all iridium, platinum and copper complexes, are to be regarded as phosphorescent compounds.

The mixture comprising the compound of the formula (1) or in accordance with the preferred embodiments and the emitting compound comprises between 99 and 1% by vol., preferably between 98 and 10% by vol., particularly preferably between 97 and 60% by vol., in particular between 95 and 80% by vol., of the compound of the formula (1) or in accordance with the preferred embodiments, based on the entire mixture comprising emitter and matrix material. Correspondingly, the mixture comprises between 1 and 99% by vol., preferably between 2 and 90% by vol., particularly preferably between 3 and 40% by vol., in particular between 5 and 20% by vol., of the emitter, based on the entire mixture comprising emitter and matrix material.

Suitable phosphorescent compounds (= triplet emitters) are, in particular, compounds which emit light, preferably in the visible region, on suitable excitation and in addition contain at least one atom having an atomic number greater than 20, preferably greater than 38 and less than 84, particularly preferably greater than 56 and less than 80, in particular a metal having this atomic number. The phosphorescent emitters used are preferably compounds which contain copper, molybdenum, tungsten, rhenium, ruthenium, osmium, rhodium, iridium, palladium, platinum, silver, gold or europium, in particular compounds which contain iridium or platinum. For the purposes of the present invention, all luminescent compounds which contain the above-mentioned metals are regarded as phosphorescent compounds.

Examples of the emitters described above are revealed by the applications WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094962, WO 2014/094961, WO 2014/094960, WO 2016/124304, WO 2016/125715, WO 2017/032439 as well as the not yet published applications WO 2018/011186 and WO 2018/041769. In general, all phosphorescent complexes as used in accordance with the prior art for phosphorescent OLEDs and as are known to the person skilled in the art in the area of organic electroluminescence are suitable, and the person skilled in the art will be able to use further phosphorescent complexes without inventive step.

Examples of suitable phosphorescent emitters are the phosphorescent emitters listed in the table below:

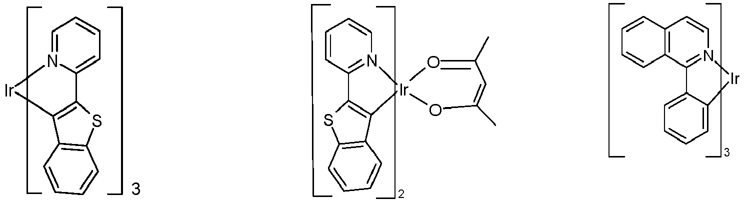

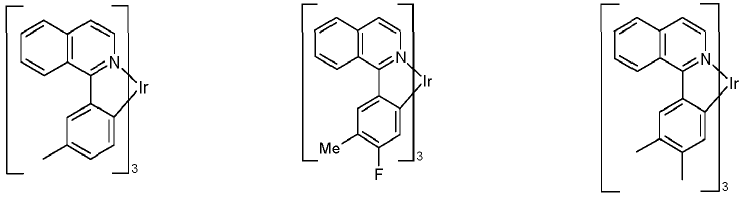

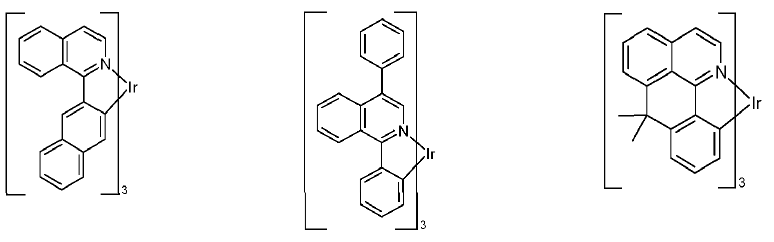

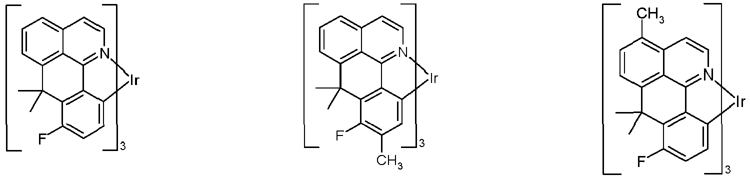

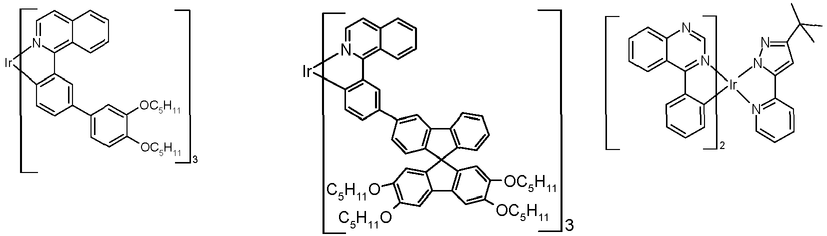

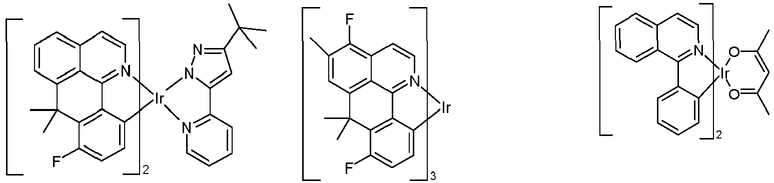

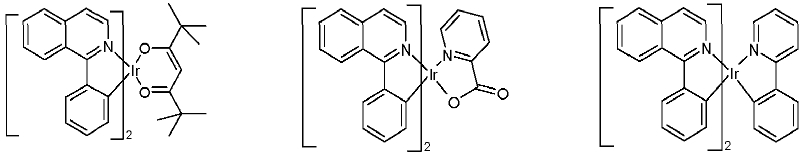

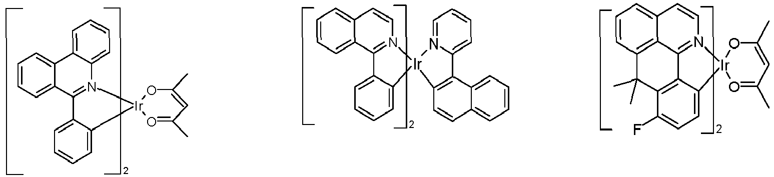

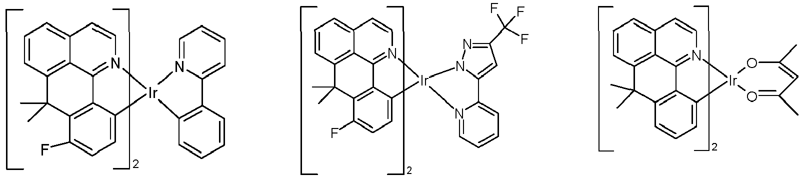

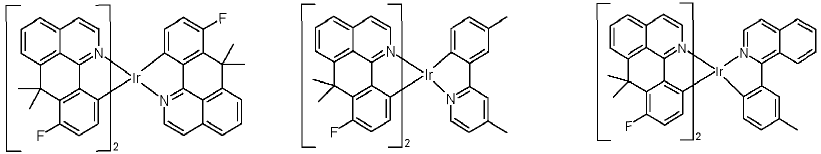

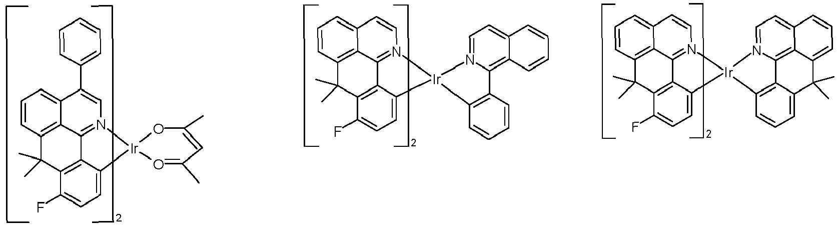

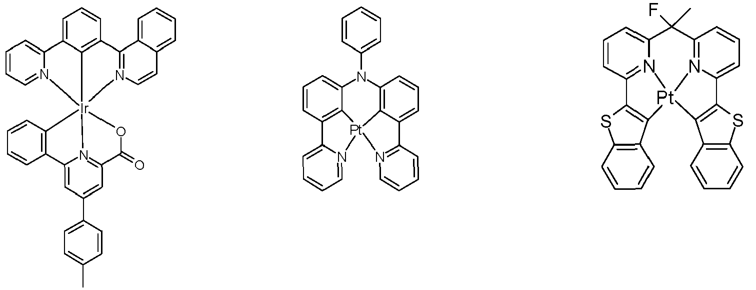

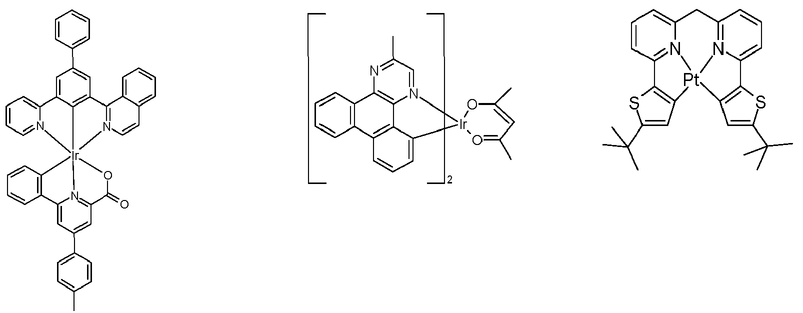

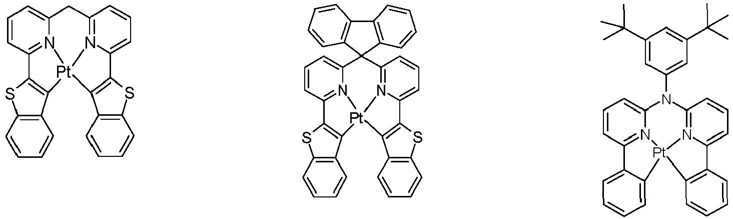

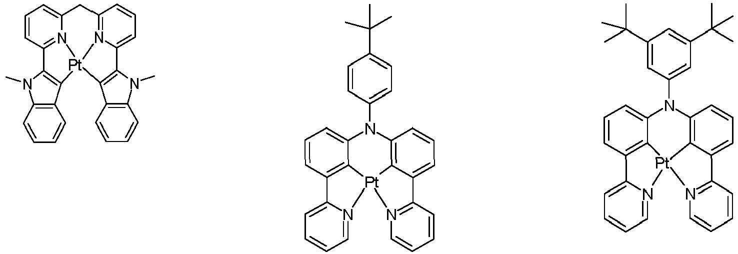

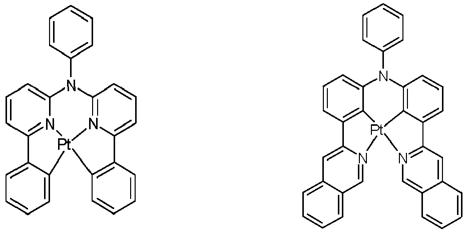

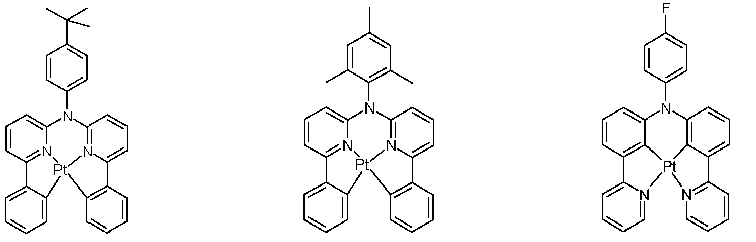

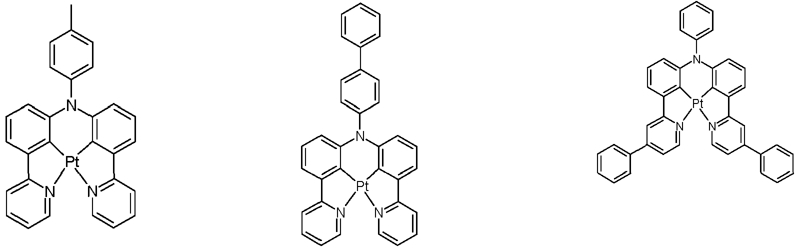

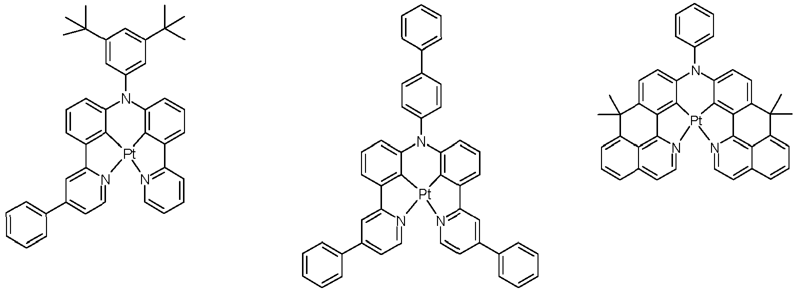

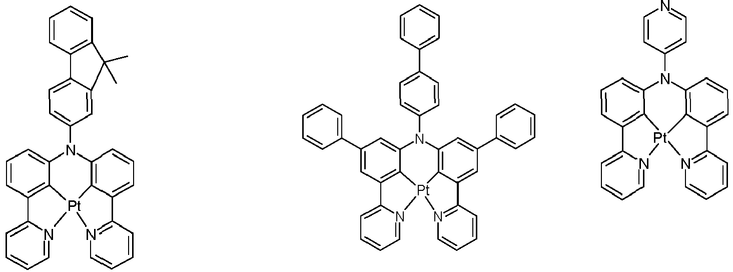

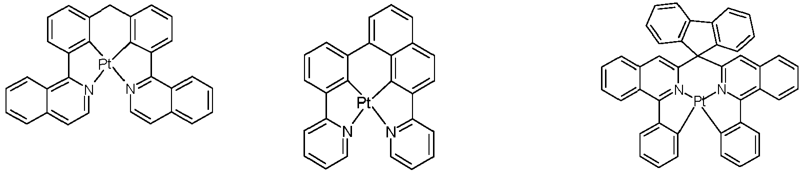

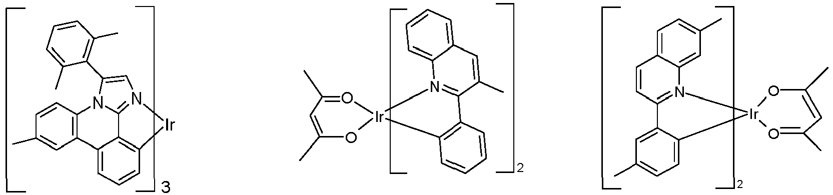

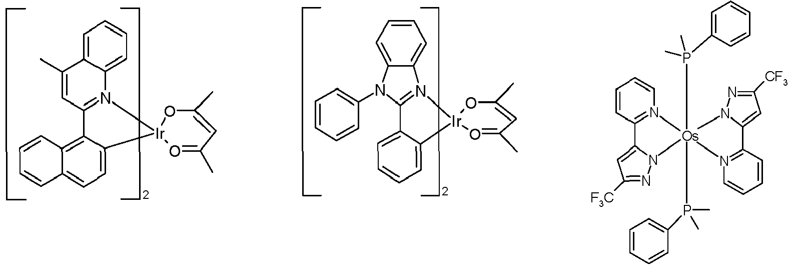

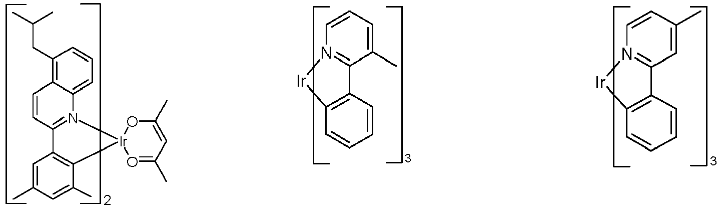

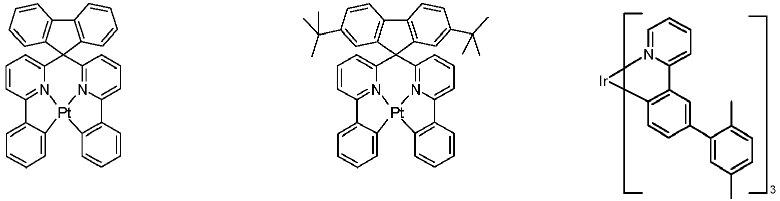

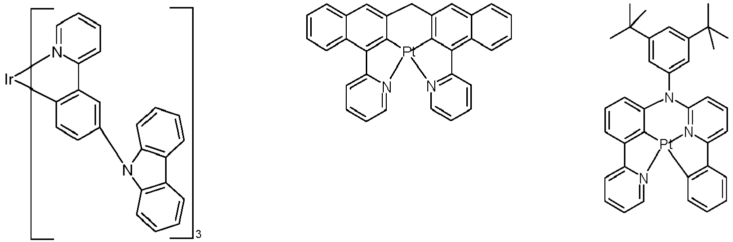

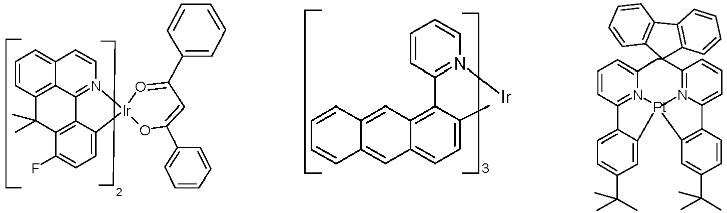

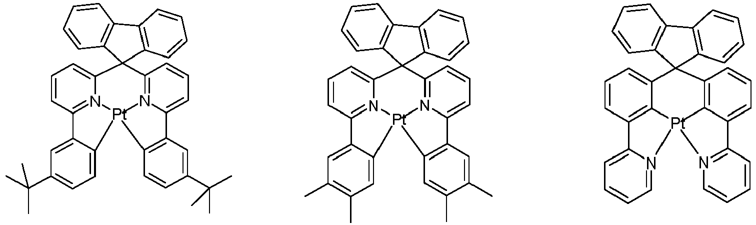

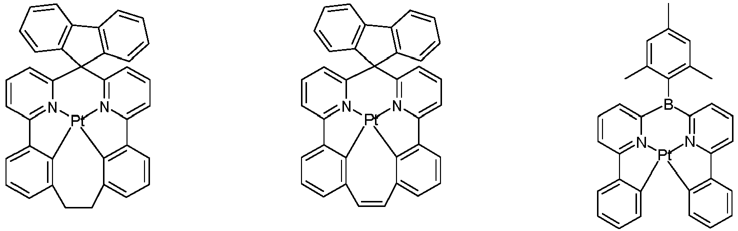

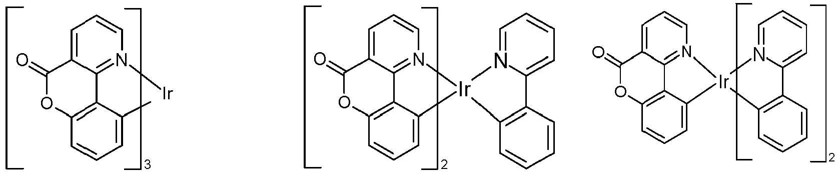

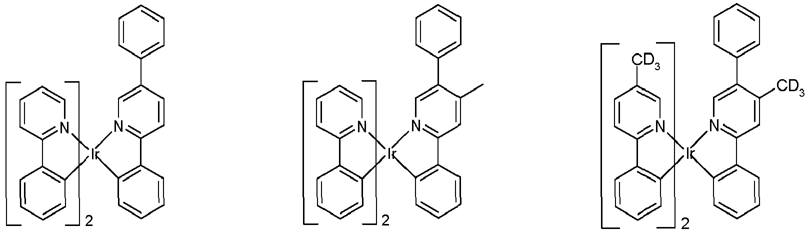

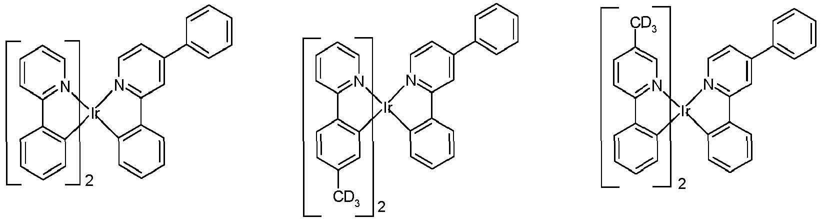

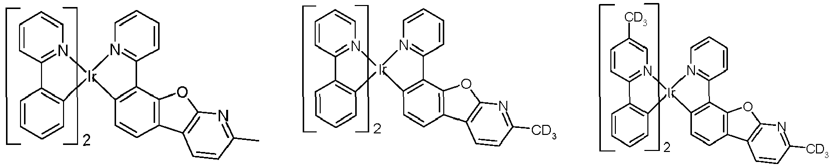

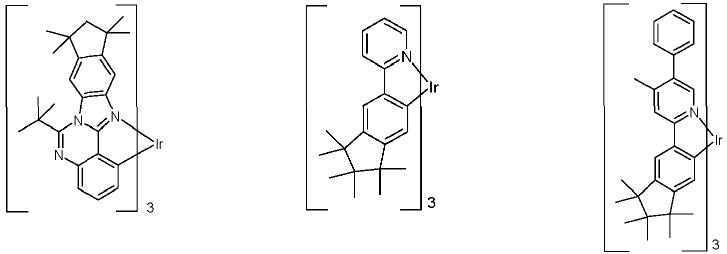

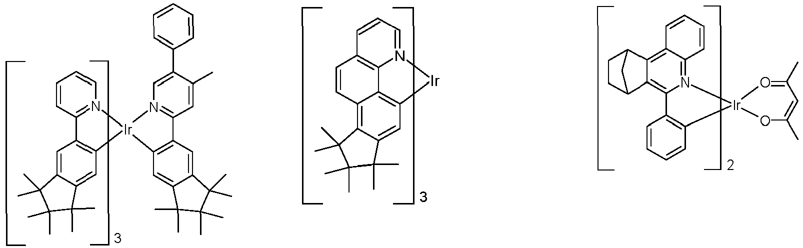

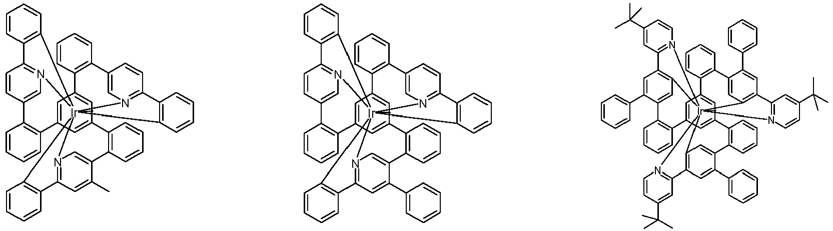

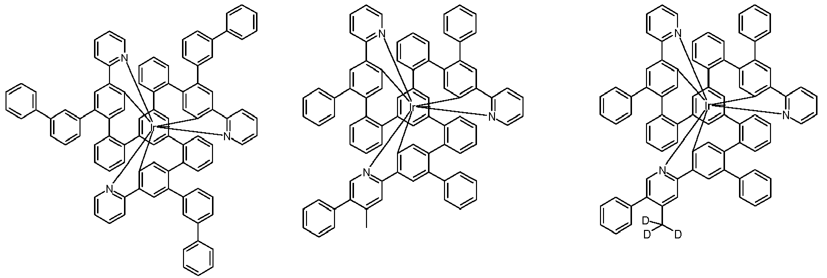

Suitable phosphorescent materials (= triplet emitters) that can be advantageously combined with the compounds of formula (1) are, as mentioned above, compounds which emit a red light on suitable excitation, which means phosphorescent materials having an excited triplet state level (T1) comprised between 550 and 680 nm.

A further preferred embodiment of the present invention is the use of the compound of the formula (1) or in accordance with the preferred embodiments as matrix material for a phosphorescent emitter in combination with a further matrix material. Particularly suitable matrix materials which can be employed in combination with the compounds of the formula (1) or in accordance with the preferred embodiments are aromatic ketones, aromatic phosphine oxides or aromatic sulfoxides or sulfones, for example in accordance with WO 2004/013080, WO 2004/093207, WO 2006/005627 or WO 2010/006680, triarylamines, carbazole derivatives, for example CBP (N,N-bis-carbazolylbiphenyl) or the carbazole derivatives disclosed in WO 2005/ 039246, US 2005/0069729, JP 2004/288381, EP 1205527 or WO 2008/ 086851, indolocarbazole derivatives, for example in accordance with WO 2007/063754 or WO 2008/056746, indenocarbazole derivatives, for example in accordance with WO 2010/136109 and WO 2011/000455, azacarbazole derivatives, for example in accordance with EP 1617710, EP 1617711, EP 1731584, JP 2005/347160, bipolar matrix materials, for example in accordance with WO 2007/137725, silanes, for example in accordance with WO 005/111172, azaboroles or boronic esters, for example in accordance with WO 2006/117052, triazine derivatives, for example in accordance with WO 2010/015306, WO 2007/063754 or WO 2008/056746, zinc complexes, for example in accordance with EP 652273 or WO 2009/062578, diazasilole or tetraazasilole derivatives, for example in accordance with WO 2010/054729, diazaphosphole derivatives, for example in accordance with WO 2010/054730, bridged carbazole derivatives, for example in accordance with US 2009/0136779, WO 2010/050778, WO 2011/042107, WO 2011/088877 or in accordance with EP 11003232.3, triphenylene derivatives, for example in accordance with WO 2012/048781, or lactams, for example in accordance with WO 2011/116865 or WO 2011/137951. A further phosphorescent emitter which emits at shorter wavelength than the actual emitter may likewise be present in the mixture as co-host.

Preferred co-host materials are triarylamine derivatives, lactams, carbazole derivatives and indenocarbazole derivatives. Preferred co-host materials are very particularly carbazole derivatives and indenocarbazole derivatives.

More preferably, the second compound comprising at least one group of formula (11) or (12) is selected from the compounds of formulae (11-1) to (12-4), 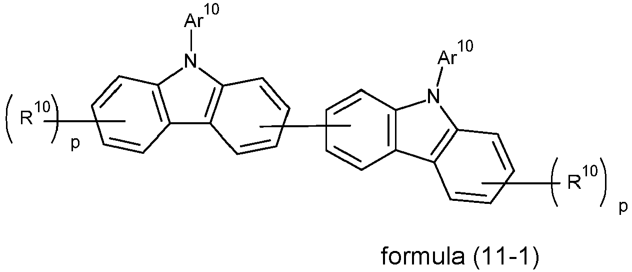 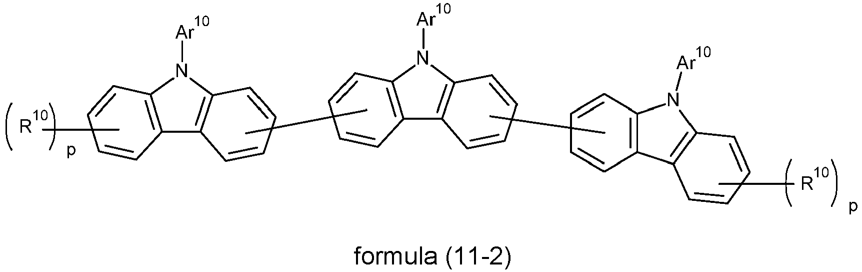 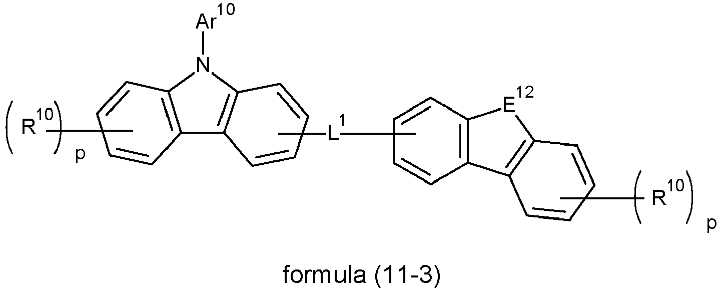 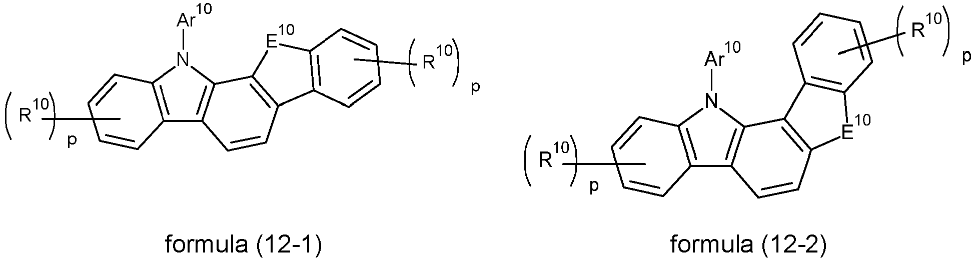 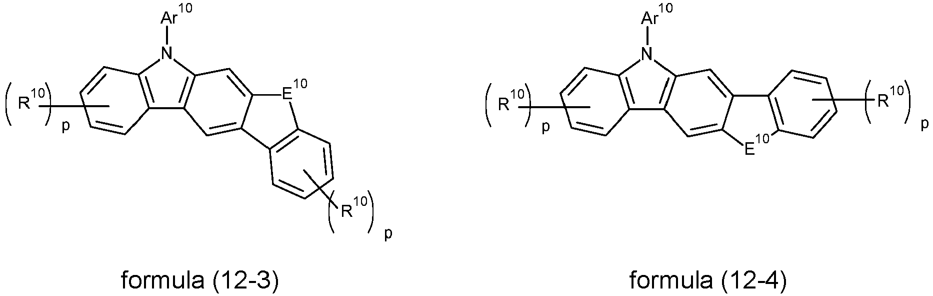
where the symbols and indices Ar¹⁰, R¹⁰, E¹⁰, L¹ and p have the same meaning as above; and
E¹² stands for -C(R⁰)₂-, -O-, -S-, -N(Ar¹⁰)-; where R⁰ has the same meaning as above.

Particularly preferably, the second compound comprising at least one group of formula (11) or (12) is selected from the compounds of formulae (13-1) to (14-4), 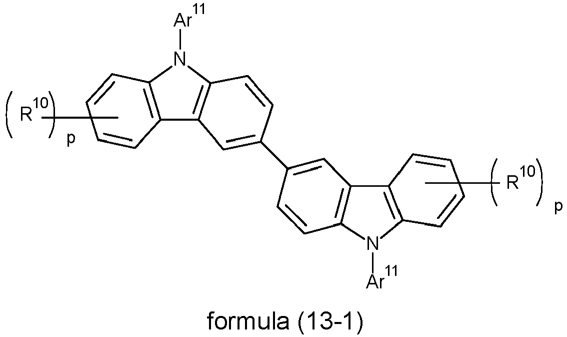 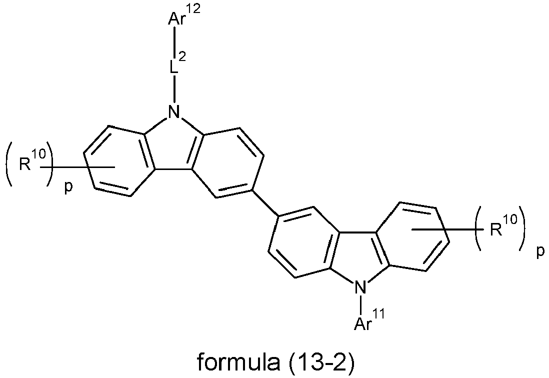 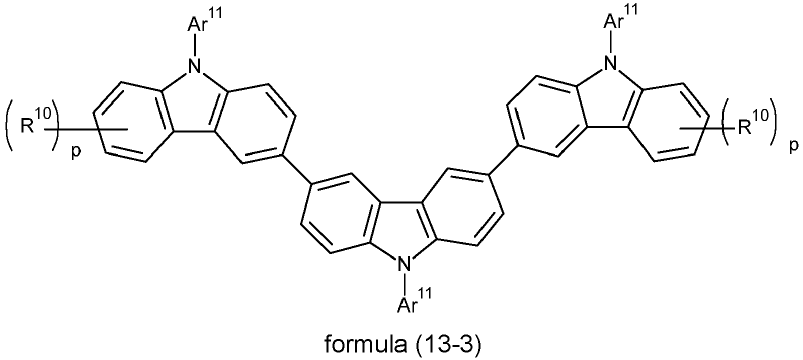 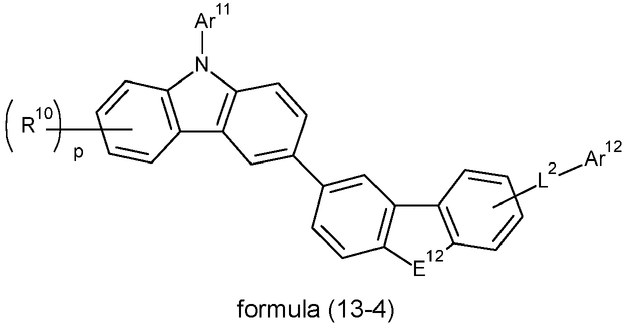 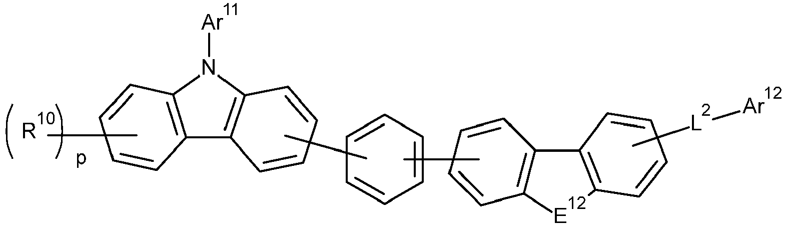 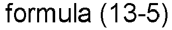 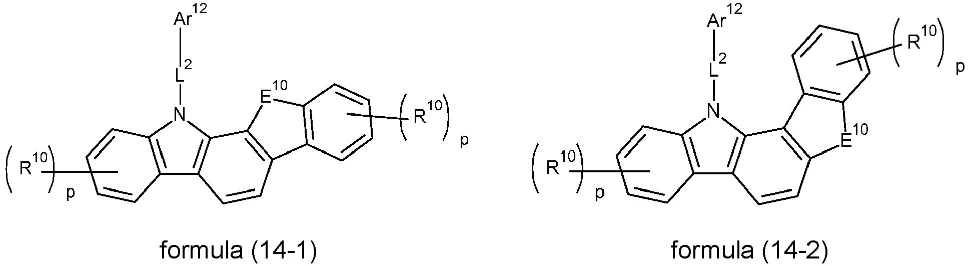 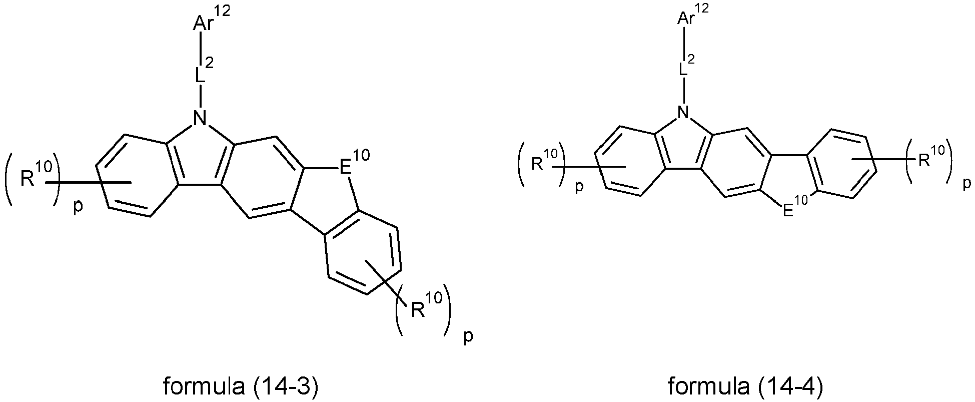 where the symbols and indices R¹⁰, E¹⁰, E¹² and p have the same meaning as above; and where:
- Ar¹⁰: stands for an aromatic or heteroaromatic ring system selected from benzene, naphthalene, pyrene, biphenyl, terphenyl, quaterphenyl, fluorene, spirobifluorene, cis- or trans-indenofluorene, dibenzofuran, dibenzothiophene, carbazole, indolocarbazole, indenocarbazole, which may be substituted by one or more radicals R; or combinations of these groups;
- L²: stands for a single bond or an aromatic or heteroaromatic rings system having 6 to 18 aromatic rings atoms, which may be substituted by one or more radicals R;
- Ar¹²: stands for an heteroaromatic ring system selected from pyridine, pyrazine, pyrimidine, triazine, which may be substituted by one or more radicals R; and
- R: has the same meaning as above.

In a further embodiment of the invention, the organic electroluminescent device according to the invention does not comprise a separate hole-injection layer and/or hole-transport layer and/or hole-blocking layer and/or electron-transport layer, i.e. the emitting layer is directly adjacent to the hole-injection layer or the anode, and/or the emitting layer is directly adjacent to the electron-transport layer or the electron-injection layer or the cathode, as described, for example, in WO 2005/053051. It is furthermore possible to use a metal complex which is identical or similar to the metal complex in the emitting layer as hole-transport or hole-injection material directly adjacent to the emitting layer, as described, for example, in WO 2009/030981.

In the further layers of the organic electroluminescent device according to the invention, it is possible to use all materials as usually employed in accordance with the prior art. The person skilled in the art will therefore be able, without inventive step, to employ all materials known for organic electroluminescent devices in combination with the compounds of the formula (1) or in accordance with the preferred embodiments.

For example, the compound of formula (1) can also be used as a matrix for semiconducting light-emitting nanoparticles. In the context of the present invention, the term "nano" denotes a size in the range from 0.1 to 999 nm, preferably from 1 to 150 nm. In a preferred embodiment, the semiconducting light-emitting nano-particle is a quantum material ("Quantum sized material"). The term "quantum material" in the sense of the present invention refers to the size of the semiconductor material itself without further connections or a further surface modification, which shows the so-called quantum confinement effect, as for example in ISBN: 978-3-662-44822-9. In one embodiment of the invention, the total size of the quantum material is in the range from 1 to 100 nm, more preferably from 1 to 30 nm and particularly preferably from 5 to 15 nm. In this case, the core of the semiconducting light-emitting nano-particle can vary. Suitable examples are CdS, CdSe, CdTe, ZnS, ZnSe, ZnSeS, ZnTe, ZnO, GaAs, GaP, GaSb, HgS, HgSe, HgSe, HgTe, InAs, InP, InPS, InPZnS, InPZn, InPGa, InSb, AlAs , AIP, AlSb, Cu₂S, Cu₂Se, CulnS₂, CulnSe₂, Cu₂(ZnSn)S₄, Cu₂(InGa) S₄, TiO₂, or a combination of said materials. In a preferred embodiment, the core of the semiconductive light-emitting particle contains one or more elements of group 13 and one or more elements of group 15 of the periodic system of the elements, for example GaAs, GaP, GaSb, InAs, InP, InPS, InPZnS, InPZn, InPGa, InSb, AlAs, AIP, AlSb, CulnS₂, CulnSe₂, Cu₂(InGa)S₄ or a combination of the mentioned materials. Particularly preferably, the core contains In- and P-atoms, z. InP, InPS, InPZnS, InPZn or InPGa. In a further embodiment of the invention, the nanoparticle contains one or more shell layers, which comprise a first element from the group 12, 13 or 14 of the periodic table and a second element from the group 15 or 16 of the periodic table. Preferably, all shell layers contain a first element from the group 12, 13 or 14 of the periodic system and a second element from the group 15 or 16 of the periodic system. In a preferred embodiment of the invention, at least one of the shell layers contains a first element from the group 12 and a second element from the group 16 of the periodic table, for example CdS, CdZnS, ZnS, ZnSe, ZnSSe, ZnSSeTe, CdS/ZnS, ZnSe/ZnS or ZnS/ZnSe. Particularly preferably, all shell layers contain a first element from the group 12 and a second element from the group 16 of the periodic table.

Preference is furthermore given to an organic electroluminescent device, characterised in that one or more layers are applied by means of a sublimation process, in which the materials are vapour-deposited in vacuum sublimation units at an initial pressure of less than 10⁻⁵ mbar, preferably less than 10⁻⁶ mbar. However, it is also possible for the initial pressure to be even lower or higher, for example less than 10⁻⁷ mbar.

Preference is likewise given to an organic electroluminescent device, characterised in that one or more layers are applied by means of the OVPD (organic vapour phase deposition) process or with the aid of carrier-gas sublimation, in which the materials are applied at a pressure between 10⁻⁵ mbar and 1 bar. A special case of this process is the OVJP (organic vapour jet printing) process, in which the materials are applied directly through a nozzle and thus structured (for example M. S. Arnold et al., Appl. Phys. Lett. 2008, 92, 053301).

Preference is furthermore given to an organic electroluminescent device, characterised in that one or more layers are produced from solution, such as, for example, by spin coating, or by means of any desired printing process, such as, for example, ink-jet printing, LITI (light induced thermal imaging, thermal transfer printing), screen printing, flexographic printing, offset printing or nozzle printing. Soluble compounds, which are obtained, for example, by suitable substitution, are necessary for this purpose.

Also possible are hybrid processes, in which, for example, one or more layers are applied from solution and one or more further layers are applied by vapour deposition. Thus, it is possible, for example, to apply the emitting layer from solution and to apply the electron-transport layer by vapour deposition.

These processes are generally known to the person skilled in the art and can be applied by him without inventive step to organic electroluminescent devices comprising the compounds according to the invention.

The compounds according to the invention generally have very good properties on use in organic electroluminescent devices. In particular, the lifetime on use of the compounds according to the invention in organic electroluminescent devices is significantly better compared with similar compounds in accordance with the prior art. The other properties of the organic electroluminescent device, in particular the efficiency and the voltage, are likewise better or at least comparable. Furthermore, the compounds have a high glass transition temperature and high thermal stability.

The invention will now be explained in greater detail by the following examples, without wishing to restrict it thereby.

### A) Syntheses Examples

The following syntheses are carried out, unless indicated otherwise, under a protective-gas atmosphere in dried solvents. The solvents and reagents can be purchased, for example, from Sigma-ALDRICH or ABCR. The corresponding CAS numbers are also indicated in each case from the compounds known from the literature.

### Example a:

### 6-(3-Triphenylen-2-yl-phenyl)-6H-benzo[c][2,6]naphthyridin-5-one

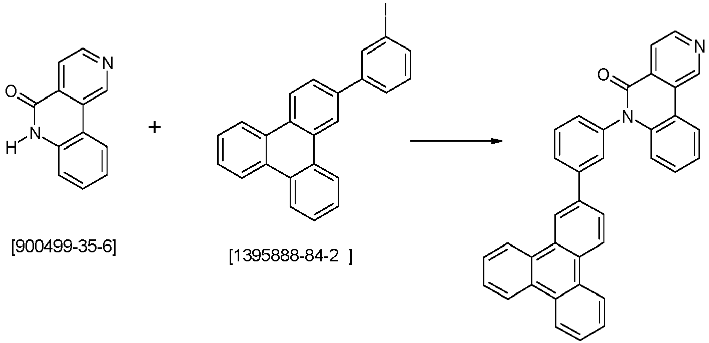

19.6 g (100 mmol) of benzo[c]-2,6-naphthyridinone, 51.6 g (120 mmol) of 2-(3-iodo-phenyl)-triphenylene and 2.3 g (20 mmol) of L-proline are dissolved in 100 mL of DMF and stirred at 150 ° C for 30 h. The solution is diluted with water, extracted with ethyl acetate twice, the combined organic phases dried over Na₂SO₄ and concentrated by rotary evaporation. The residue is purified by chromatography (EtOAc / hexane: 2/3). The residue is recrystallized from toluene and from dichloromethane / toluene and finally sublimed under high vacuum. The yield is 39 g (78 mmol) 66% of theory.

The following compounds are obtained analogously:

| **Ex.** | **Educt 1** | **Educt 2** | **Product** | **Yield** |
|---|---|---|---|---|
| 1a | 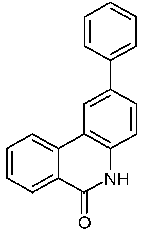 [157848-49-2] | 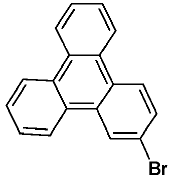 [19111-87-6 ] | 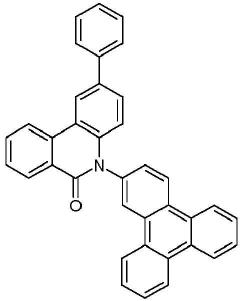 | 53% |
| 2a | 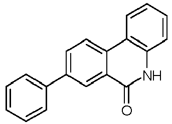 [109553-68-6 ] | 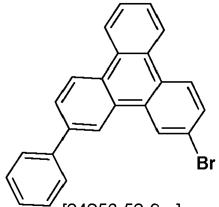 [24253-52-9 ] | 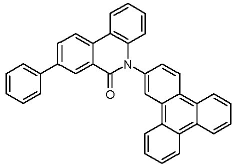 | 60% |
| 3a | 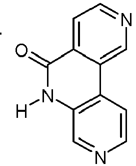 [1186039-92-8] | 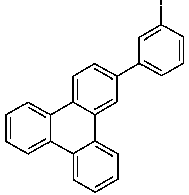 [1395888-84-2 ] | 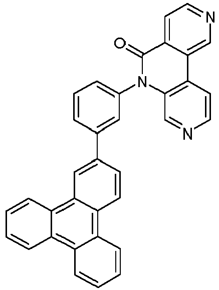 | 66% |
| 4a | 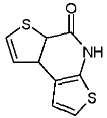 40197-38-4 | 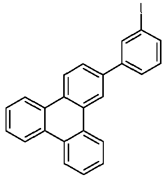 [1395888-84-2 ] | 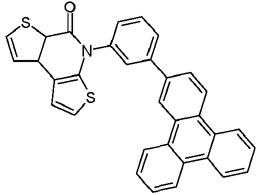 | 62% |
| 5a | 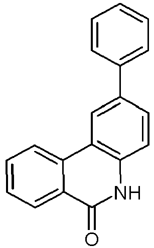 [157848-49-2] | 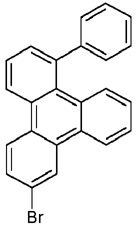 [1888414-28-5] | 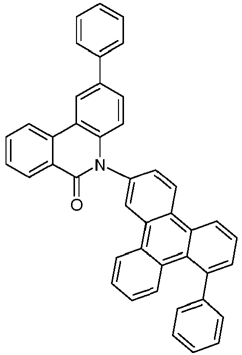 | 77% |
| 6a | 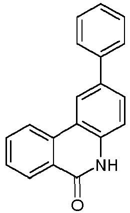 [157848-49-2] | 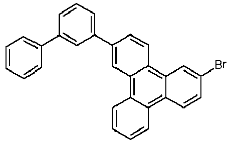 [1616514-20-5 ] | 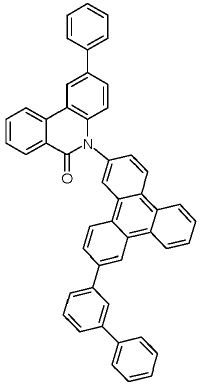 | 75% |
| 7a | 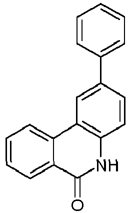 [157848-49-2] | 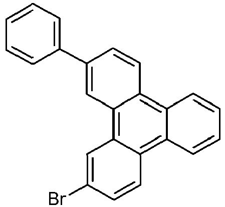 [1616514-13-6 ] | 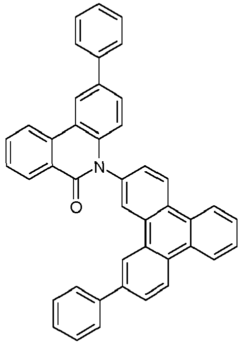 | 78% |
| 8a | 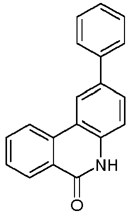 [157848-49-2] | 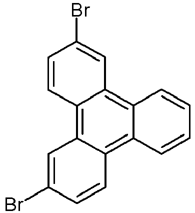 [1570190-91-8 ] | 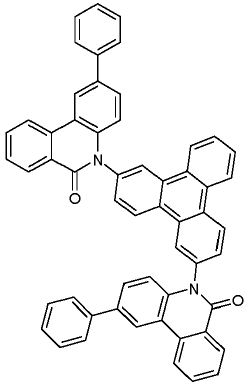 | 64% |
| 9a | 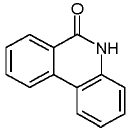 [ 1015-89-0] | 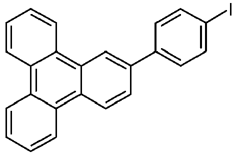 [1505512-76-4] | 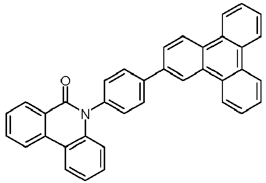 | 79% |
| 10a | 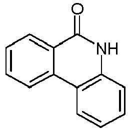 [ 1015-89-0] | 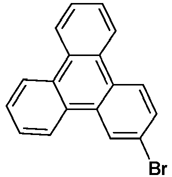 [19111-87-6 ] | 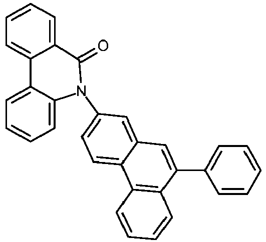 | 80% |
| 11a | 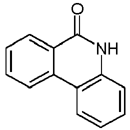 [ 1015-89-0] | 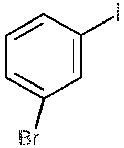 | 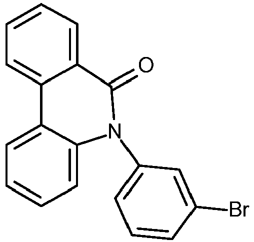 | 77% |

### Example b: 5-(4-Brom-phenyl)-5H-phenanthridin-6-one

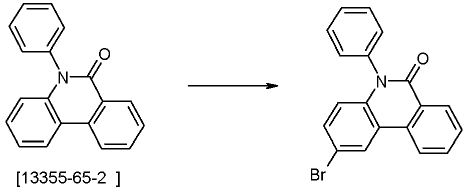

6.0 g (22.2 mmol) of 5-phenyl-5H-phenanthridin-6-one are initially put in 150 mL of DMF. A solution of 4 g (22.5 mmol) of NBS is then added dropwise in 100 mL of DMF at room temperature, with exclusion of light. The mixture is further stirred during 4 h at room temperature. Then, the mixture is mixed with 150 mL of water and extracted with CH₂Cl₂. The organic phase is dried over MgSO4 and the solvents are removed in vacuo. The product is stirred with hot hexane and filtered. Yield: 7.4 g (21 mmol), 80% of theory.

The following compounds are obtained analogously:

| **Ex.** | **Educt 1** | **Product 1** | **Yield** |
|---|---|---|---|
| 1b | 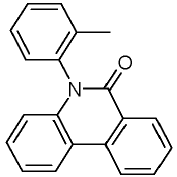 [129647-00-3 ] | 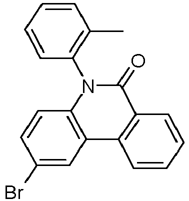 | 68 % |
| 2b | 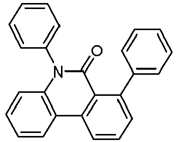 | 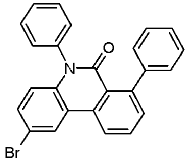 | 85% |

Analogously, the following compounds are obtained with 2 equivalents of NBS in chloroform as solvent:

| **Ex.** | **Educt 1** | **Product 1** | **Yield** |
|---|---|---|---|
| 3b | 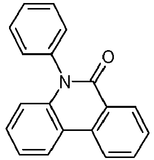 | 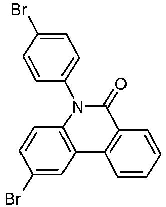 | 87 % |
| 4b | 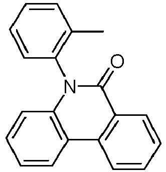 | 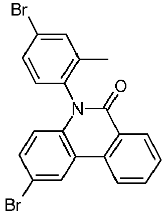 | 82 % |
| 5b | 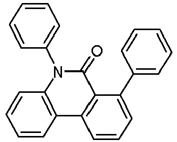 | 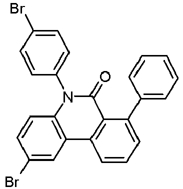 | 83% |

### Example c: 5-(4-Triphenylen-2-yl-phenyl)-5H-phenanthridin-6-one

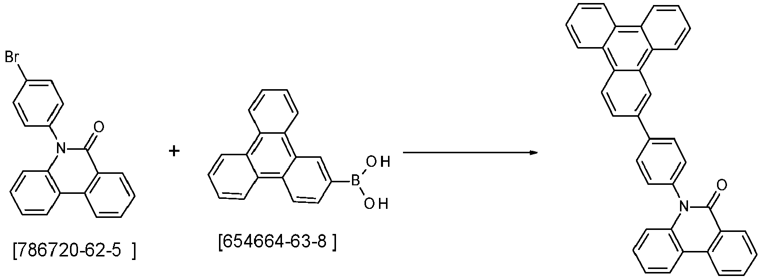

29.9 g (110.0 mmol) of triphenylene-2-boronic acid, 38 g (110.0 mmol) of 5-(4-bromo-phenyl)-5H-phenanthridin-6-one and 44.6 g (210.0 mmol) of tripotassium phosphate are suspended in 500 mL of toluene, 500 mL of dioxane and 500 mL of water. Then, 913 mg (3.0 mmol) of tri-o-tolylphosphine and subsequently 112 mg (0.5 mmol) of palladium(II)acetate are added to this suspension, and the reaction mixture is heated under reflux for 16 h. After cooling, the organic phase is collected, filtered through silica gel, washed three times with 200 mL of water and then concentrated to dryness. The residue is recrystallized from toluene and from dichloromethane / isopropanol and finally sublimed under high vacuum, purity is 99.9%. The yield is 43 g (86 mmol), corresponding to 80% of theory.

The following compounds are obtained analogously:

| **Ex.** | **Educt 1** | **Educt 2** | **Product** | **Yield** |
|---|---|---|---|---|
| 1c | 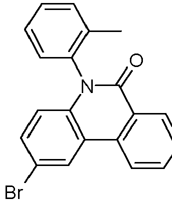 | 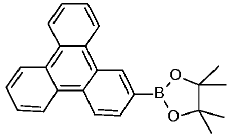 [890042-13-4 ] | 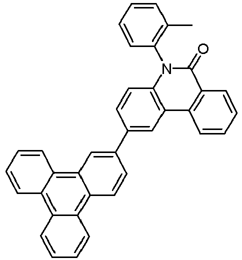 | 84% |
| 2c | 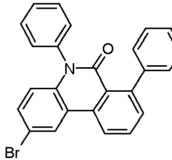 | 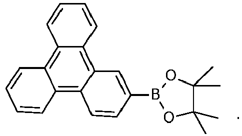 [890042-13-4 ] | 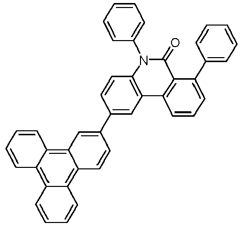 | 68% |
| 3c | 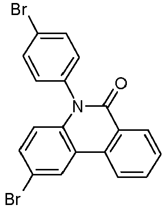 | 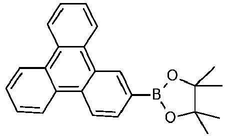 [890042-13-4 ] | 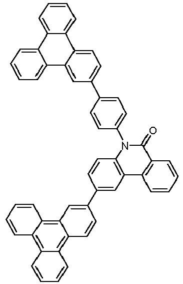 | 67% |
| 4c | 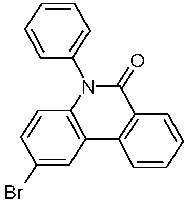 | 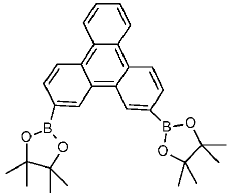 [1394813-63-8 ] | 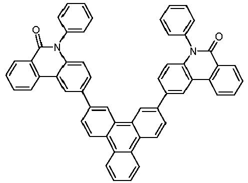 | 71% |
| 5c | 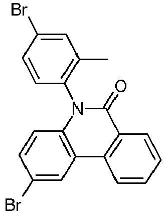 | 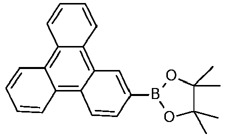 [890042-13-4 ] | 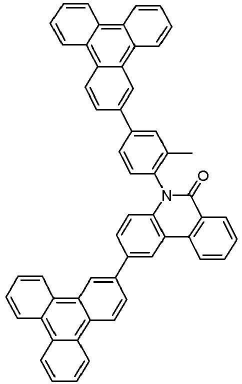 | 75% |
| 6c | 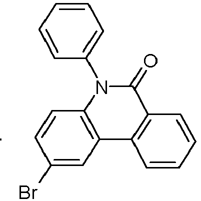 | 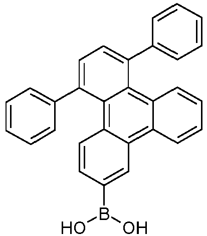 [1714136-45-4] | 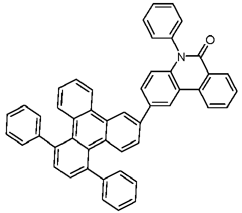 | 76% |
| 7c | 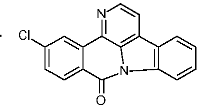 [1629245-82-4 ] | 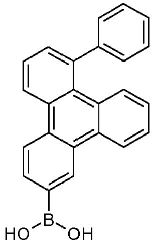 [2020404-50-4] | 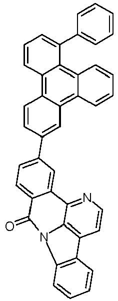 | 70% |
| 8c | 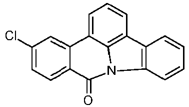 [1629245-81-3 ] | 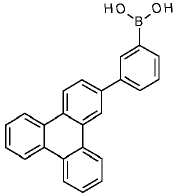 [1235876-72-8 ] | 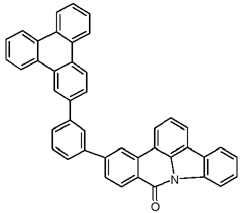 | 66% |
| 9c | 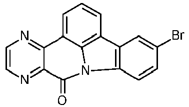 [1369958-42-8 ] | 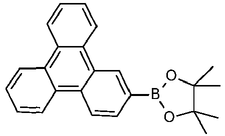 [890042-13-4 ] | 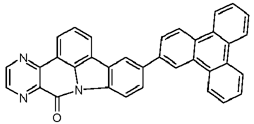 | 73% |
| 10c | 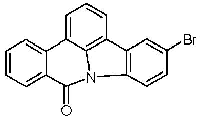 | 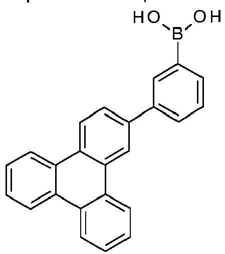 [1235876-72-8 ] | 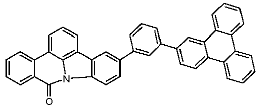 | 71% |
| 11c | 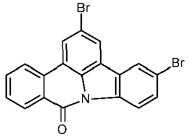 [1346571-48-9 ] | 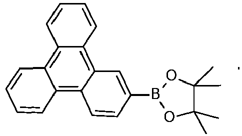 [890042-13-4 ] | 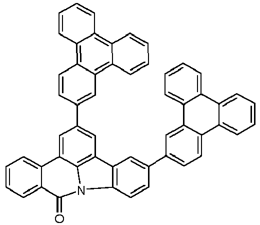 | 80% |
| 12c | 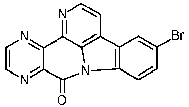 [1346571-41-2] | 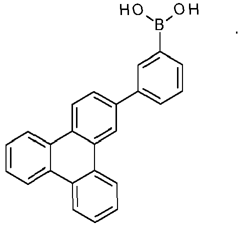 [1235876-72-8 ] | 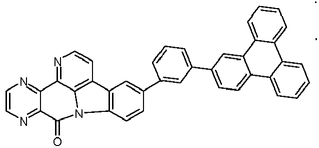 | 78% |
| 13c | 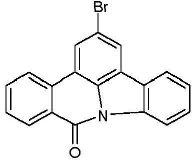 [1346571-39-8] | 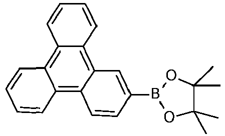 [890042-13-4 ] | 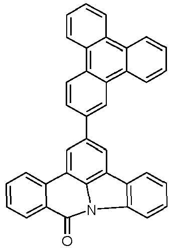 | 81% |
| 14c | 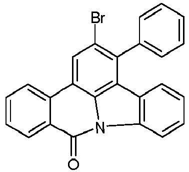 [25890-89-5 ] | 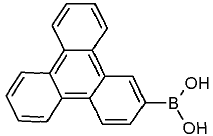 [654664-63-8 ] | 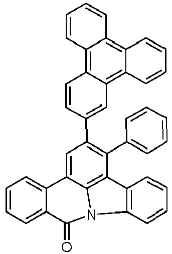 | 63% |
| 15c | 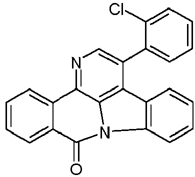 [904503-80-6 ] | 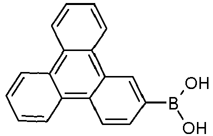 [654664-63-8 ] | 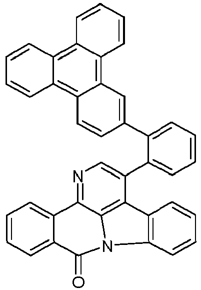 | 59% |
| 16c | 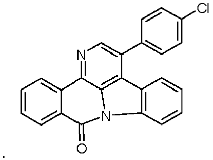 [904503-77-1 ] | 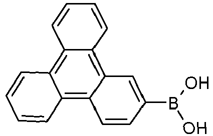 [654664-63-8 ] | 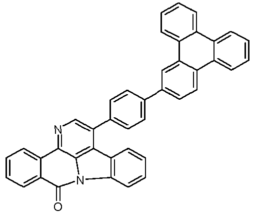 | 79% |
| 17c | 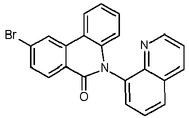 [2088465-03-4 ] | 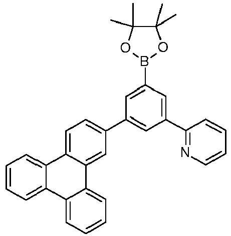 [1943719-84-3 ] | 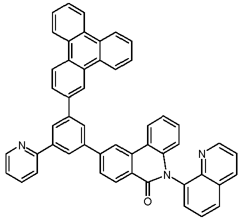 | 75% |
| 18c | 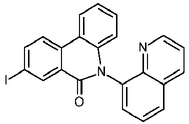 [2088464-91-7] | 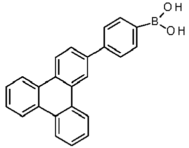 [1620765-26-5] | 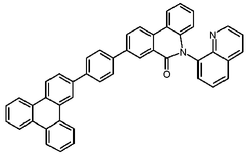 | 79% |
| 19c | 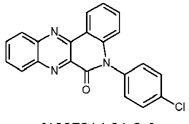 [1887014-91-6] | 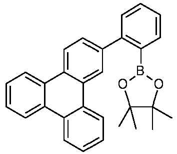 [1638272-53-3] | 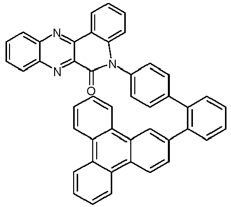 | 64% |
| 20c | 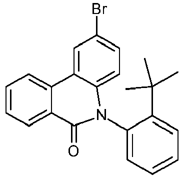 [1840905-70-5] | 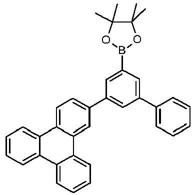 [1372893-20-3] | 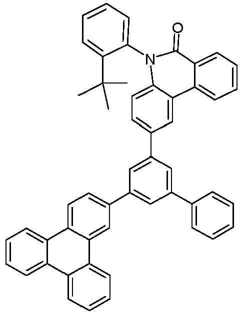 | 81% |
| 21c | 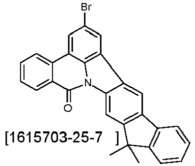 | 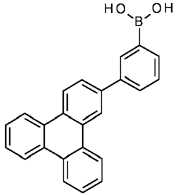 [1235876-72-8] | 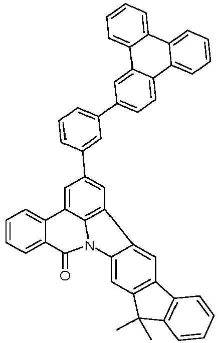 | 77% |
| 22c | 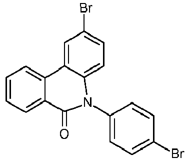 [1433190-03-4] | 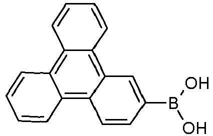 [654664-63-8] | 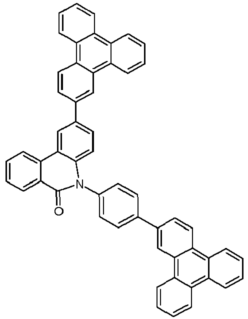 | 79% |
| 23c | 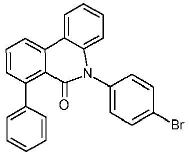 1433190-00-1] | 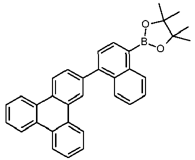 [1158227-50-9] | 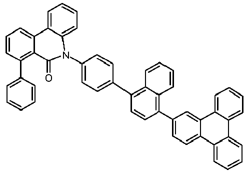 | 76% |
| 24c | 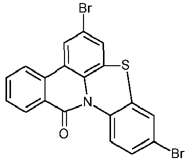 [1346571-50-3] | 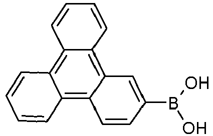 [654664-63-8] | 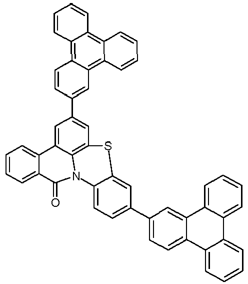 | 68% |
| 25c | 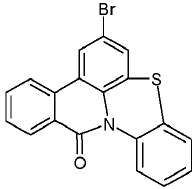 [1346571-45-6] | 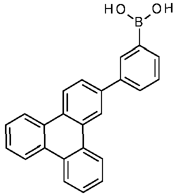 [1235876-72-8] | 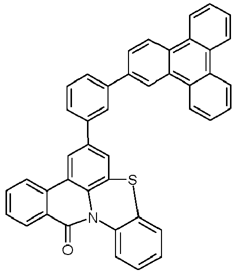 | 84% |
| 26c | 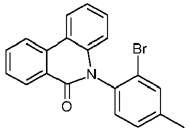 [88312-88-3] | 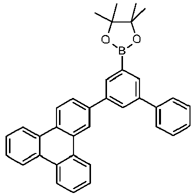 [1372893-20-3] | 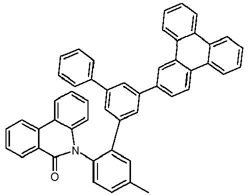 | 63% |
| 27c | 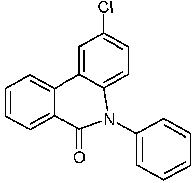 [101879-84-9] | 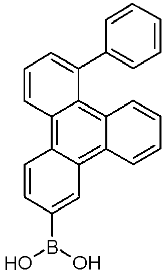 [2020404-50-4] | 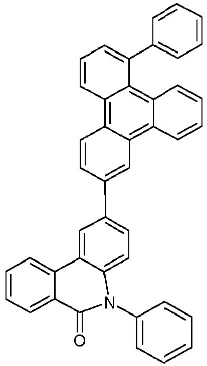 | 81% |
| 28C | 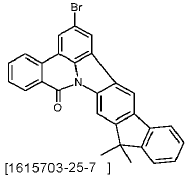 | 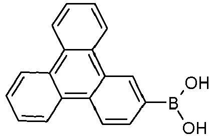 [654664-63-8] | 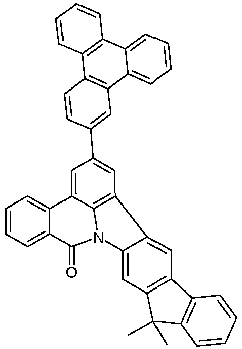 | 67% |
| 29c | 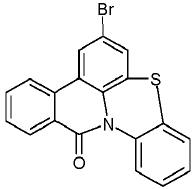 [1346571-45-6] | 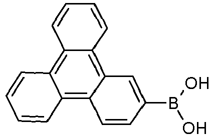 [654664-63-8] | 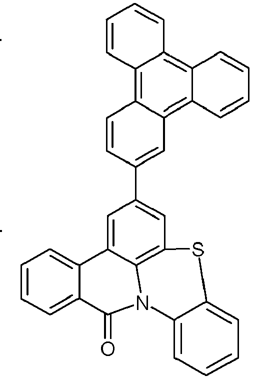 | 86% |
| 30c | 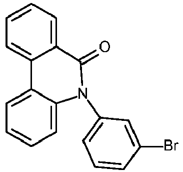 | 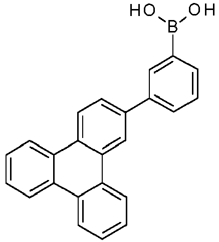 [1235876-72-8 ] | 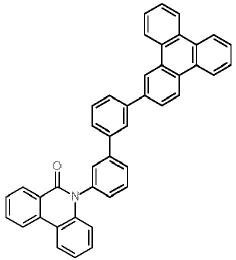 | 83% |
| 31c | 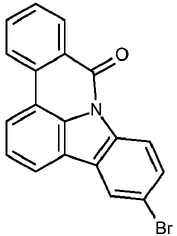 1346571-40-1] | 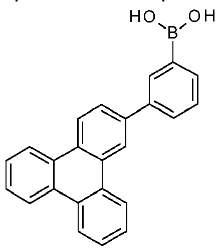 [1235876-72-8 ] | 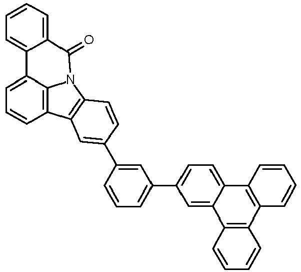 | 84% |

### A) Fabrication of OLEDs

The following examples V1 and E1 (see Table 1) show data of OLEDs.

### Substrate pre-treatment of examples V1 - E1:

Glass plates with structured ITO (50 nm, indium tin oxide) form the substrates on which the OLEDs are processed. Before evaporation of the OLED materials, the substrates are cleaned in a wet process (using filtered deionized water and the detergent "Extran" of Merck KGaA). Glass substrates are then dried for 15 minutes at 170°C.

Subsequently the clean and dry substrates are exposed to a oxygen and subsequently to an Argon plasma.

The OLEDs have in principle the following layer structure: substrate / hole-transport layer (HTL) / optional interlayer (IL) / electron-blocking layer (EBL) / emission layer (EML) / optional hole-blocking layer (HBL) / electron-transport layer (ETL) / optional electron-injection layer (EIL) and finally a cathode. The cathode is formed by an aluminium layer with a thickness of 100 nm. The exact layer structure is denoted in Table 1 (ITO and Aluminium layers are omitted for clarity). The materials used for the OLED fabrication are presented in Table 2.

All materials are applied by thermal vapour deposition in a vacuum chamber. The emission layer here always consists of at least one matrix material (host material) and an emitting dopant (emitter), which is admixed with the matrix material or matrix materials in a certain proportion by volume by co-evaporation. An expression such as CbzT1:SdT1:TEG1(41%:41%:18%) here means that material CbzT1 is present in the layer in a proportion by volume of 41%, SdT1 is present in the layer in a proportion of 41 % and TEG1 is present in the layer in a proportion of 18%. Analogously, the electron-transport layer may also consist of a mixture of two materials.

The OLEDs are characterized by standard methods.

For this purpose, the electroluminescence spectra and the lifetime are determined. The electroluminescent spectra are determined at a brightness of 1000 cd/m² and the corresponding CIE 1931 x and y color coordinates are determined.

Lifetime LT is defined as the time in hours (h), after which the starting brightness at constant current density j₀, is reduced to a certain level L1 in % of the starting brightness.

Here L1=80% means, that the given lifetime LT corresponds to the time after which the brightness is reduced to 80% of its starting value.

### Host materials in phosphorescent OLEDs

The compounds of formula (1) are used in the emission layer (EML) of a phosphorescent green OLED. The inventive combination of the compound CbzT1 with one of the compound EG1 to EG5 is used as matrix material in the emission layer.

In the following section several examples are described in more detail to show the advantages of the inventive OLEDs.

### Host material in phosphorescent OLEDs

By using the compounds according to the state of the art like in example V1 with high emitter concentrations in the EML of e.g. 18%, good color coordinates with x/y in the range of 0.33/0.63 can be reached. Nevertheless, the lifetime of such devices is not very high.

A substantial improvement of the lifetime by 25-30% versus the state of the art like in example V1 at similar color coordinates, can be reached for example by using the inventive combination of CbzT1 and one of the compound EG1 to EG5 and similar emitter concentrations (e.g. 18%) in the emission layer like in example E1.

**Table 1: Structure of the OLEDs**

| Ex | HIL Thickness | HTL Thickness | EBL Thickness | EML Thickness | HBL | ETL Thickness | ElL Thickness |
|---|---|---|---|---|---|---|---|
| V1 | HATCN 5nm | SpMA1 230nm | SpMA2 20nm | CbzT1 :SdT1 :TEG1 (41 °,6:41 °,6:18°,6) 30nm | ST2 10nm | ST2:LiQ (50%:50%) 30nm | LiQ 1nm |
| E1 | HATCN 5nm | SpMA1 230nm | SpMA2 20nm | CbzT1:EG1:TEG1 (41%:41%:18%) 30nm | ST2 10nm | ST2:LiQ (50%:50%) 30nm | LiQ 1nm |
| E2 | HATCN 5nm | SpMA1 230nm | SpMA2 20nm | CbzT1:EG2:TEG1 (41%:41°,6:18°,6) 30nm | ST2 10nm | ST2:LiQ (50%:50%) 30nm | LiQ 1nm |
| E3 | HATCN 5nm | SpMA1 230nm | SpMA2 20nm | CbzT1:EG3:TEG1 (41%:41°,6:18°,6) 30nm | ST2 10nm | ST2:LiQ (50%:50%) 30nm | LiQ 1nm |
| E4 | HATCN 5nm | SpMA1 230nm | SpMA2 20nm | CbzT1:EG4:TEG1 (41%:41°,6:18°,6) 30nm | ST2 10nm | ST2:LiQ (50%:50%) 30nm | LiQ 1nm |
| E5 | HATCN 5nm | SpMA1 230nm | SpMA2 20nm | CbzT1:EG5:TEG1 (41%:41°,6:18°,6) 30nm | ST2 10nm | ST2:LiQ (50%:50%) 30nm | LiQ 1nm |

**Table 2: Chemical structures of the materials used in the OLEDs**

| | |
|---|---|
| 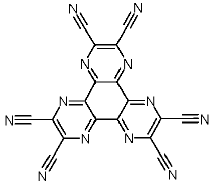 | 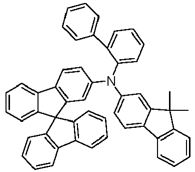 |
| HATCN | SpMA1 |
| 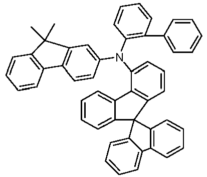 | 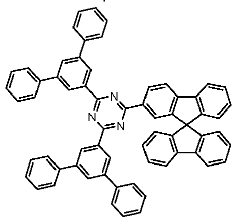 |
| SpMA2 | ST2 |
| 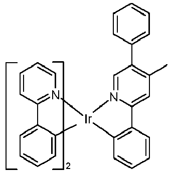 | 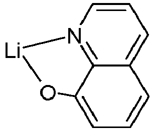 |
| TEG1 | LiQ |
| 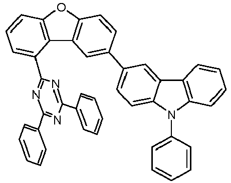 | 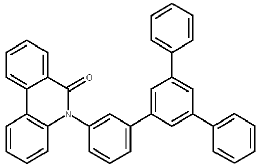 |
| CbzT1 | SdT1 |
| 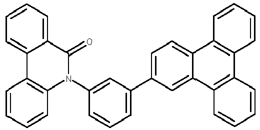 | 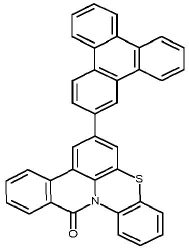 |
| EG1 | EG2 |
| 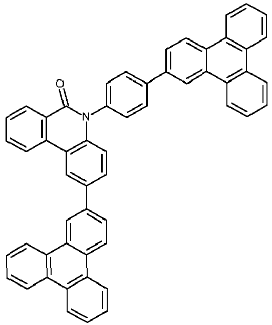 | 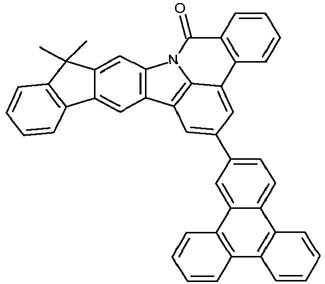 |
| EG3 | EG4 |
| 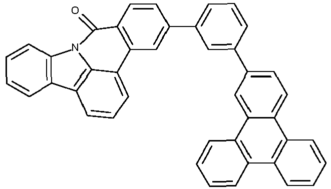 | |
| EG5 | |

| Table 3: OLEDs Data | | | | | | | |
|---|---|---|---|---|---|---|---|
| Ex.. | U1000 (V) | SE1000 (cd/A) | EQE 1000 (%) | CIE x/y bei 1000 cd/m² | L₀; j₀ | L₁ | LD |
| | | | | | | % | (h) |
| E1 | <3.7 | >57 | >15 | 0.32/0.62 | 20 mA/cm² | 80 | 90 |
| E2 | <3.6 | >60 | >18 | 0.3210.62 | 20 mA/cm² | 80 | 125 |
| E3 | <3.5 | >56 | >18 | 0.32/0.62 | 20 mA/cm² | 80 | 120 |
| E4 | <3.2 | >54 | >18 | 0.32/0.62 | 20 mA/cm² | 80 | 127 |
| E5 | <3.3 | >59 | >18 | 0.32/0.62 | 20 mA/cm² | 80 | 128 |

## Claims

1. Organic electroluminescent device comprising a mixture comprising a compound of the formula (1), 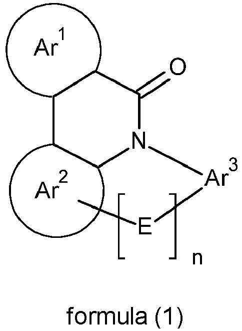 where the following applies to the symbols and indices used:
Ar¹ stands on each occurrence, identically or differently, for an aromatic or heteroaromatic ring system having 5 to 60 aromatic ring atoms, which may in each case be substituted by one or more radicals R¹;
Ar² stands on each occurrence, identically or differently, for an aromatic or heteroaromatic ring system having 5 to 60 aromatic ring atoms, which may in each case be substituted by one or more radicals R²;
Ar³ stands on each occurrence, identically or differently, for an aromatic or heteroaromatic ring system having 5 to 60 aromatic ring atoms, which may in each case be substituted by one or more radicals R³;
-E- is selected from a single bond, -B(R⁰)-, -C(R⁰)₂-, -Si(R⁰)₂-, -C(=O)-,-C(=NR⁰)-, -C(=C(R⁰)₂)-, -O-, -S-, -S(=O)-, -S(O₂)-, -N(R⁰)-, -P(R⁰)- and-P((=O)R⁰)-;
R⁰, R¹, R², R³ stand on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 40 aromatic ring atoms, which may be substituted by one or more radicals R, where two radicals R⁰, two radicals R¹, two radicals R² and/or two radicals R³ may form a monocyclic or polycyclic, aliphatic, aromatic or heteroaromatic ring system with one another, which may be substituted by one or more radicals R;
R stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R')₃, B(OR')₂, OSO₂R', a straight-chain alkyl, alkoxy or thioalkyl group having 1 to 40 C atoms or branched or cyclic alkyl, alkoxy or thioalkyl group having 3 to 40 C atoms, each of which may be substituted by one or more radicals R', where in each case one or more non-adjacent CH₂ groups may be replaced by R'C=CR', C=C, Si(R')₂, Ge(R)₂, Sn(R')₂, C=O, C=S, C=Se, P(=O)(R'), SO, SO₂, O, S or CONR' and where one or more H atoms may be replaced by D, F, Cl, Br, I, CN or NO₂, an aromatic or heteroaromatic ring systems having 5 to 60 aromatic ring atoms, which may in each case be substituted by one or more radicals R', or an aryloxy group having 5 to 40 aromatic ring atoms, which may be substituted by one or more radicals R', where two radicals R may form a ring system with one another, which may be substituted by one or more radicals R';
Ar is an aromatic or heteroaromatic ring system having 5 to 24 aromatic ring atoms, which may in each case also be substituted by one or more radicals R';
R' stands on each occurrence, identically or differently, for H, D, F, Cl, Br, I, CN, a straight-chain alkyl, alkoxy or thioalkyl group having 1 to 20 C atoms or branched or cyclic alkyl, alkoxy or thioalkyl group having 3 to 20 C atoms, where in each case one or more non-adjacent CH₂ groups may be replaced by SO, SO₂, O, S and where one or more H atoms may be replaced by D, F, Cl, Br or I, or an aromatic or heteroaromatic ring system having 5 to 24 C atoms;
n is 0 or 1; wherein when n is 0, the group -E- is absent;
where the compound of formula (1) comprises at least one group Ar³, R¹, R² or R³, which stand for a triphenylene derivative of the formula (T), 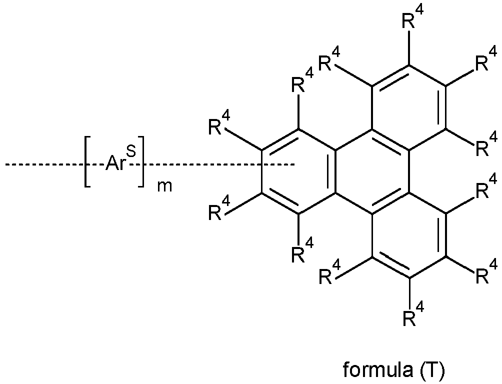
where the dashed bond indicates the bonding to the structure of formula (1), and where:
R⁴ is C at the position of the bonding to Ar^{S} or, R⁴ is C at the position of the bonding to the structure of formula (1) if m is 0; and at other positions, R⁴ stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 40 aromatic ring atoms, which may be substituted by one or more radicals R, where two radicals R⁴ may form a monocyclic or polycyclic, aliphatic, aromatic or heteroaromatic ring system, which may be substituted by one or more radicals R;
Ar^{S} is an aromatic or heteroaromatic ring system having 5 to 60 aromatic ring atoms, which may in each case also be substituted by one or more radicals R; and
m is an integer selected from 0, 1, 2, 3 or 4; and
and a second compound selected from compounds of formulae (11) and (12), 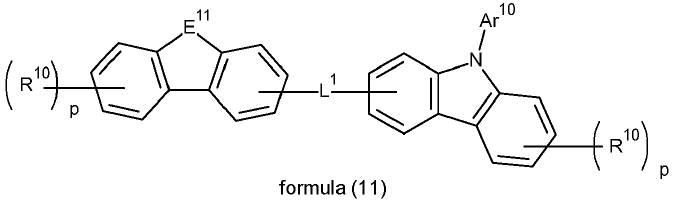 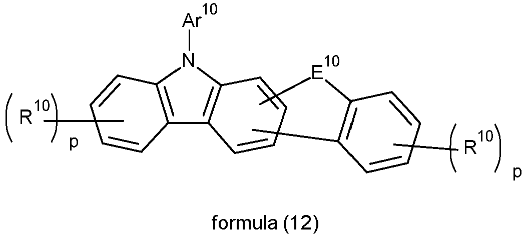
where:
R¹⁰ stands on each occurrence, identically or differently, for H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, a straight-chain alkyl, alkoxy or thioalkyl group having 1 to 40 C atoms or branched or a cyclic alkyl, alkoxy or thioalkyl group having 3 to 40 C atoms, each of which may be substituted by one or more radicals R, where in each case one or more non-adjacent CH₂ groups may be replaced by RC=CR, C=C, Si(R)₂, Ge(R)₂, Sn(R)₂, C=O, C=S, C=Se, P(=O)(R), SO, SO₂, O, S or CONR and where one or more H atoms may be replaced by D, F, Cl, Br, I, CN or NO₂, an aromatic or heteroaromatic ring systems having 5 to 60 aromatic ring atoms, which may in each case be substituted by one or more radicals R, or an aryloxy group having 5 to 40 aromatic ring atoms, which may be substituted by one or more radicals R, where two radicals R¹⁰ may form a monocyclic or polycyclic, aliphatic, aromatic or heteroaromatic ring system with one another, which may be substituted by one or more radicals R;
Ar¹⁰ is an aromatic or heteroaromatic ring system having 5 to 60 aromatic ring atoms, which may in each case also be substituted by one or more radicals R;
p is an integer selected from 0, 1, 2, 3 and 4;
E¹⁰ stands for -C(R⁰)₂-, -Si(R⁰)₂-, -C(=O)-, -C(=NR⁰)-, -C(=C(R⁰)₂)-, -O-, -S-, -S(=O)-, -S(O₂)-, -N(Ar¹⁰)-, -P(R⁰)- or -P((=O)R⁰)-;
E¹¹ stands for -C(R⁰)₂-, -Si(R⁰)₂-, -C(=O)-, -C(=NR⁰)-, -C(=C(R⁰)₂)-, -O-, -S-, -S(=O)-, -S(O₂)-, -N(Ar¹⁰)-, -P(R⁰)- or -P((=O)R⁰)-; and
L¹ is a single bond or an aromatic or heteroaromatic ring system having 5 to 60 aromatic ring atoms, which may in each case also be substituted by one or more radicals R,
where the mixture is employed as a matrix material for phosphorescent emitters in a light-emitting layer; and
where the following compounds are excluded from the compounds of formula (1):

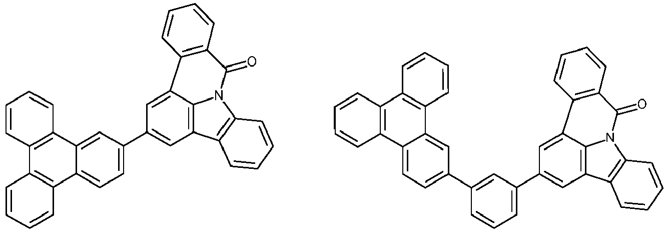

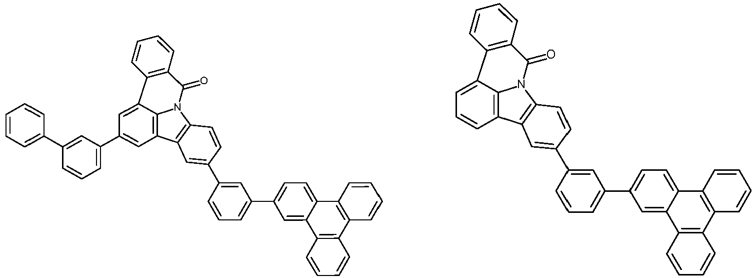

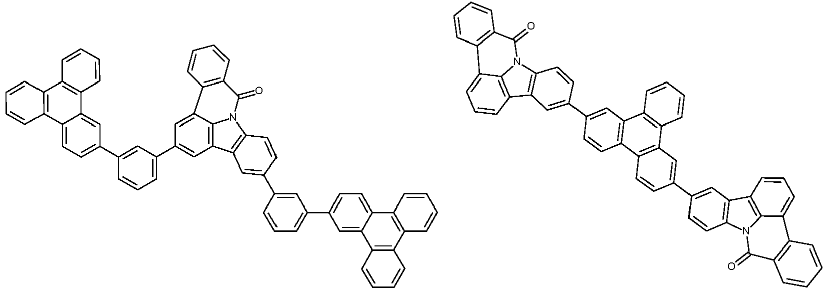

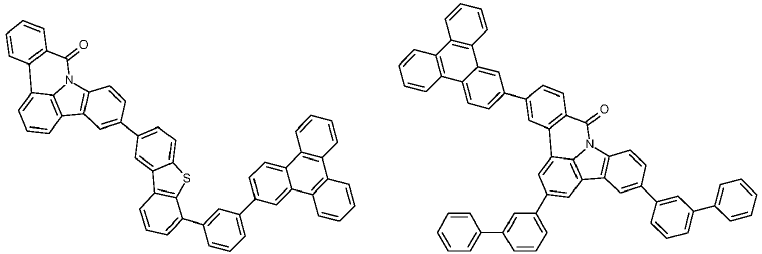

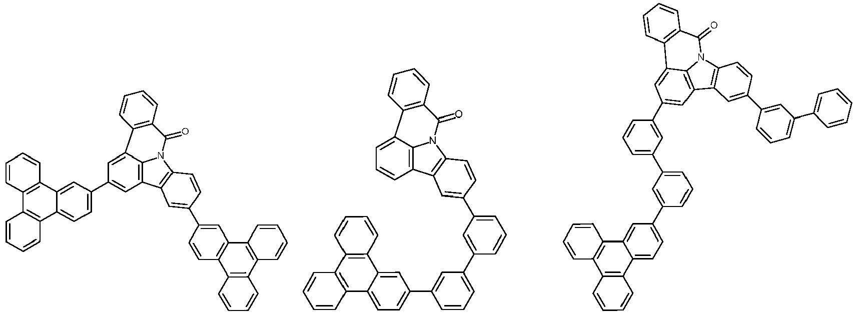

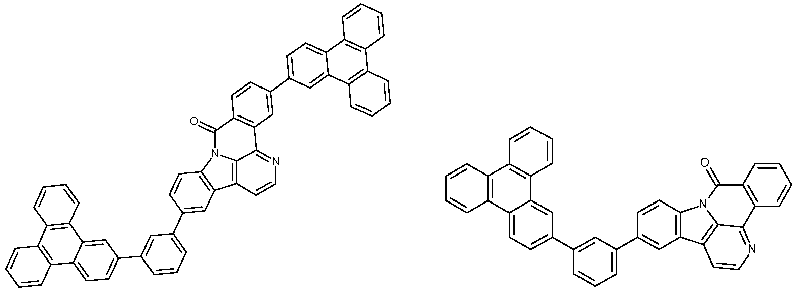

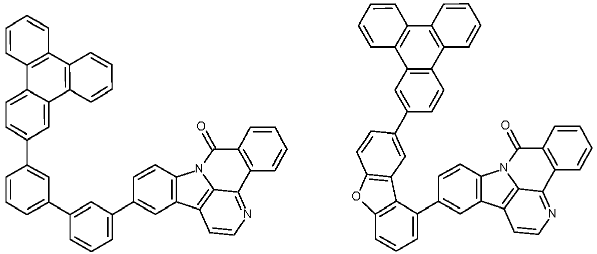

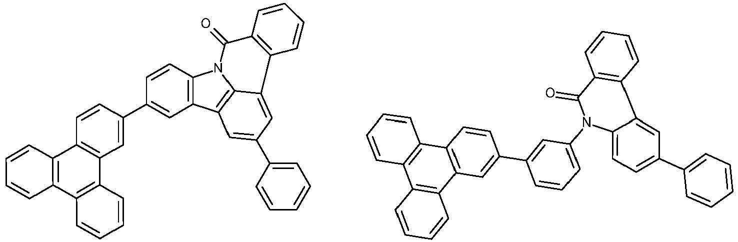

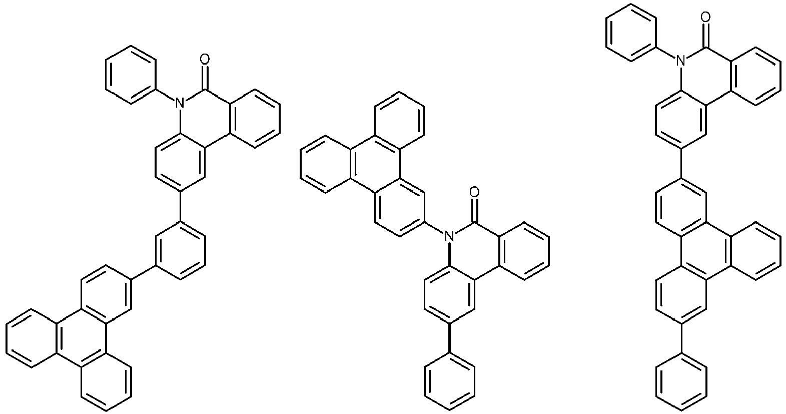

2. Organic electroluminescent device according to claim 1, **characterized in that** the compound of formula (1) is selected from compounds of formulae (1A) or (1B), 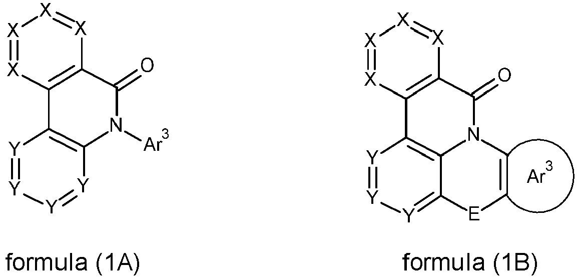 where
the symbol -E- has the same meaning as in claim 1;
X is CR¹ or N;
Y is CR² or N;
where the symbols R¹, R² have the same meaning as in claim 1; and
Ar³ has the same meaning as in claim 1, with the proviso that Ar³, in formula (1B), is connected to the group -E- and the atom N via two adjacent C atoms as represented in formula (1B);
and where in formula (1A), at least one group Ar³, R¹ or R² stands for a triphenylene derivative of the formula (T) and, in formula (1B), at least one group R¹ or R² stands for a triphenylene derivative of the formula (T), where the triphenylene derivative of the formula (T) is as defined in claim 1.

3. Organic electroluminescent device according to claim 1 or 2, **characterized in that** the compound of formula (1) is selected from the compounds of formulae (2), (3), (4), (5), (6) or (7), 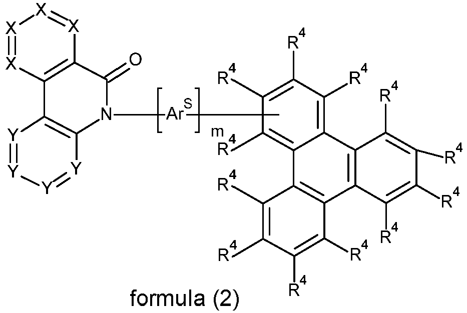 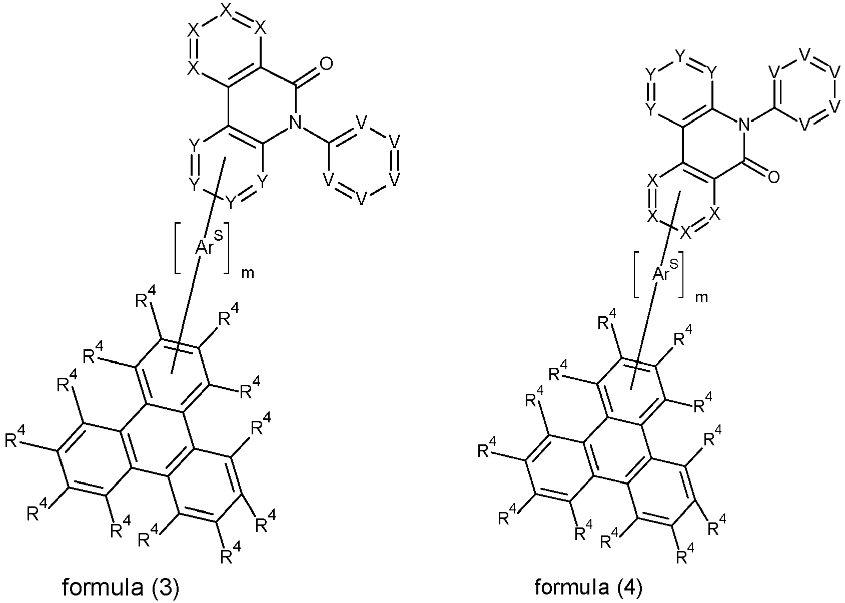 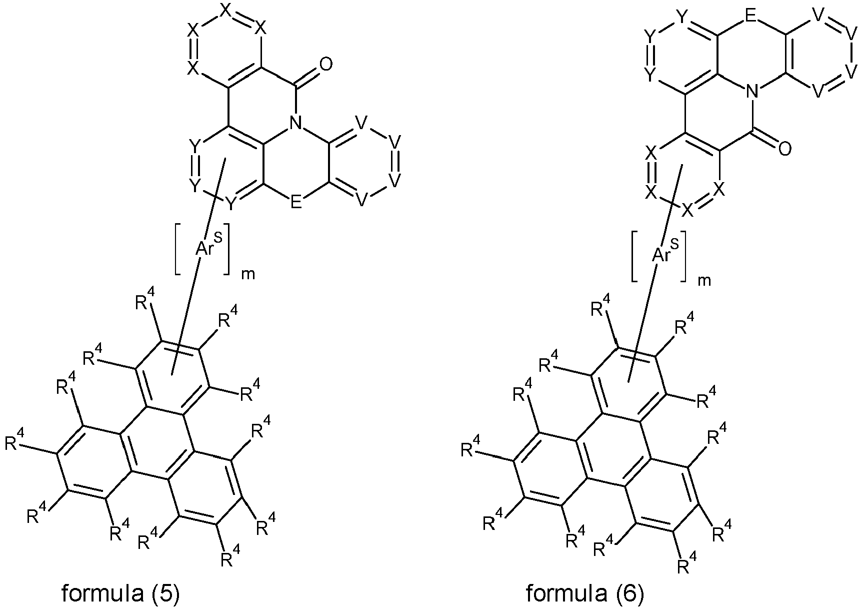 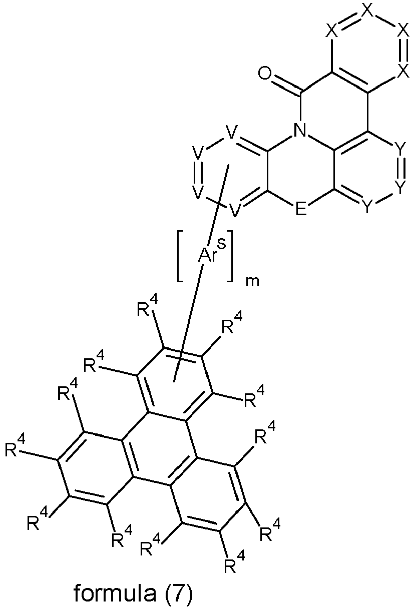
where the symbols and indices E, R⁴, Ar^{S} and m have the same meaning as in claim 1; and where
X is CR¹ or N, with the proviso that X is C at the position of the bonding to Ar^{S} or, if m is 0 and Ar^{S} is absent, to the triphenylene group in formulae (4) and (6);
Y is CR² or N, with the proviso that Y is C at the position of the bonding to Ar^{S} or, if m is 0 and Ar^{S} is absent, to the triphenylene group in formulae (3) and (5);
V is CR³ or N, with the proviso that V is C at the position of the bonding to Ar^{S} or, if m is 0 and Ar^{S} is absent, to the triphenylene group in formula (7); or two adjacent groups V stand for a group of formula (V-1) or (V-2), 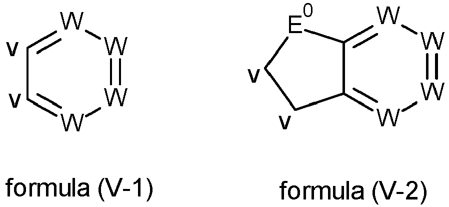
where the symbols ^{V} indicate the corresponding adjacent groups V in formulae (2) to (7);
E⁰ stands for -C(R⁰)₂-, -Si(R⁰)₂-, -C(=O)-, -C(=NR⁰)-, -C(=C(R⁰)₂)-, -O-, -S-, -S(=O)-, -S(O₂)-, -N(R⁰)-, -P(R⁰)- or -P((=O)R⁰)-; where R⁰ has the same meaning as in claim 1; and
W stands for CR or N.

4. Organic electroluminescent device according to one or more of the preceding claims, **characterized in that** the index m is equal to 0 or 1.

5. Organic electroluminescent device according to one or more of the preceding claims, **characterized in that** the group Ar^{S} stands on each occurrence, identically or differently, for phenyl, biphenyl, fluorene, spirobifluorene, naphthalene, phenanthrene, anthracene, dibenzofuran, dibenzothiophene, carbazole, pyridine, pyrimidine, pyrazine, pyridazine, triazine, benzopyridine, benzopyridazine, benzopyrimidine and quinazoline, each of which may be substituted by one or more radicals R.

6. Organic electroluminescent device according to one or more of the preceding claims, **characterized in that** the group Ar^{S} stands on each occurrence, identically or differently, for phenyl, biphenyl, fluorene, naphthalene, dibenzofuran, dibenzothiophene and carbazole, each of which may be substituted by one or more radicals R.

7. Organic electroluminescent device according to one or more of the claims 2 to 6, **characterized in that**, in the compound of formula (1), there is 0 or 1 group X standing for N in the six-membered ring comprising the groups X, there is 0 or 1 group Y standing for N in the six-membered ring comprising the groups Y and there is 0 or 1 group V in the six-membered ring comprising the groups V.

8. Organic electroluminescent device according to one or more of the preceding claims, **characterized in that** the compound of formula (1) is selected from compounds of formulae (2A) to (7B), 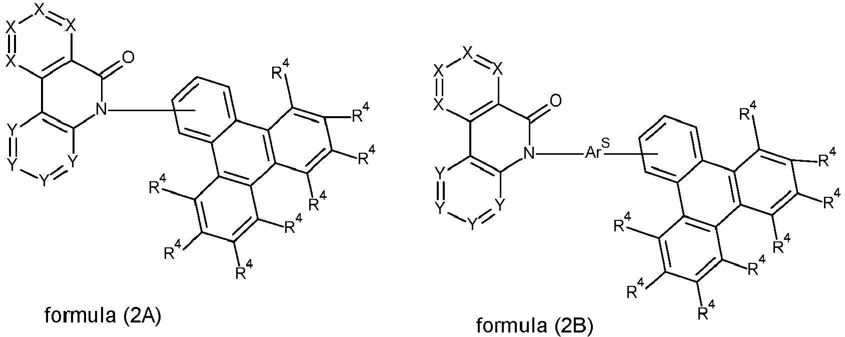 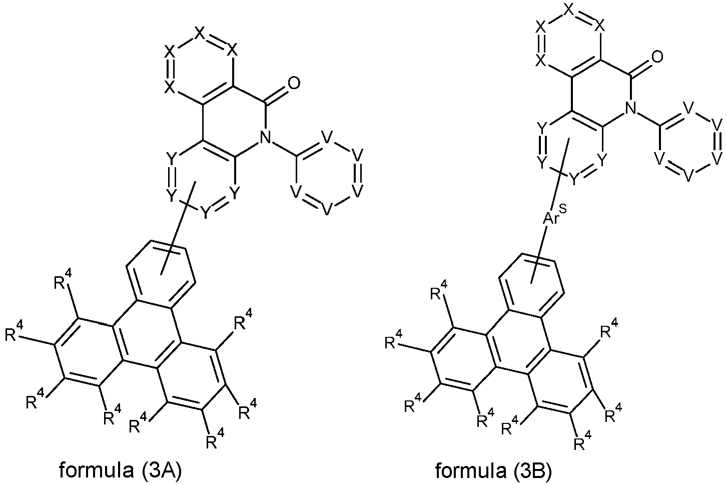 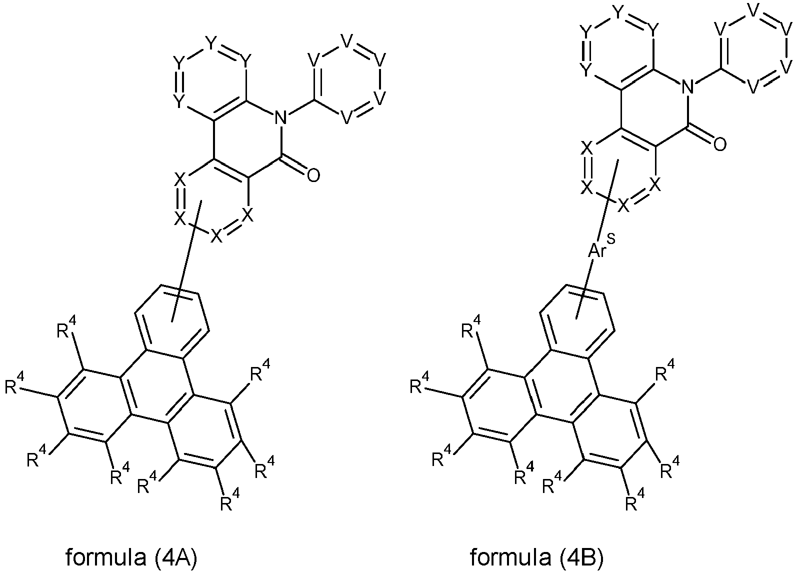 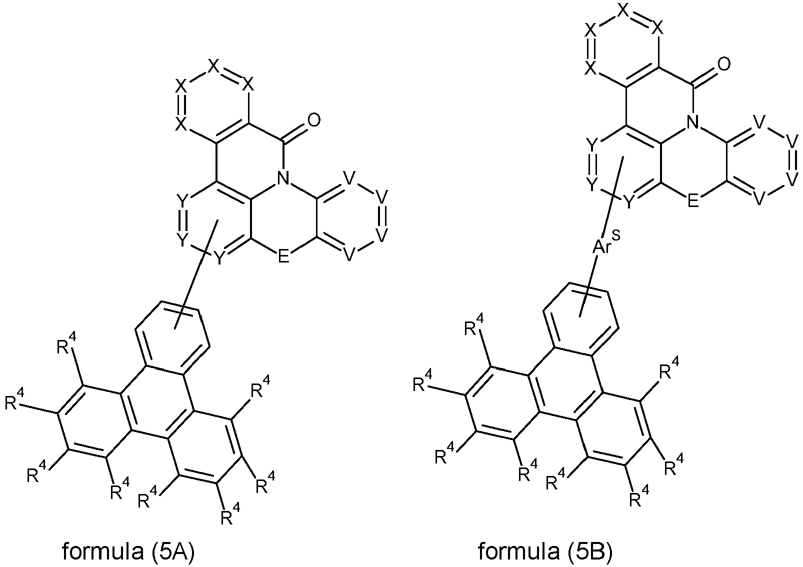 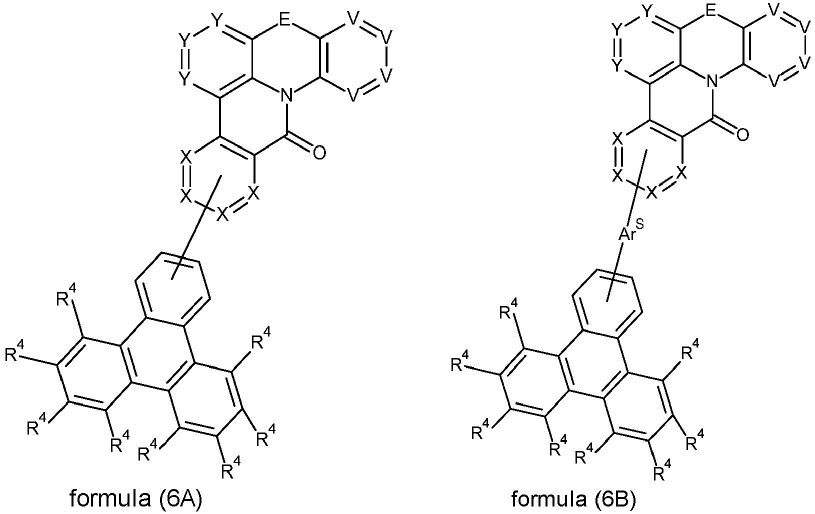 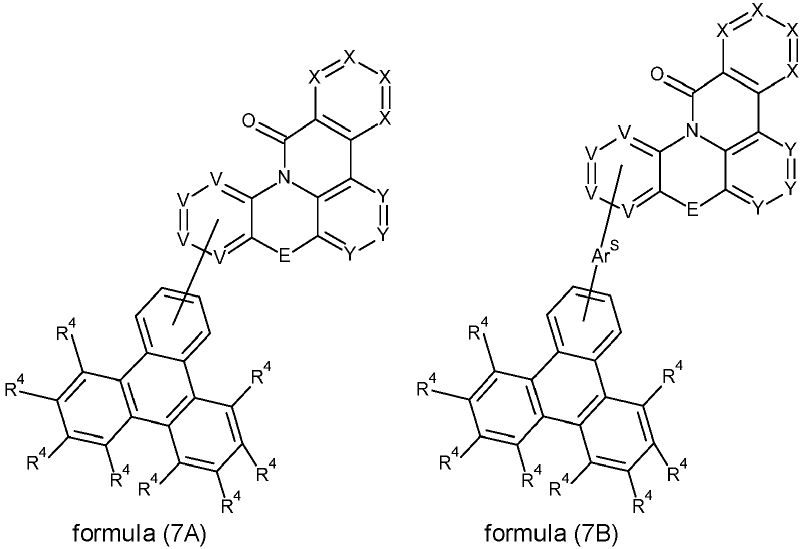 where the symbols E, Ar^{S} and R⁴ have the same meaning as in claim 1, and the symbols X, Y and V have the same meaning as in claim 3.

9. Organic electroluminescent device according to one or more of the preceding claims, **characterized in that** the compound of formula (1) is selected from compounds (2A-1) to (7B-1), 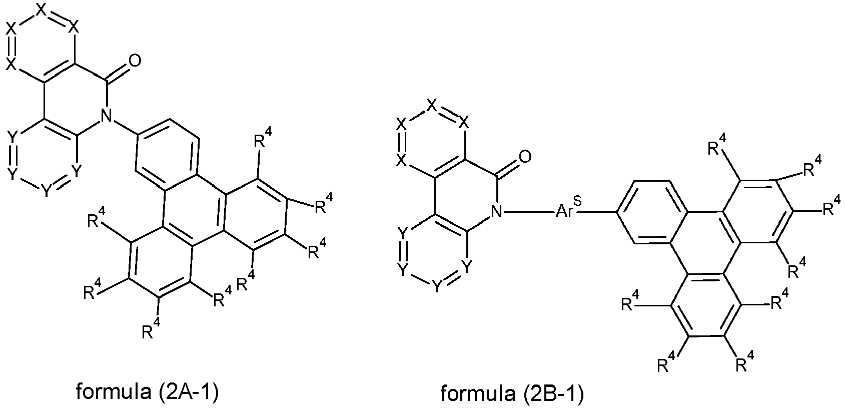 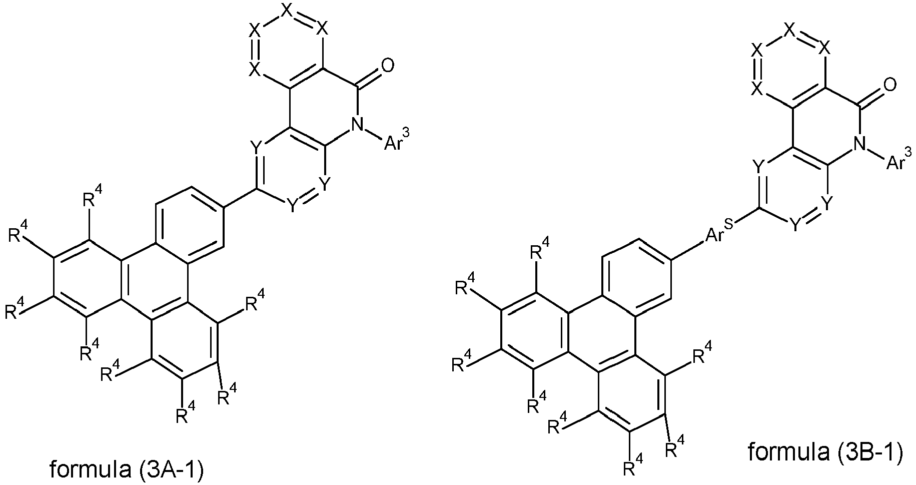 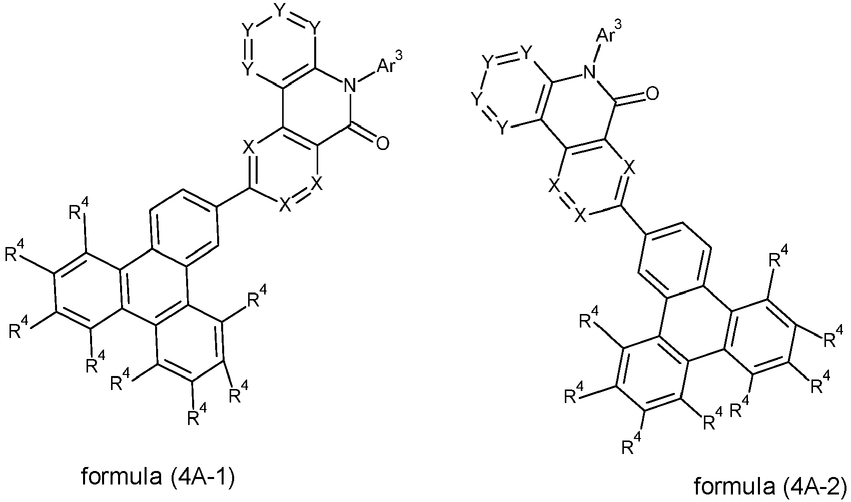 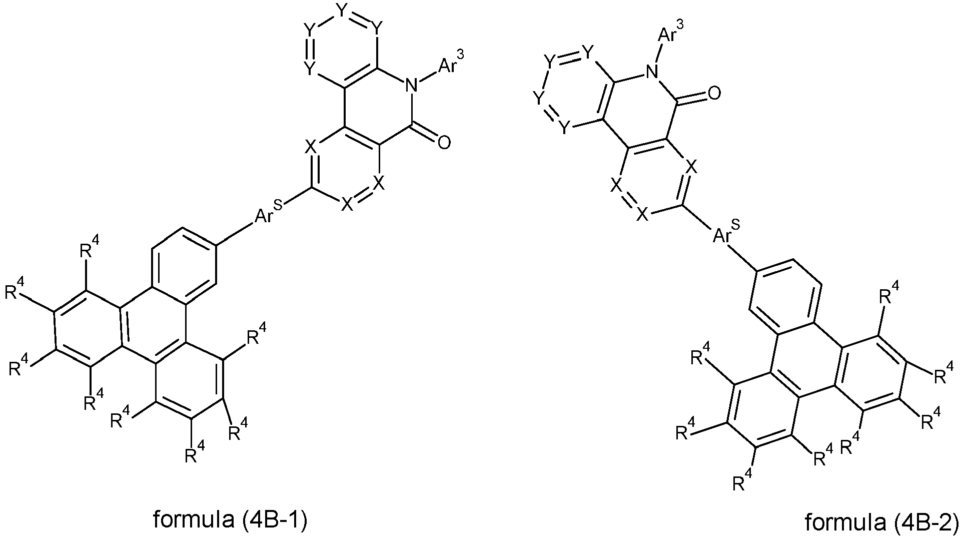 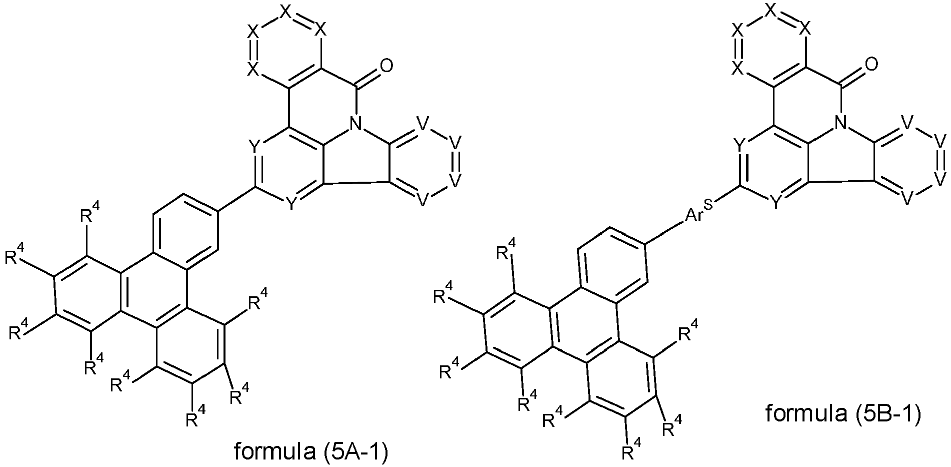 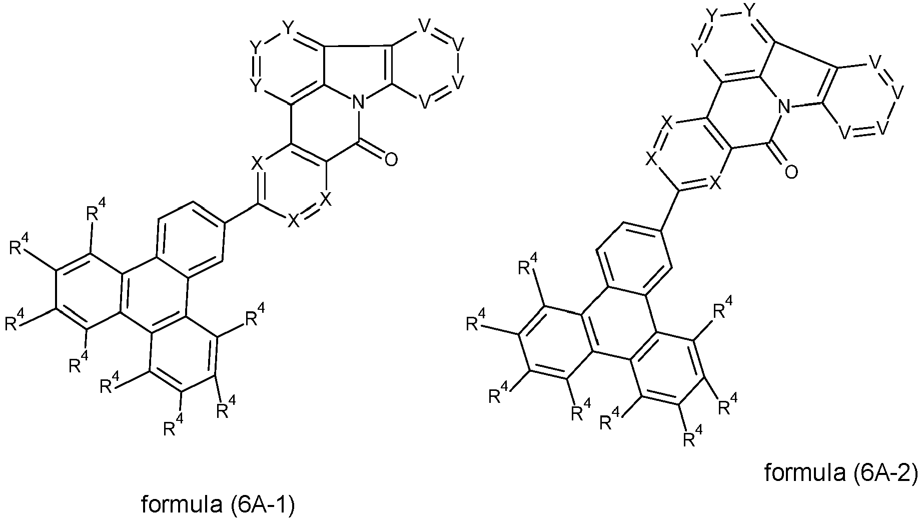 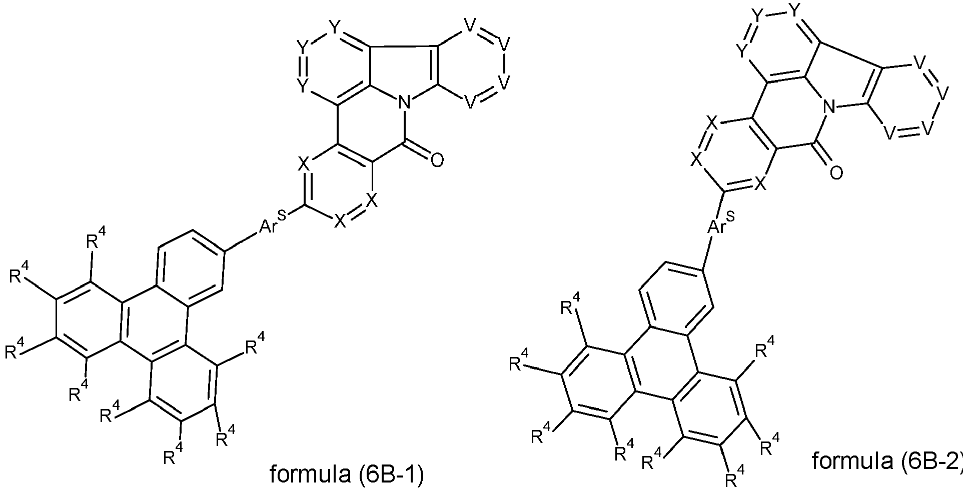 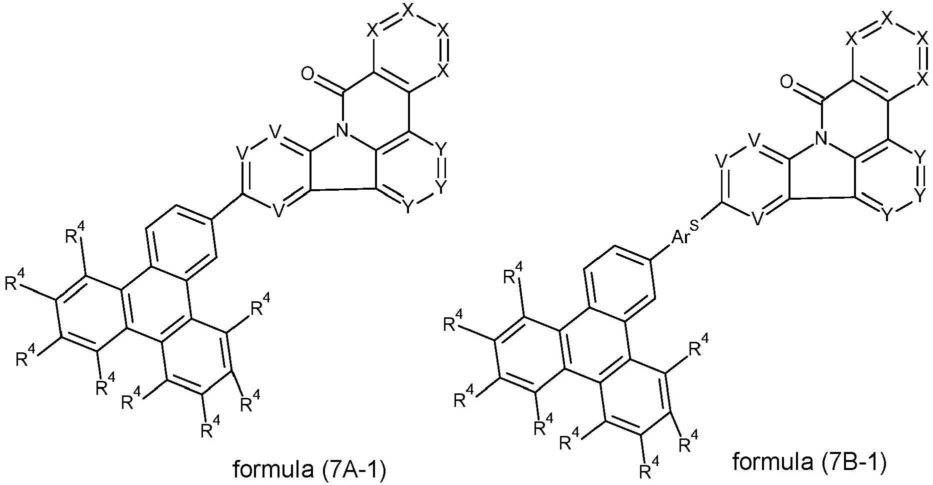 where the symbol R⁴ has the same meaning as in claim 1 and the symbols X, Y, V have the same meaning as in claim 3.

10. Organic electroluminescent device according to one or more of the preceding claims, **characterized in that** the compound of formula (1) comprises at least one of the substituent R¹ or R², which is selected from aromatic and heteroaromatic ring systems.

11. Organic electronic device according to one or more of the preceding claims, **characterized in that** the second compound is selected from compounds of formulae (11-1) to (12-4), 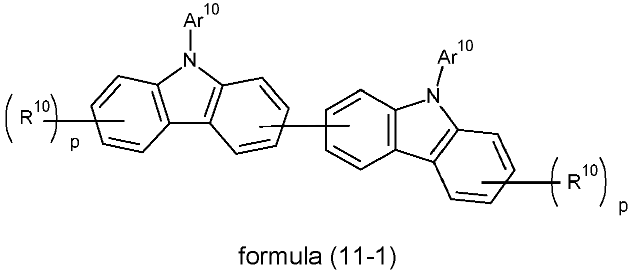 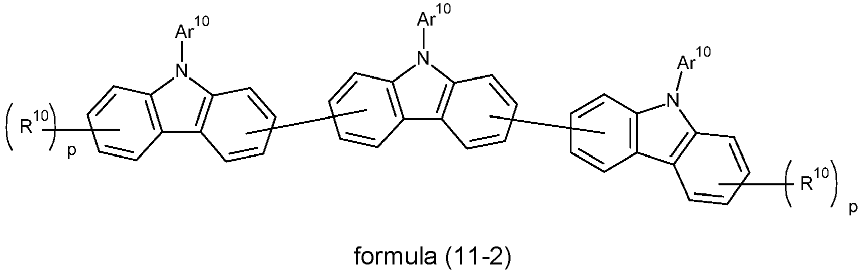 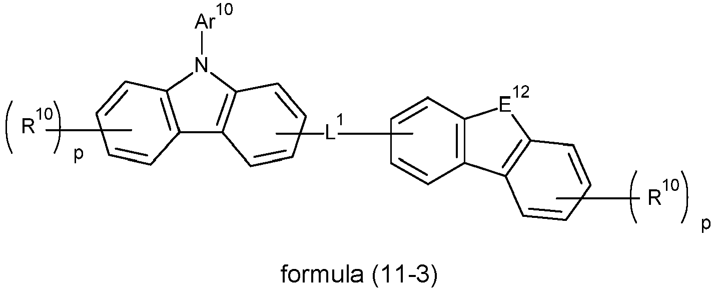 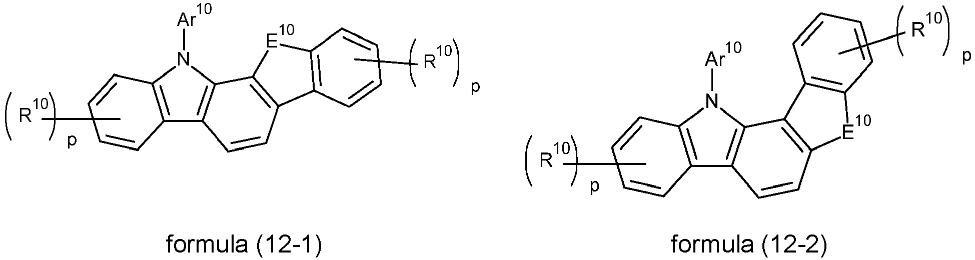 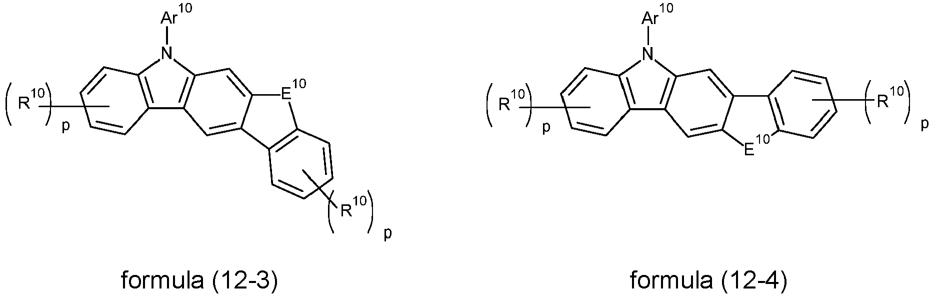
where the symbols and indices Ar¹⁰, R¹⁰, E¹⁰, L¹ and p have the same meaning as in claim 1; and
E¹² stands for -C(R⁰)₂-, -O-, -S-, -N(Ar¹⁰)-; where R⁰ has the same meaning as in claim 1.

12. Organic electronic device according to one or more of the preceding claims, **characterized in that** the second component is selected from compounds of formulae (13-1) to (14-4), 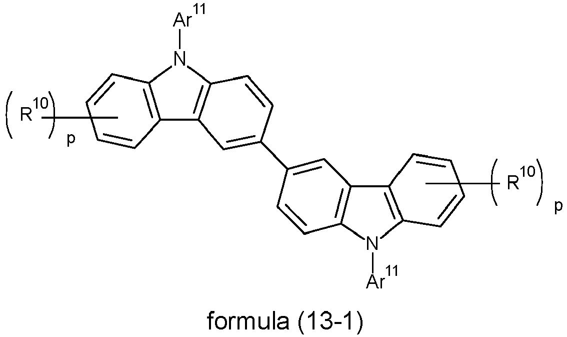 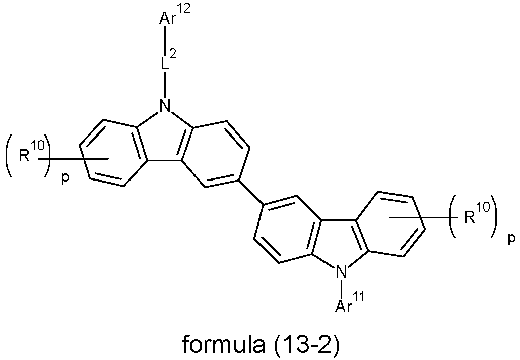 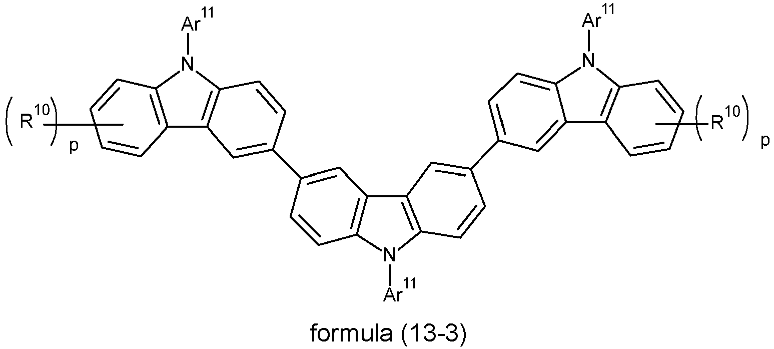 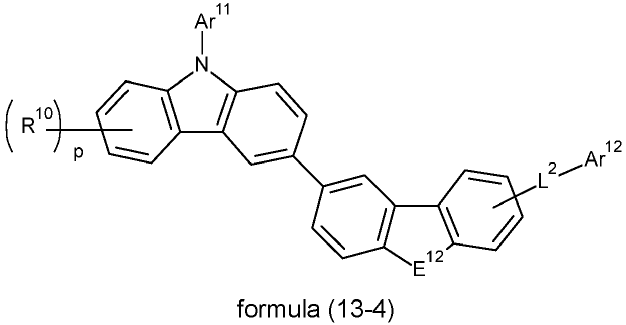 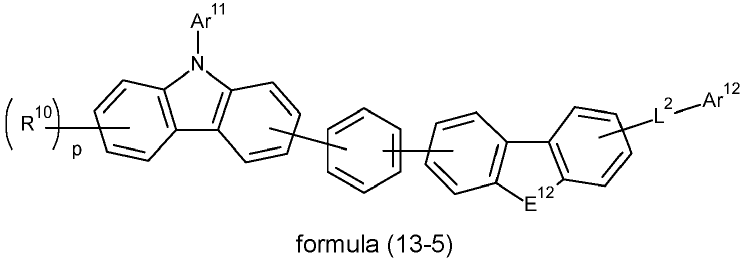 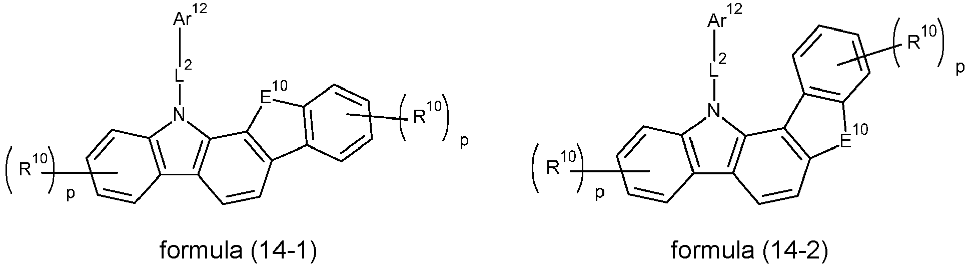 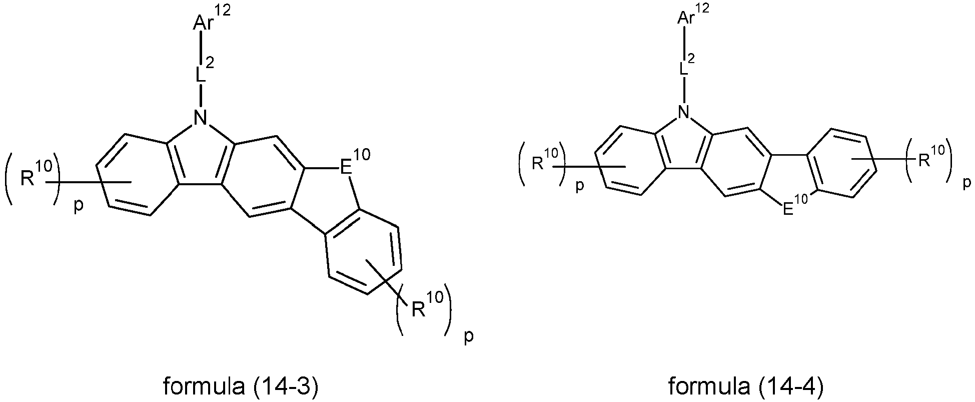 where the symbols and indices R¹⁰, E¹⁰ and p have the same meaning as in claim 11, the symbol E¹² has the same meaning as in claim 13; and
Ar¹⁰ stands for an aromatic or heteroaromatic ring system selected from benzene, naphthalene, pyrene, biphenyl, terphenyl, quaterphenyl, fluorene, spirobifluorene, cis- or trans-indenofluorene, dibenzofuran, dibenzothiophene, carbazole, indolocarbazole, indenocarbazole, which may be substituted by one or more radicals R; or combinations of these groups;
L² stands for a single bond or an aromatic or heteroaromatic rings system having 6 to 18 aromatic rings atoms, which may be substituted by one or more radicals R;
Ar¹² stands for an heteroaromatic ring system selected from pyridine, pyrazine, pyrimidine, triazine, which may be substituted by one or more radicals R;
R has the same meaning as in claim 1.

## Patentansprüche

1. Organische Elektrolumineszenzvorrichtung, umfassend eine Mischung, umfassend eine Verbindung der Formel (1), 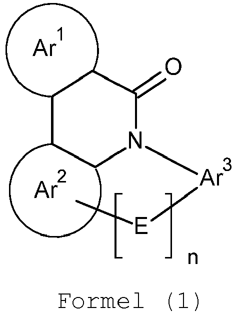 wobei für die verwendeten Symbole und Indizes Folgendes gilt:
Ar¹ steht bei jedem Auftreten gleich oder verschieden für ein aromatisches oder heteroaromatisches Ringsystem mit 5 bis 60 aromatischen Ringatomen, das jeweils durch einen oder mehrere Reste R¹ substituiert sein kann;
Ar² steht bei jedem Auftreten gleich oder verschieden für ein aromatisches oder heteroaromatisches Ringsystem mit 5 bis 60 aromatischen Ringatomen, das jeweils durch einen oder mehrere Reste R² substituiert sein kann;
Ar³ steht bei jedem Auftreten gleich oder verschieden für ein aromatisches oder heteroaromatisches Ringsystem mit 5 bis 60 aromatischen Ringatomen, das jeweils durch einen oder mehrere Reste R³ substituiert sein kann;
-E- ist aus einer Einfachbindung, -B(R⁰)-, -C(R⁰)₂-,-Si(R⁰)₂-, -C(=O)-, -C(=NR⁰)-, -C(=C(R⁰)₂)-, -O-, -S-, -S(=O)-, -S(O₂)-, -N(R⁰)-, -P(R⁰)- und -P((=O)R⁰)-ausgewählt;
R⁰, R¹, R², R³ stehen bei jedem Auftreten gleich oder verschieden für H, D, F, Cl, Br, I, CHO, CN, N(Ar)₂, C(=O)Ar, P(=O) (Ar)₂, S(=O)Ar, S(=O)₂Ar, NO₂, Si(R)₃, B(OR)₂, OSO₂R, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O) (R), SO, SO₂, O, S oder CONR ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 40 aromatischen Ringatomen, die durch einen oder mehrere Reste R substituiert sein kann, wobei zwei Reste R⁰, zwei Reste R¹, zwei Reste R² und/oder zwei Reste R³ miteinander ein monocyclisches oder polycyclisches, aliphatisches, aromatisches oder heteroaromatisches Ringsystem, das durch einen oder mehrere Reste R substituiert sein kann, bilden können;
R steht bei jedem Auftreten gleich oder verschieden für H, D, F, Cl, Br, I, CHO, CN, N(Ar)₂, C(=O)Ar, P(=O) (Ar)₂, S(=O)Ar, S(=O) ₂Ar, NO₂, Si(R')₃, B(OR')₂, OSO₂R', eine geradkettige Alkyl-, Alkoxy- oder Thioalkylgruppe mit 1 bis 40 C-Atomen oder verzweigte oder cyclische Alkyl-, Alkoxy- oder Thioalkylgruppe mit 3 bis 40 C-Atomen, wobei jede dieser Gruppen durch einen oder mehrere Reste R' substituiert sein kann, wobei jeweils eine oder mehrere nicht benachbarte CH₂-Gruppen durch R'C=CR', C=C, Si(R')₂, Ge(R')₂, Sn(R')₂, C=O, C=S, C=Se, P(=O)(R'), SO, SO₂, O, S oder CONR' ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, die jeweils durch einen oder mehrere Reste R' substituiert sein können, oder eine Aryloxygruppe mit 5 bis 40 aromatischen Ringatomen, die durch einen oder mehrere Reste R' substituiert sein kann, wobei zwei Reste R miteinander ein Ringsystem, das durch einen oder mehrere Reste R' substituiert sein kann, bilden können;
Ar ist ein aromatisches oder heteroaromatisches Ringsystem mit 5 bis 24 aromatischen Ringatomen, das jeweils auch durch einen oder mehrere Reste R' substituiert sein kann;
R' steht bei jedem Auftreten gleich oder verschieden für H, D, F, Cl, Br, I, CN, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen, wobei jeweils eine oder mehrere nicht benachbarte CH₂-Gruppen durch SO, SO₂, O, S ersetzt sein können und wobei ein oder mehrere H-Atome durch D, F, Cl, Br oder I ersetzt sein können, oder ein aromatisches oder heteroaromatisches Ringsystem mit 5 bis 24 C-Atomen;
n ist 0 oder 1; wobei dann, wenn n 0 ist, die Gruppe -E- fehlt;
wobei die Verbindung der Formel (1) mindestens eine Gruppe Ar³, R¹, R² oder R³ umfasst, die für ein Triphenylenderivat der Formel (T) steht, 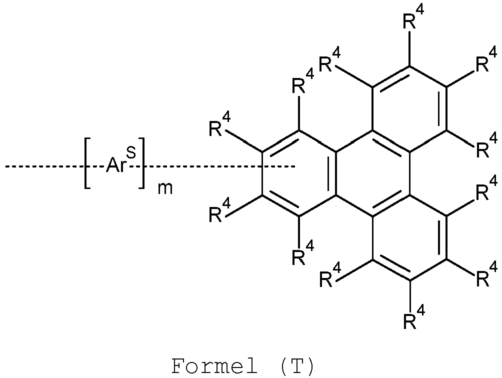
wobei die gestrichelte Bindung die Anbindung an die Struktur der Formel (1) anzeigt und wobei:
R⁴ C an der Position der Anbindung an Ar^{S} ist oder R⁴ C an der Position der Anbindung an die Struktur der Formel (1) ist, wenn m 0 ist; und an anderen Positionen R⁴ bei jedem Auftreten gleich oder verschieden für H, D, F, Cl, Br, I, CHO, CN, N(Ar)₂, C(=O)Ar, P(=O) (Ar)₂, S(=O)Ar, S(=O)₂Ar, NO₂, Si(R)₃, B(OR)₂, OSO₂R, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O) (R), SO, SO₂, O, S oder CONR ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, das jeweils durch einen oder mehrere Reste R substituiert sein kann, oder eine Aryloxygruppe mit 5 bis 40 aromatischen Ringatomen, die durch einen oder mehrere Reste R substituiert sein kann, steht, wobei zwei Reste R⁴ ein monocyclisches oder polycyclisches, aliphatisches, aromatisches oder heteroaromatisches Ringsystem, das durch einen oder mehrere Reste R substituiert sein kann, bilden können;
Ar^{S} für ein aromatisches oder heteroaromatisches Ringsystem mit 5 bis 60 aromatischen Ringatomen steht, das jeweils auch durch einen oder mehrere Reste R substituiert sein kann; und
m eine ganze Zahl ist, die aus 0, 1, 2, 3 oder 4 ausgewählt ist; und
eine zweite Verbindung, die aus Verbindungen der Formel (11) und (12) ausgewählt ist, 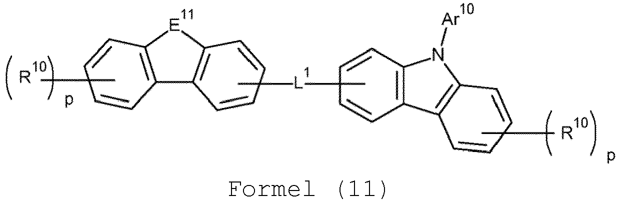 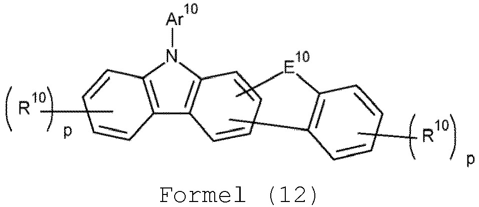
wobei:
R¹⁰ bei jedem Auftreten gleich oder verschieden für H, D, F, Cl, Br, I, CHO, CN, N(Ar)₂, C(=O)Ar, P(=O) (Ar)₂, S(=O)Ar, S(=O)₂Ar, NO₂, Si(R)₃, B(OR)₂, OSO₂R, eine geradkettige Alkyl-, Alkoxy- oder Thioalkylgruppe mit 1 bis 40 C-Atomen oder verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte CH₂-Gruppen durch RC=CR, C=C, Si(R)₂, Ge(R)₂, Sn(R)₂, C=O, C=S, C=Se, P(=O) (R), SO, SO₂, O, S oder CONR ersetzt sein können und wobei ein oder mehrere H-Atome durch D, F, Cl, Br, I, CN oder NO₂ ersetzt sein können, ein aromatisches oder heteroaromatisches Ringsystem mit 5 bis 60 aromatischen Ringatomen, die jeweils durch einen oder mehrere Reste R substituiert sein können, oder eine Aryloxygruppe mit 5 bis 40 aromatischen Ringatomen, die durch einen oder mehrere Reste R substituiert sein kann, steht, wobei zwei Reste R¹⁰ miteinander ein monocyclisches oder polycyclisches aliphatisches, aromatisches oder heteroaromatisches Ringsystem, das durch einen oder mehrere Reste R substituiert sein kann, bilden können;
Ar¹⁰ ein aromatisches oder heteroaromatisches Ringsystem mit 5 bis 60 aromatischen Ringatomen ist, das jeweils auch durch einen oder mehrere Reste R substituiert sein kann;
p eine ganze Zahl ist, die aus 0, 1, 2, 3 und 4 ausgewählt ist;
E¹⁰ für -C(R⁰)₂-, -Si(R⁰)₂-, -C(=O)-, -(=NR⁰)-,-C(=C(R⁰)₂)-, -O-, -S-, -S(=O)-, -S(O₂)-, -N(Ar¹⁰)-,-P(R⁰)- oder -P((=O)R⁰)- steht;
E¹¹ für -C(R⁰)₂-, -Si(R⁰)₂-, -C(=O)-, -C(=NR⁰)-,-C(=C(R⁰)₂)-, -O-, -S-, -S(=O)-, -S(O₂)-, -N(Ar¹⁰)-,-P(R⁰)- oder -P((=O)R⁰)- steht; und
L¹ eine Einfachbindung oder ein aromatisches oder heteroaromatisches Ringsystem mit 5 bis 60 aromatischen Ringatomen, das jeweils auch durch einen oder mehrere Reste R substituiert sein kann, ist,
wobei die Mischung als Matrixmaterial für phosphoreszierende Emitter in einer lichtemittierenden Schicht eingesetzt wird; und
wobei die folgenden Verbindungen aus den Verbindungen der Formel (1) ausgeschlossen sind:

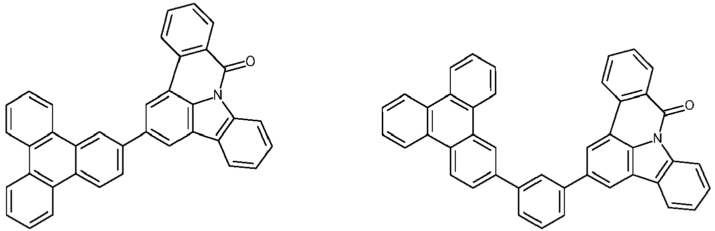

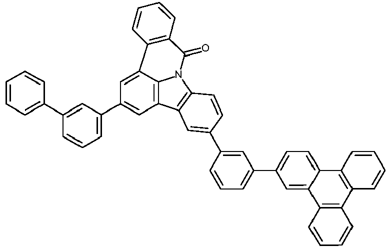

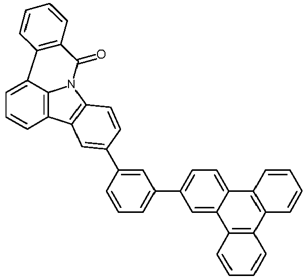

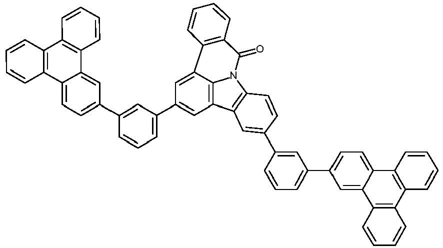

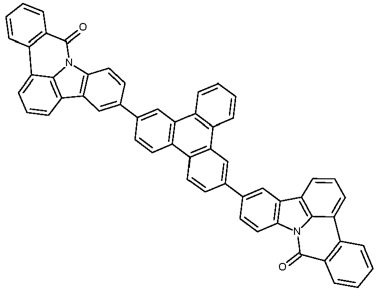

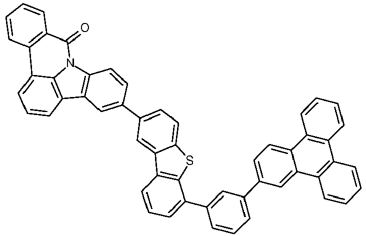

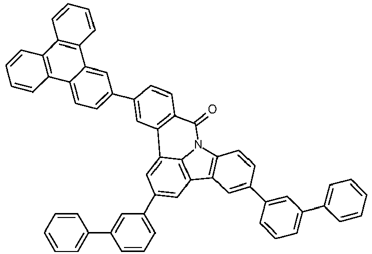

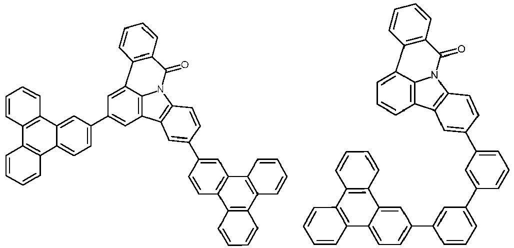

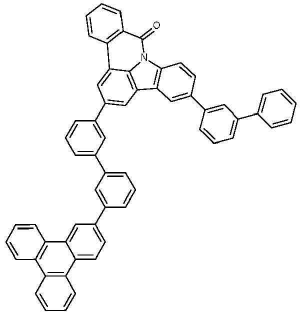

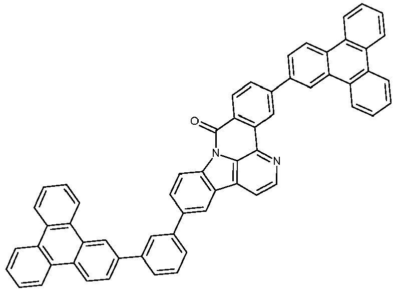

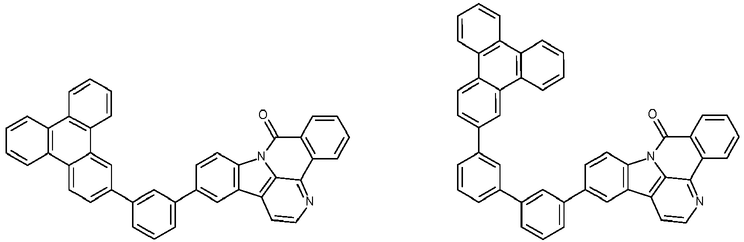

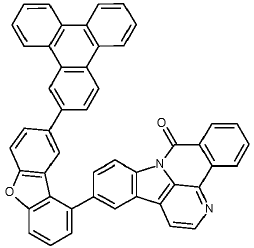

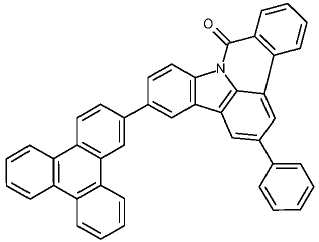

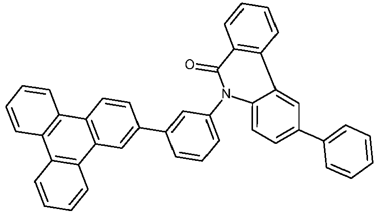

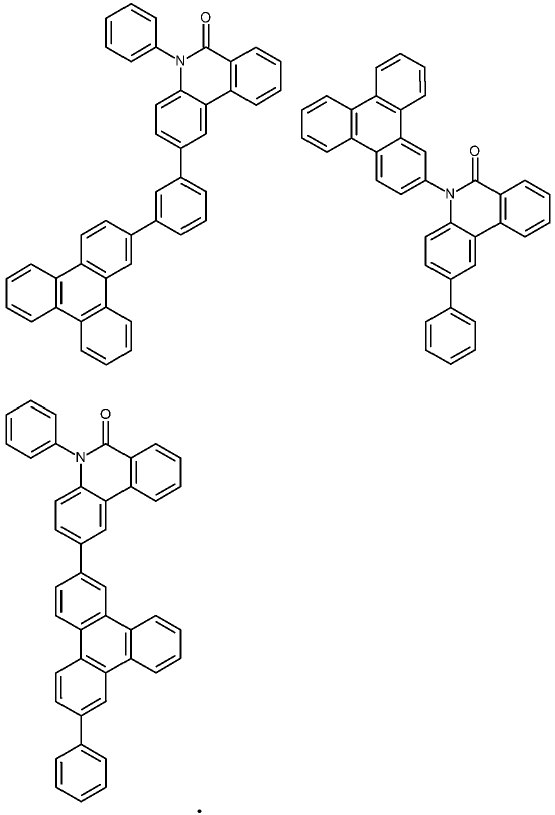

2. Organische Elektrolumineszenzvorrichtung nach Anspruch 1, **dadurch gekennzeichnet, dass** die Verbindung der Formel (1) aus Verbindungen der Formeln (1A) oder (1B) ausgewählt ist, 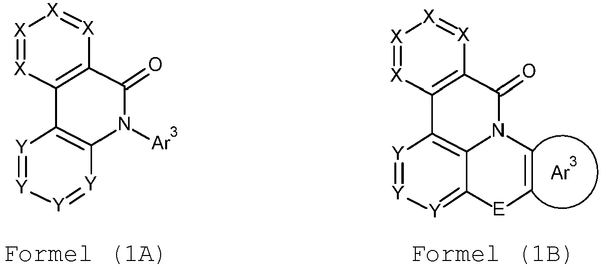 wobei
das Symbol -E- die gleiche Bedeutung wie in Anspruch 1 hat;
X für CR¹ oder N steht;
Y für CR² oder N steht;
wobei die Symbole R¹, R² gleiche Bedeutung wie in Anspruch 1 haben; und
Ar³ die gleiche Bedeutung wie in Anspruch 1 hat, mit der Maßgabe, dass Ar³ in Formel (1B) über zwei benachbarte C-Atome an die Gruppe -E- und das Atom N gebunden ist, wie in Formel (1B) dargestellt;
und wobei in Formel (1A) mindestens eine Gruppe Ar³, R¹ oder R² für ein Triphenylenderivat der Formel (T) steht und in Formel (1B) mindestens eine Gruppe R¹ oder R² für ein Triphenylenderivat der Formel (T) steht, wobei das Triphenylenderivat der Formel (T) wie in Anspruch 1 definiert ist.

3. Organische Elektrolumineszenzvorrichtung nach Anspruch 1 oder 2, **dadurch gekennzeichnet, dass** die Verbindung der Formel (1) aus den Verbindungen der Formeln (2), (3), (4), (5), (6) oder (7) ausgewählt ist, 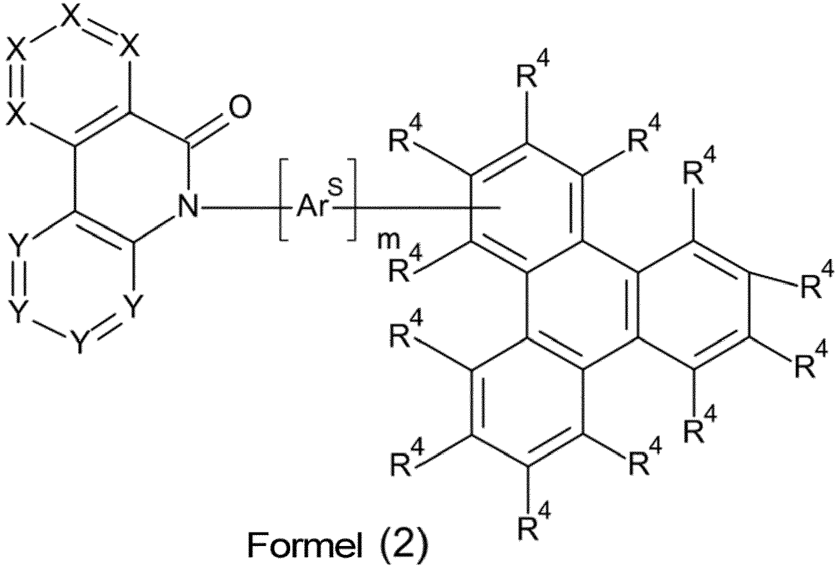 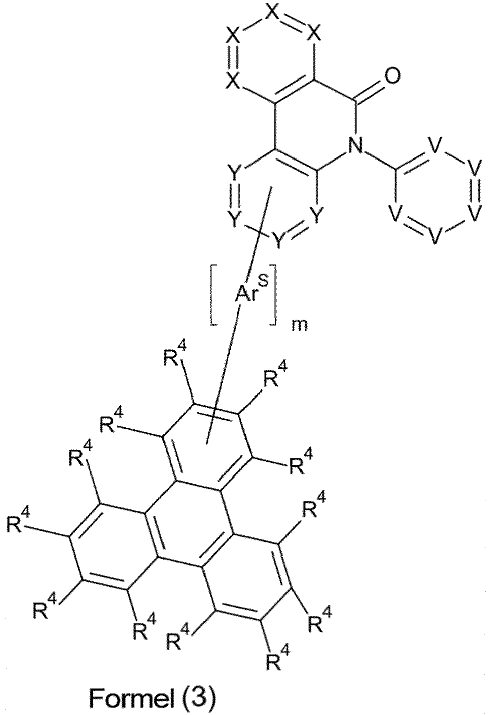 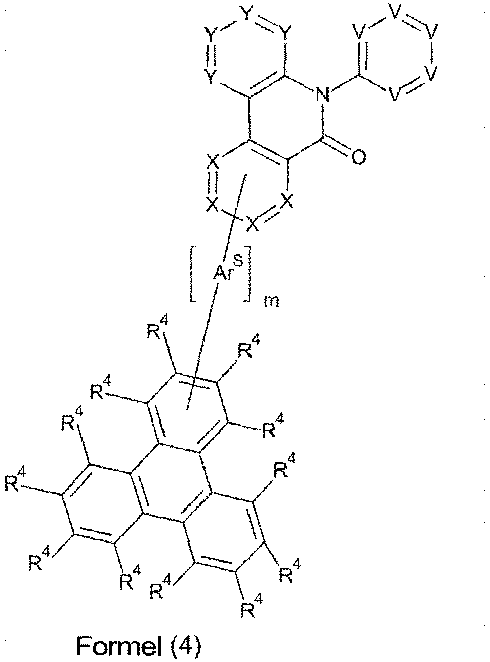 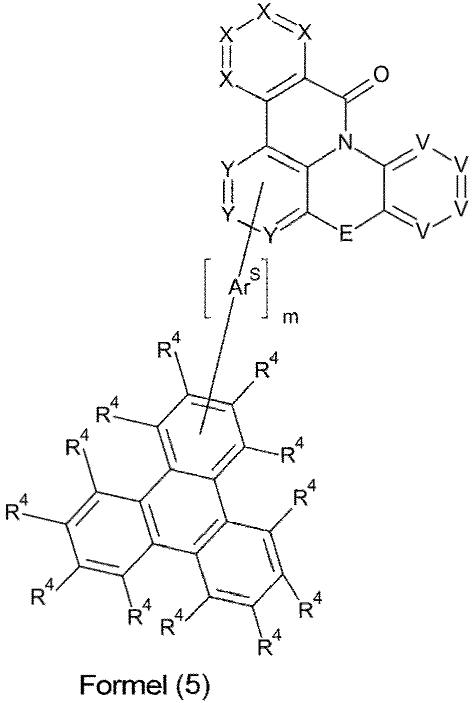 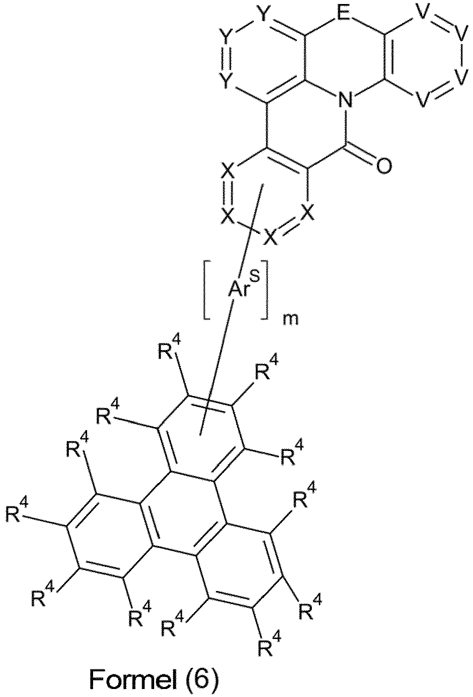 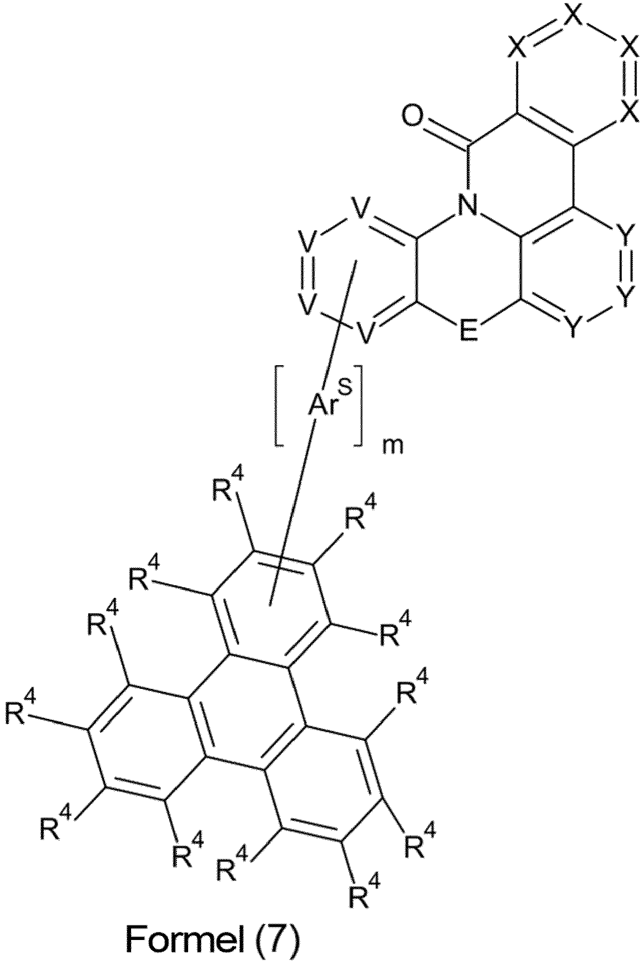 wobei die Symbole und Indizes E, R⁴, Ar^{S} und m die gleiche Bedeutung wie in Anspruch 1 haben; und wobei
X für CR¹ oder N steht, mit der Maßgabe, dass X C an der Position der Anbindung an Ar^{S} oder dann, wenn m 0 ist und Ar^{S} fehlt, an die Triphenylengruppe in den Formeln (4) und (6) ist;
Y für CR² oder N steht, mit der Maßgabe, dass Y C an der Position der Anbindung an Ar^{S} oder dann, wenn m 0 ist und Ar^{S} fehlt, an die Triphenylengruppe in den Formeln (3) und (5) ist;
V für CR³ oder N steht, mit der Maßgabe, dass V C an der Position der Anbindung an Ar^{S} oder dann, wenn m 0 ist und Ar^{S} fehlt, an die Triphenylengruppe in Formel (7) ist; oder zwei benachbarte Gruppen V für eine Gruppe der Formel (V-1) oder (V-2) stehen, 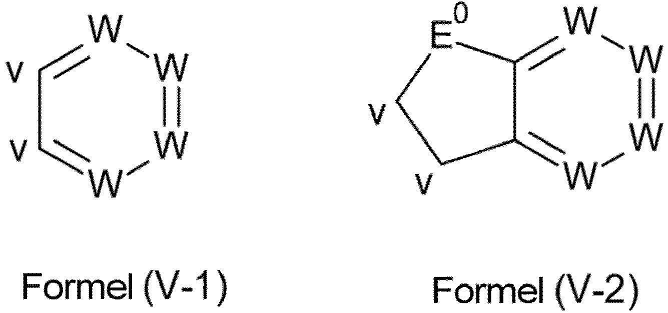
wobei die Symbole ^{V} die entsprechenden benachbarten Gruppen V in den Formeln (2) bis (7) anzeigen;
E⁰ für -C(R⁰)₂-, -Si(R⁰)₂-, -C(=O) -, -(=NR⁰)-,-C(=C(R⁰)₂)-, -O-, -S-, -S(=O)-, -S(O₂)-, -N(R⁰)-,-P(R⁰)- oder -P((=O)R⁰)- steht; wobei R° die gleiche Bedeutung wie in Anspruch 1 hat; und
W für CR oder N steht.

4. Organische Elektrolumineszenzvorrichtung nach einem oder mehreren der vorhergehenden Ansprüche, **dadurch gekennzeichnet, dass** der Index m gleich 0 oder 1 ist.

5. Organische Elektrolumineszenzvorrichtung nach einem oder mehreren der vorhergehenden Ansprüche, **dadurch gekennzeichnet, dass** die Gruppe Ar^{S} bei jedem Auftreten gleich oder verschieden für Phenyl, Biphenyl, Fluoren, Spirobifluoren, Naphthalin, Phenanthren, Anthracen, Dibenzofuran, Dibenzothiophen, Carbazol, Pyridin, Pyrimidin, Pyrazin, Pyridazin, Triazin, Benzopyridin, Benzopyridazin, Benzopyrimidin und Chinazolin steht, wobei jede dieser Gruppen durch einen oder mehrere Reste R substituiert sein kann.

6. Organische Elektrolumineszenzvorrichtung nach einem oder mehreren der vorhergehenden Ansprüche, **dadurch gekennzeichnet, dass** die Gruppe Ar^{S} bei jedem Auftreten gleich oder verschieden für Phenyl, Biphenyl, Fluoren, Naphthalin, Dibenzofuran, Dibenzothiophen und Carbazol steht, wobei jede dieser Gruppen durch einen oder mehrere Reste R substituiert sein kann.

7. Organische Elektrolumineszenzvorrichtung nach einem oder mehreren der Ansprüche 2 bis 6, **dadurch gekennzeichnet, dass** in der Verbindung der Formel (1) 0 oder 1 Gruppe X, die für N steht, in dem sechsgliedrigen Ring, der die Gruppen X umfasst, vorliegt, 0 oder 1 Gruppe Y, die für N steht, in dem sechsgliedrigen Ring, der die Gruppen Y umfasst, vorliegt und 0 oder 1 Gruppe V, die für N steht, in dem sechsgliedrigen Ring, der die Gruppen V umfasst, vorliegt.

8. Organische Elektrolumineszenzvorrichtung nach einem oder mehreren der vorhergehenden Ansprüche, **dadurch gekennzeichnet, dass** die Verbindung der Formel (1) aus Verbindungen der Formeln (2A) bis (7B) ausgewählt ist, 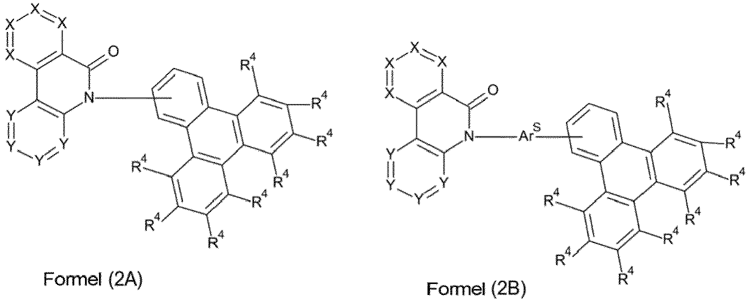 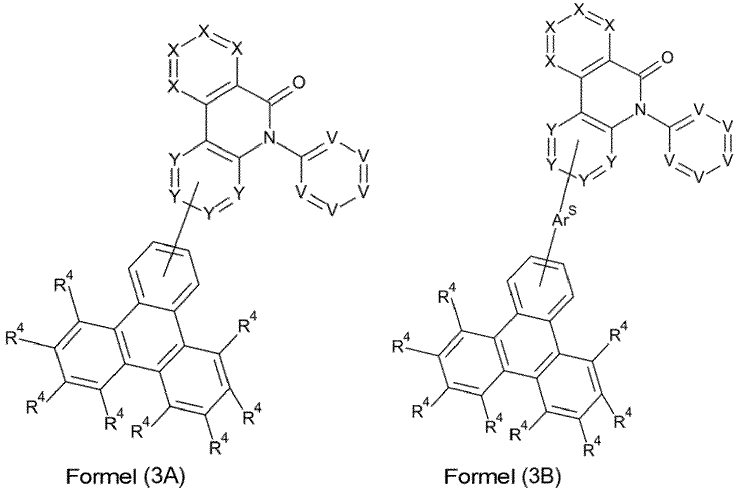 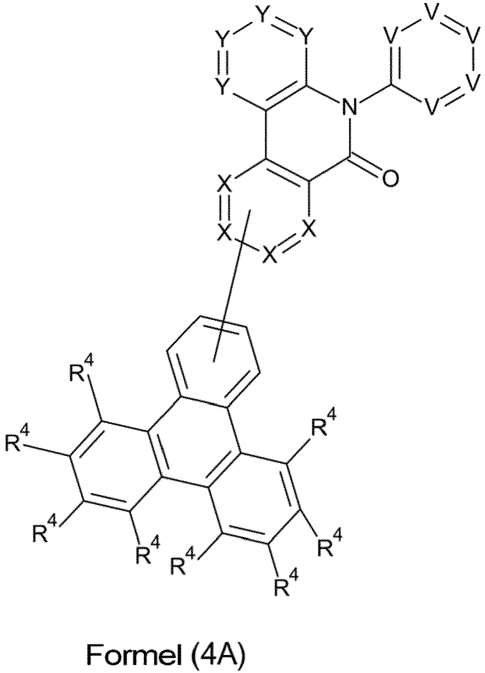 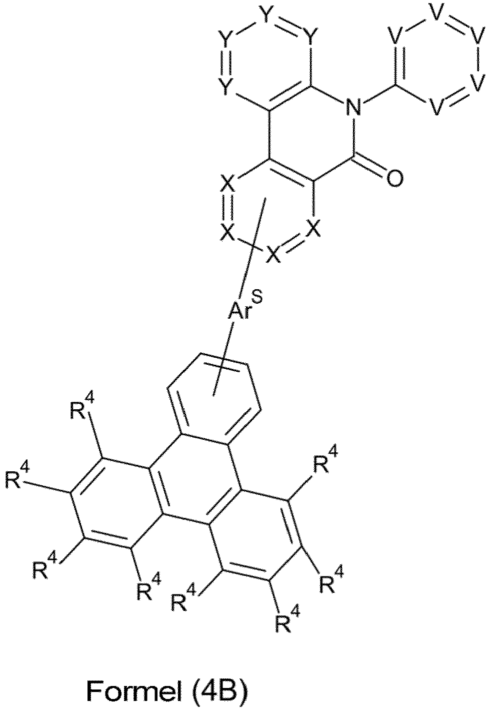 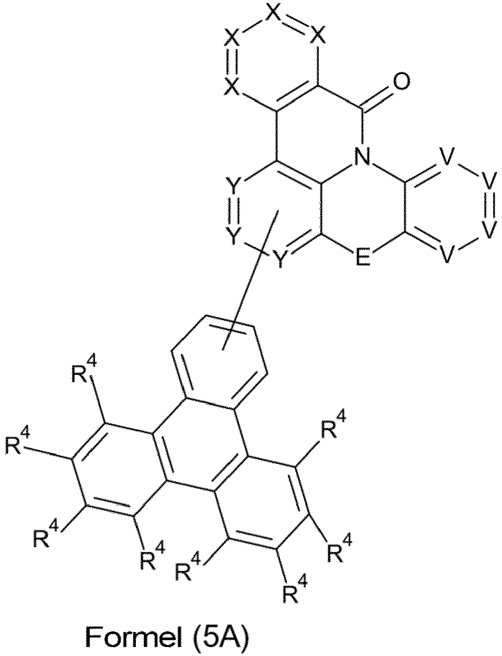 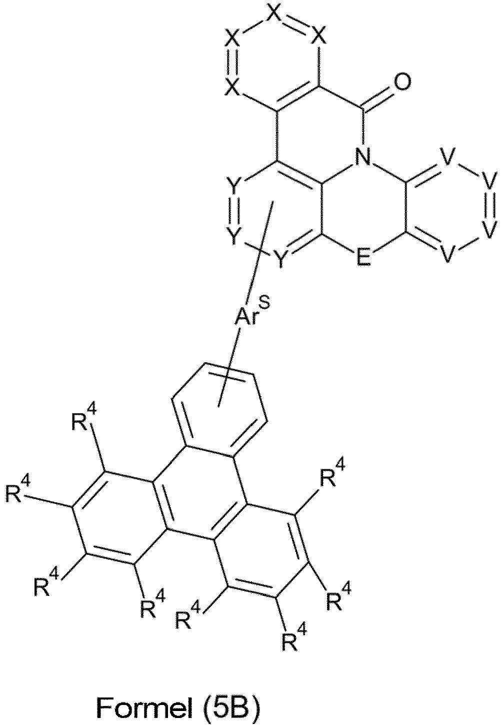 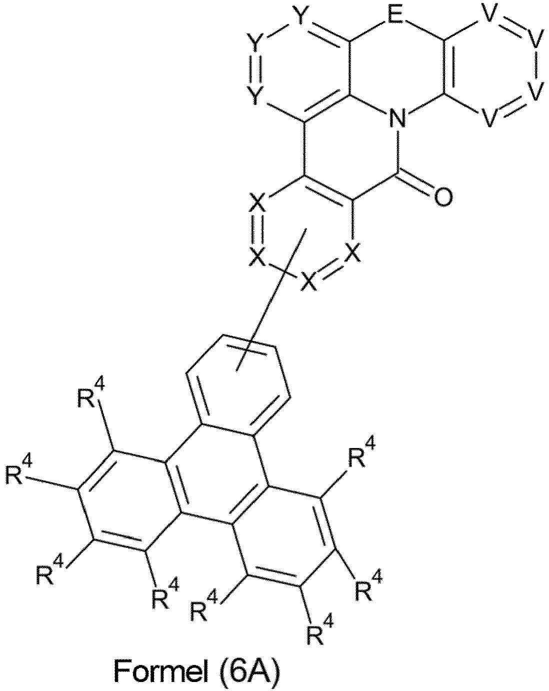 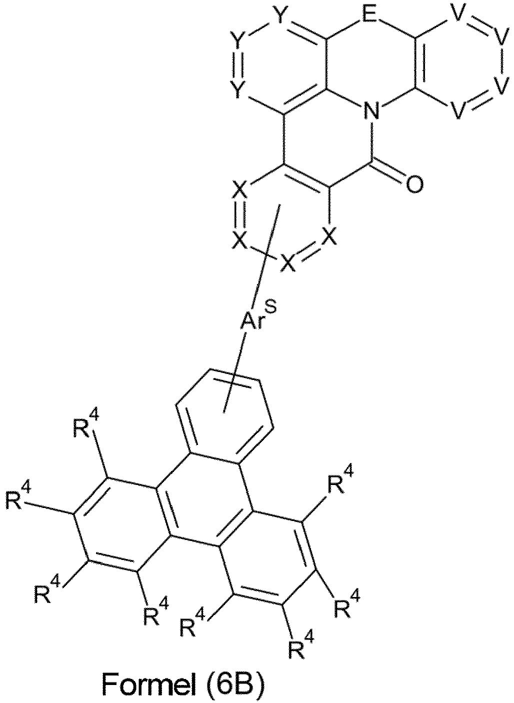 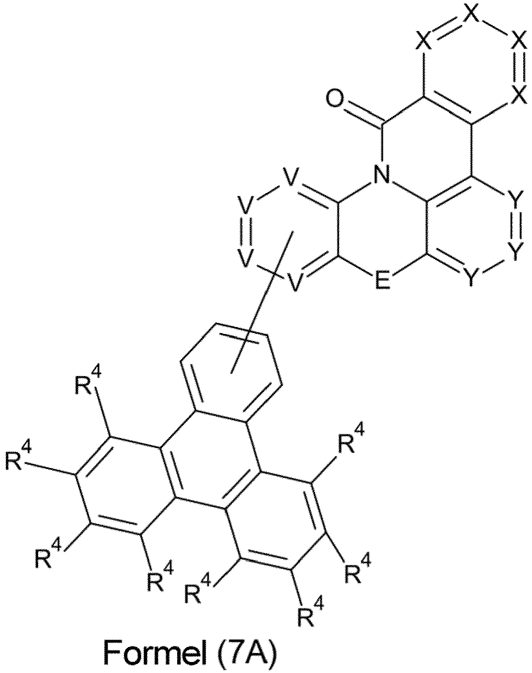 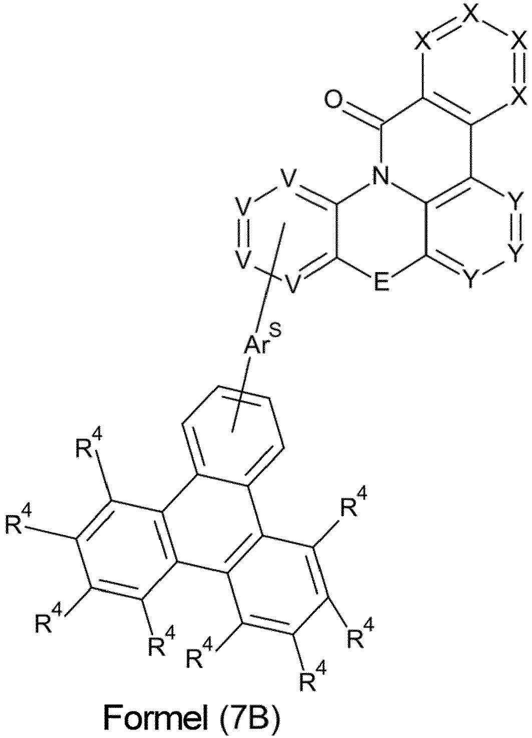 wobei die Symbole E, Ar^{S} und R⁴ die gleiche Bedeutung wie in Anspruch 1 haben und die Symbole X, Y und V die gleiche Bedeutung wie in Anspruch 3 haben.

9. Organische Elektrolumineszenzvorrichtung nach einem oder mehreren der vorhergehenden Ansprüche, **dadurch gekennzeichnet, dass** die Verbindung der Formel (1) aus Verbindungen der Formel (2A-1) bis (7B-1) ausgewählt ist, 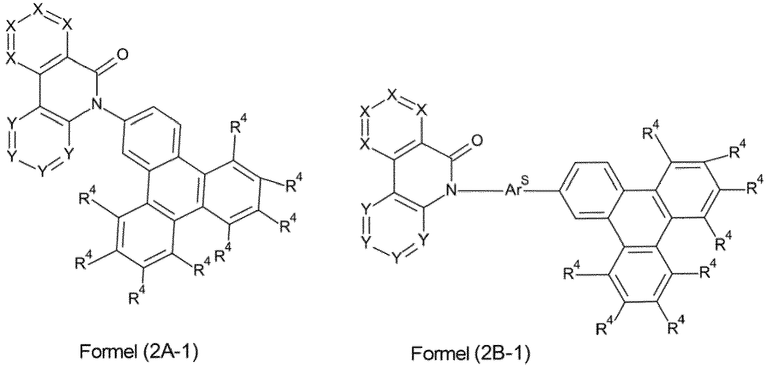 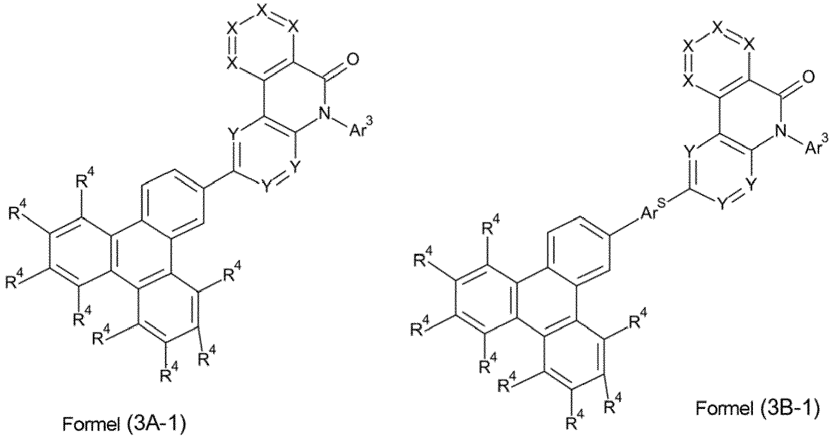 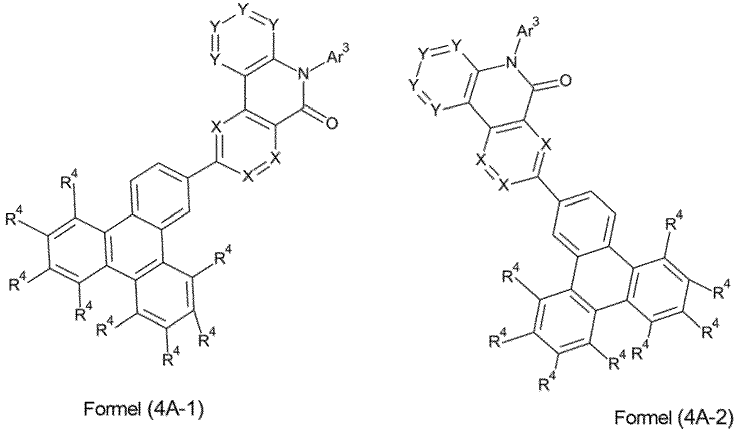 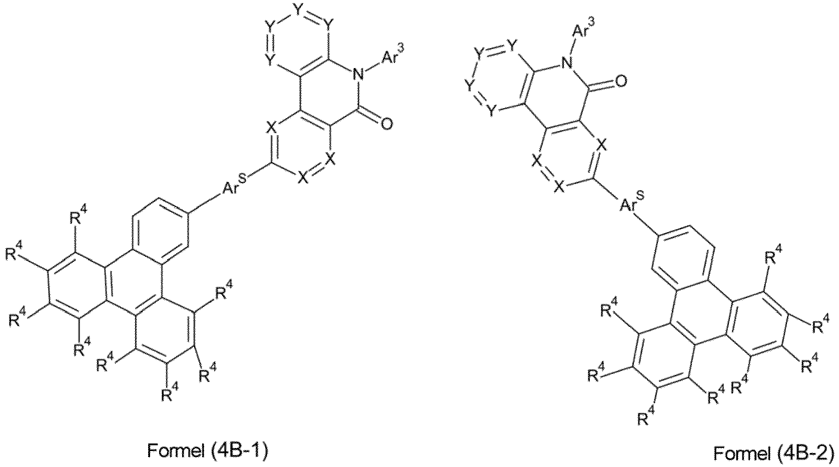 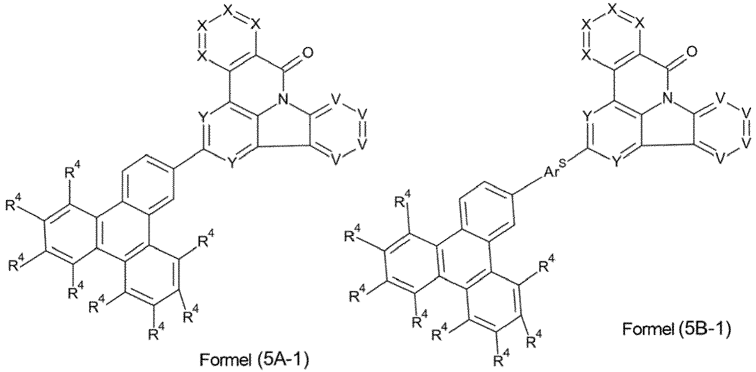 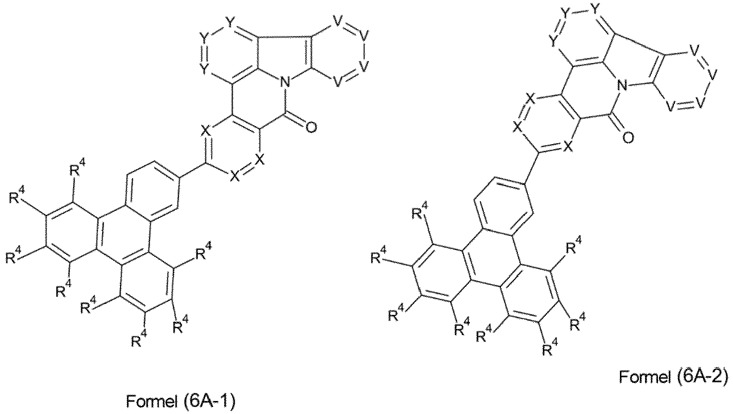 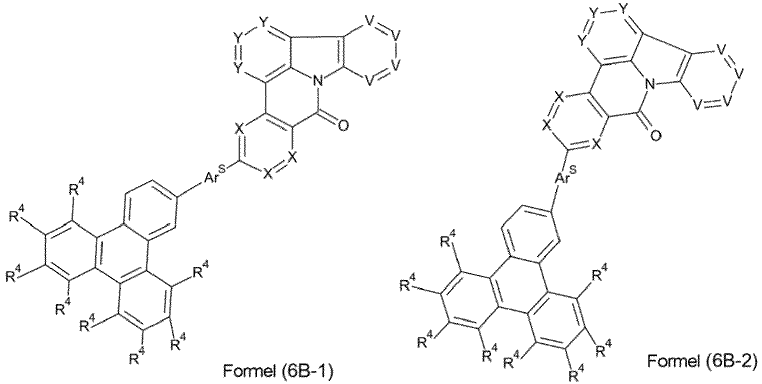 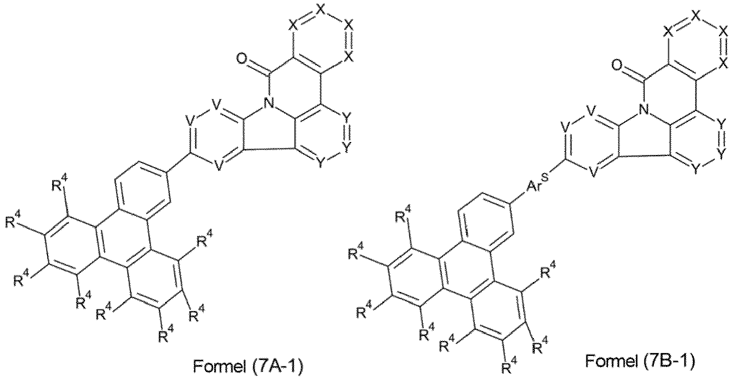 wobei das Symbol R⁴ die gleiche Bedeutung wie in Anspruch 1 hat und die Symbole X, Y und V die gleiche Bedeutung wie in Anspruch 3 haben.

10. Organische Elektrolumineszenzvorrichtung nach einem oder mehreren der vorhergehenden Ansprüche, **dadurch gekennzeichnet, dass** die Verbindung der Formel (1) mindestens einen der Substituenten R¹ oder R², die aus aromatischen und hydroaromatischen Ringsystemen ausgewählt sind, umfasst.

11. Organische Elektrolumineszenzvorrichtung nach einem oder mehreren der vorhergehenden Ansprüche, **dadurch gekennzeichnet, dass** die zweite Verbindung aus Verbindungen der Formeln (11-1) bis (12-4) ausgewählt ist, 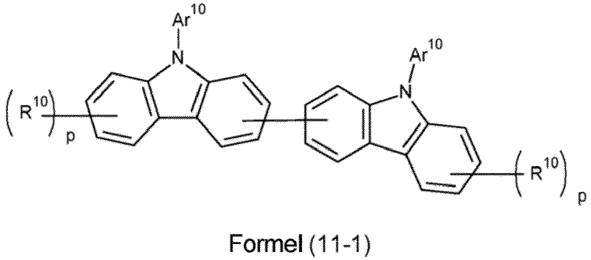 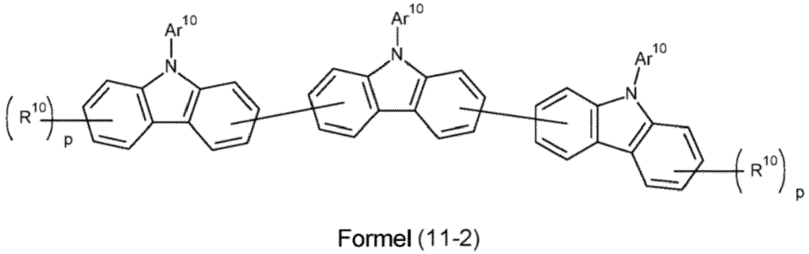 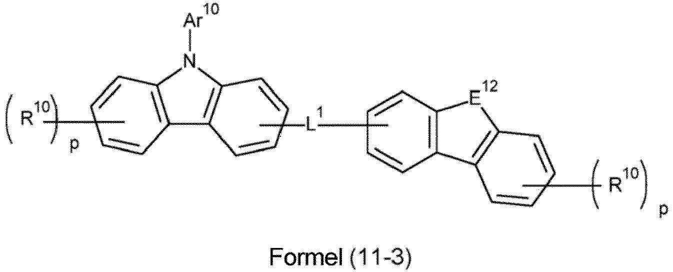 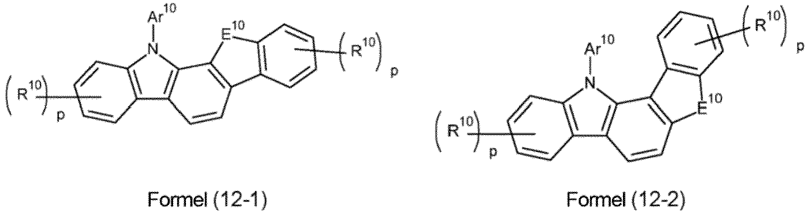 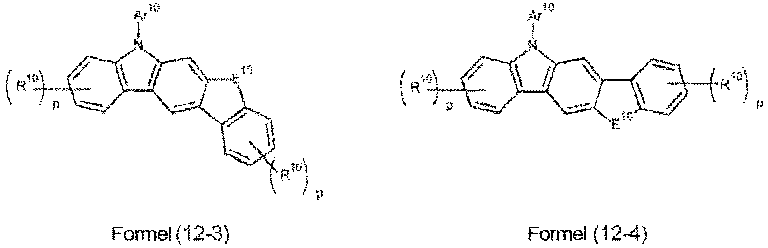
wobei die Symbole und Indizes Ar¹⁰, R¹⁰, E¹⁰, L¹ und p die gleiche Bedeutung wie in Anspruch 1 haben; und
E¹² für -C(R⁰)₂-, -O-, -S-, -N(Ar¹⁰)- steht; wobei R° die gleiche Bedeutung wie in Anspruch 1 hat.

12. Organische elektronische Vorrichtung nach einem oder mehreren der vorhergehenden Ansprüche, **dadurch gekennzeichnet, dass** die zweite Komponente aus Verbindungen der Formeln (13-1) bis (14-4) ausgewählt ist, 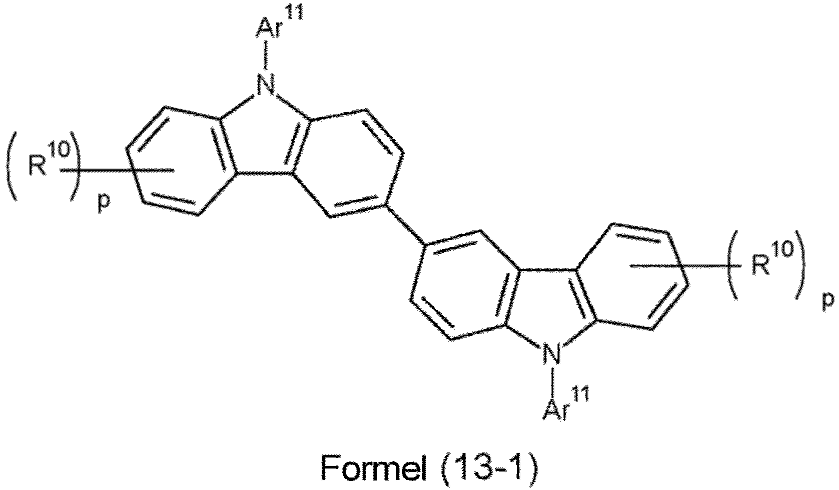 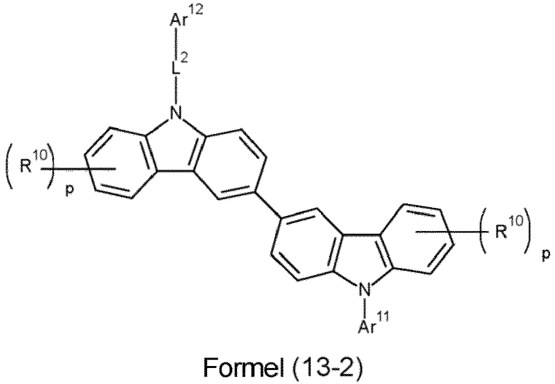 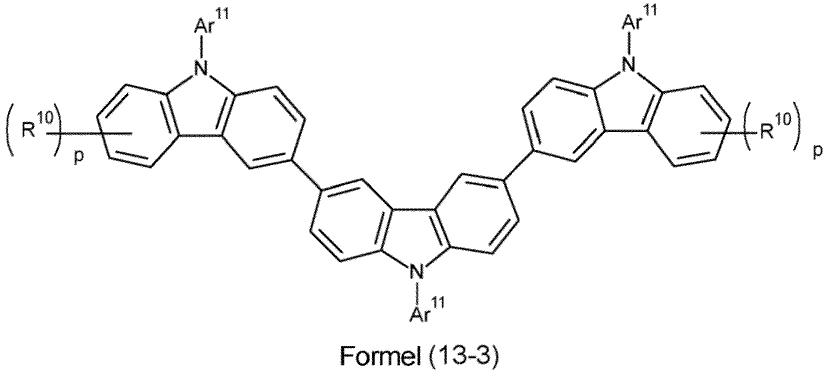 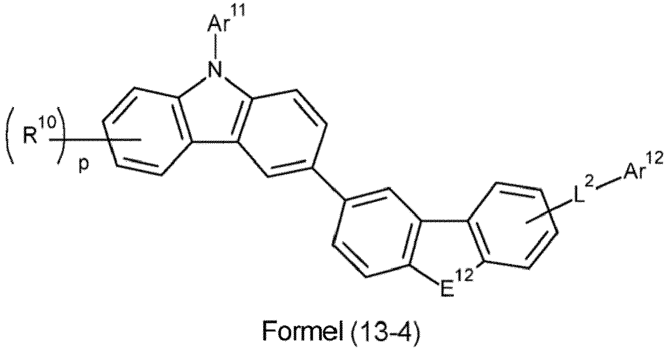 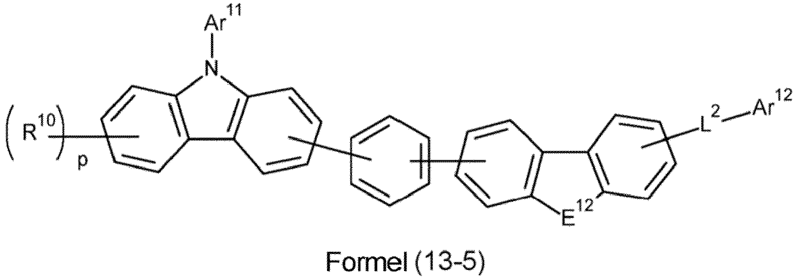 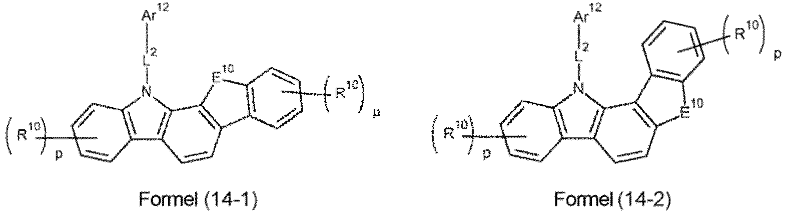 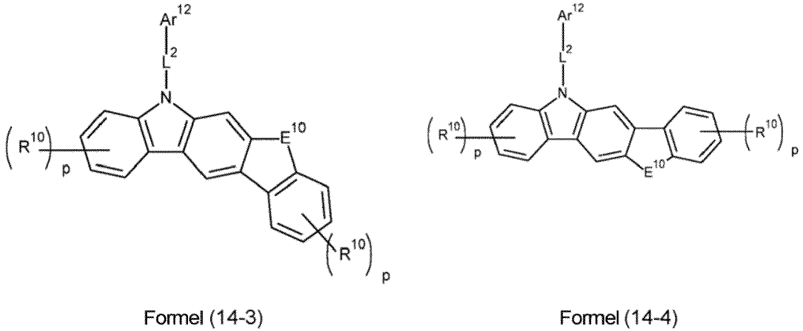 wobei die Symbole und Indizes R¹⁰, E¹⁰ und p die gleiche Bedeutung wie in Anspruch 11 haben und das Symbol E¹² die gleiche Bedeutung wie in Anspruch 13 hat; und
Ar¹⁰ für ein aromatisches oder heteroaromatisches Ringsystem steht, das aus Benzol, Naphthalin, Pyren, Biphenyl, Terphenyl, Quaterphenyl, Fluoren, Spirobifluoren, cis- oder trans-Indenofluoren, Dibenzofuran, Dibenzothiophen, Carbazol, Indolocarbazol, Indenocarbazol, die durch einen oder mehrere Reste R substituiert sein können, oder Kombinationen dieser Gruppen ausgewählt ist;
L² für eine Einfachbindung oder ein aromatisches oder heteroaromatisches Ringsystem mit 6 bis 18 aromatischen Ringatomen, das jeweils durch einen oder mehrere Reste R substituiert sein kann, steht;
Ar¹² für ein heteroaromatisches Ringsystem, das aus Pyridin, Pyrazin, Pyrimidin, Triazin, die durch einen oder mehrere Reste R substituiert sein können, ausgewählt ist, steht;
R die gleiche Bedeutung wie in Anspruch 1 hat.

## Revendications

1. Dispositif électroluminescent organique comprenant un mélange comprenant un composé de formule (1), 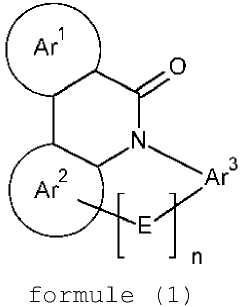 où les points suivants s'appliquent aux symboles et indices utilisés :
Ar¹ désigne à chaque occurrence, identique ou différent, un système cyclique aromatique ou hétéroaromatique ayant 5 à 60 atomes de cycle aromatique, qui peut dans chaque cas, être substitué par un ou plusieurs radicaux R¹ ;
Ar² désigne à chaque occurrence, identique ou différent, un système cyclique aromatique ou hétéroaromatique ayant 5 à 60 atomes de cycle aromatique, qui peut dans chaque cas, être substitué par un ou plusieurs radicaux R² ;
Ar³ désigne à chaque occurrence, identique ou différent, un système cyclique aromatique ou hétéroaromatique ayant 5 à 60 atomes de cycle aromatique, qui peut dans chaque cas, être substitué par un ou plusieurs radicaux R³ ;
-E- est choisi parmi une simple liaison, -B(R⁰)-, -C(R⁰)₂-, -Si(R⁰)₂-, -C(=O)-C(=NR⁰)-, -C(=C(R⁰)₂)-, -O-, -S-, - S(=O)-, -S(O₂)-, -N(R⁰)-, -P(R⁰)- et - P((=O)R⁰)- ;
R⁰, R¹, R², R³ désignent à chaque occurrence, identiques ou différents, H, D, F, Cl, Br, I, CHO, CN, N(Ar)₂, C(=O)Ar, P(=O)(Ar)₂, S(=O)Ar, S(=O) ₂Ar, NO₂, Si(R)₃, B(OR)₂, OSO₂R, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R, où dans chaque cas, un ou plusieurs groupes CH₂ non adjacents peuvent être remplacés par RC=CR, C=C, Si(R)₂, Ge(R)₂, Sn(R)₂, C=O, C=S, C=Se, P(=O)(R), SO, SO₂, O, S ou CONR et où un ou plusieurs atomes H peuvent être remplacés par D, F, Cl, Br, I, CN ou NO₂, un système cyclique aromatique ou hétéroaromatique ayant 5 à 60 atomes de cycle aromatique, qui peut dans chaque cas, être substitué par un ou plusieurs radicaux R, ou un groupe aryloxy ayant 5 à 40 atomes de cycle aromatique, qui peut être substitué par un ou plusieurs radicaux R, où deux radicaux R⁰, deux radicaux R¹, deux radicaux R² et/ou deux radicaux R³ peuvent former un système cyclique monocyclique ou polycyclique, aliphatique, aromatique ou hétéroaromatique l'un avec l'autre, qui peut être substitué par un ou plusieurs radicaux R ;
R désigne à chaque occurrence, identique ou différent, H, D, F, Cl, Br, I, CHO, CN, N(Ar)₂, C(=O)Ar, P(=O) (Ar)₂, S(=O)Ar, S(=O)₂Ar, NO₂, Si(R')₃, B(OR')₂, OSO₂R', un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R', où dans chaque cas, un ou plusieurs groupes CH₂ non adjacents peuvent être remplacés par R C=CR, C=C, Si(R')₂, Ge(R')₂, Sn(R')₂, C=O, C=S, C=Se, P(=O)(R'), SO, SO₂, O, S ou CONR' et où un ou plusieurs atomes H peuvent être remplacés par D, F, Cl, Br, I, CN ou NO₂, un système cyclique aromatique ou hétéroaromatique ayant 5 à 60 atomes de cycle aromatique, qui peut dans chaque cas, être substitué par un ou plusieurs radicaux R, ou un groupe aryloxy ayant 5 à 40 atomes de cycle aromatique, qui peut être substitué par un ou plusieurs radicaux R, où deux radicaux R peuvent former un système cyclique l'un avec l'autre, qui peut être substitué par un ou plusieurs radicaux R' ;
Ar est un système cyclique aromatique ou hétéroaromatique ayant 5 à 24 atomes de cycle aromatique, qui peut dans chaque cas, également être substitué par un ou plusieurs radicaux R' ;
R' désigne à chaque occurrence, identique ou différent, H, D, F, Cl, Br, I, CN, un groupe alkyle, alcoxy ou thioalkyle à chaîne linéaire ayant 1 à 20 atomes C ou un groupe alkyle, alcoxy ou thioalkyle ramifié ou cyclique ayant 3 à 20 atomes C, où dans chaque cas, un ou plusieurs groupes CH₂ non adjacents peuvent être remplacés par SO, SO₂, O, S et où un ou plusieurs atomes H peuvent être remplacés par D, F, Cl, Br ou I, ou un système cyclique aromatique ou hétéroaromatique ayant 5 à 24 atomes C ;
n est 0 ou 1 ; dans lequel, lorsque n est 0, le groupe -E-est absent ;
où le composé de formule (1) comprend au moins un groupe Ar³, R¹, R² ou R³, qui désignent un dérivé de triphénylène de formule (T), 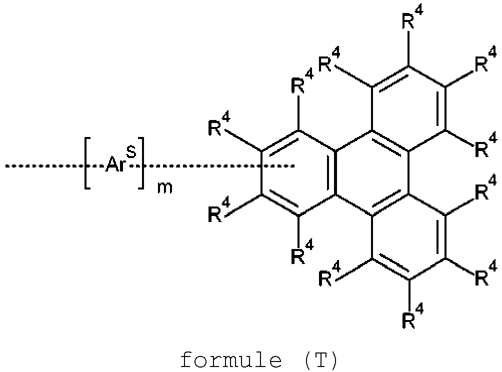
où le trait pointillé indique la liaison à la structure de formule (1), et où :
R⁴ est C à la position de la liaison à Ar^{S} ou, R⁴ est C à la position de la liaison à la structure de formule (1) si m est 0 ;
et aux autres positions, R⁴ désigne à chaque occurrence, identique ou différent, H, D, F, Cl, Br, I, CHO, CN, N(Ar)₂, C(=O)Ar, P(=O)(Ar)₂, S(=O)Ar, S(=O)₂Ar, NO₂, Si(R)3, B(OR)₂, OSO₂R, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R, où dans chaque cas, un ou plusieurs groupes CH₂ non adjacents peuvent être remplacés par RC=CR, C=C, Si(R)₂, Ge(R)₂, Sn(R)₂, C=O, C=S, C=Se, P (=O) (R), SO, SO₂, O, S ou CONR et où un ou plusieurs atomes H peuvent être remplacés par D, F, Cl, Br, I, CN ou NO₂, un système cyclique aromatique ou hétéroaromatique ayant 5 à 60 atomes de cycle aromatique, qui peut dans chaque cas, être substitué par un ou plusieurs radicaux R, ou un groupe aryloxy ayant 5 à 40 atomes de cycle aromatique, qui peut être substitué par un ou plusieurs radicaux R, où deux radicaux R⁴ peuvent former un système cyclique monocyclique ou polycyclique, aliphatique, aromatique ou hétéroaromatique, qui peut être substitué par un ou plusieurs radicaux R ;
Ar^{S} est un système cyclique aromatique ou hétéroaromatique ayant 5 à 60 atomes de cycle aromatique, qui peut dans chaque cas, également être substitué par un ou plusieurs radicaux R ; et
m est un entier choisi parmi 0, 1, 2, 3 ou 4 ; et
et un deuxième composé choisi parmi les composés de formules (11) et (12). 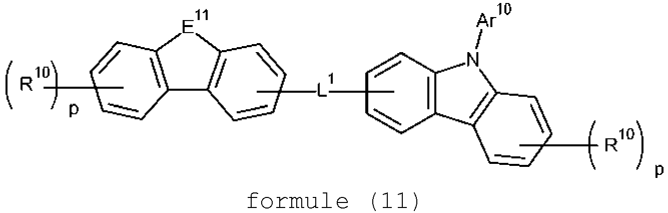 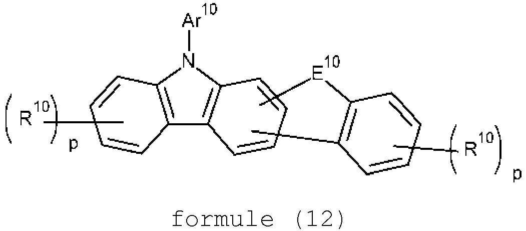
où :
R¹⁰ désigne à chaque occurrence, identique ou différent, H, D, F, Cl, Br, I, CHO, CN, N(Ar)₂, C(=O)Ar, P(=O)(Ar)₂, S(=O)Ar, S(=O)₂Ar, NO₂, Si(R)₃, B(OR)₂, OSO₂R, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R, où dans chaque cas, un ou plusieurs groupes CH₂ non adjacents peuvent être remplacés par RC=CR, C=C, Si(R)₂, Ge(R)₂, Sn(R)₂, C=O, C=S, C=Se, P(=O)(R), SO, SO₂, O, S ou CONR et où un ou plusieurs atomes H peuvent être remplacés par D, F, Cl, Br, I, CN ou NO₂, un système cyclique aromatique ou hétéroaromatique ayant 5 à 60 atomes de cycle aromatique, qui peut dans chaque cas, être substitué par un ou plusieurs radicaux R, ou un groupe aryloxy ayant 5 à 40 atomes de cycle aromatique, qui peut être substitué par un ou plusieurs radicaux R, où deux radicaux R¹⁰ peuvent former un système cyclique monocyclique ou polycyclique, aliphatique, aromatique ou hétéroaromatique l'un avec l'autre, qui peut être substitué par un ou plusieurs radicaux R ;
Ar¹⁰ est un système cyclique aromatique ou hétéroaromatique ayant 5 à 60 atomes de cycle aromatique, qui peut dans chaque cas, également être substitué par un ou plusieurs radicaux R ;
p est un entier choisi parmi 0, 1, 2, 3 et 4 ;
E¹⁰ désigne -C(R⁰)₂-, -Si(R⁰)₂-, -C(=O)-, -C(=NR⁰)-, - C(=C(R⁰)₂)-, -O-, -S-, -S(=O)-, -S(O₂)-, -N(Ar¹⁰)-, -P(R⁰)- ou -P((=O)R⁰)- ;
E¹¹ désigne -C(R⁰)₂-, -Si(R⁰)₂-, -C(=O)-, -C(=NR⁰)-, - C(=C(R⁰)₂)-, -O-, -S-, -S(=O)-, -S(O₂)-, -N(Ar¹⁰)-, -P(R⁰)- ou -P((=O)R⁰)- ; et
L¹ est une simple liaison ou un système cyclique aromatique ou hétéroaromatique ayant 5 à 60 atomes de cycle aromatique, qui peut dans chaque cas, également être substitué par un ou plusieurs radicaux R,
où le mélange est utilisé en tant que matériau de matrice pour des émetteurs phosphorescents dans une couche électroluminescente ; et
où les composés suivants sont exclus des composés de formule (1) :

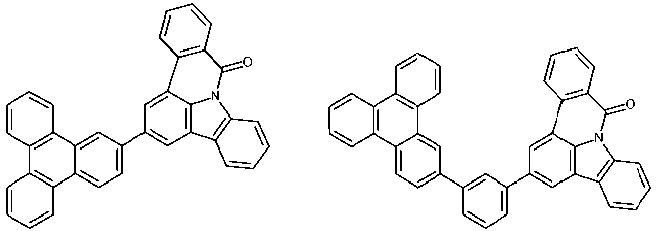

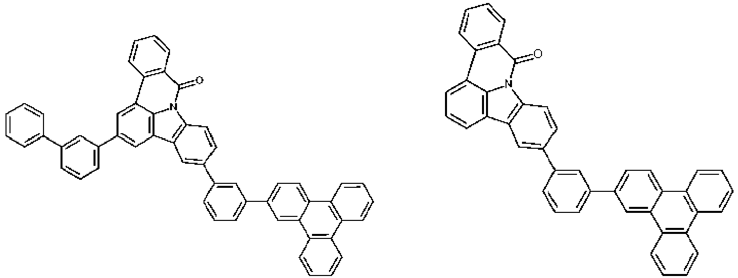

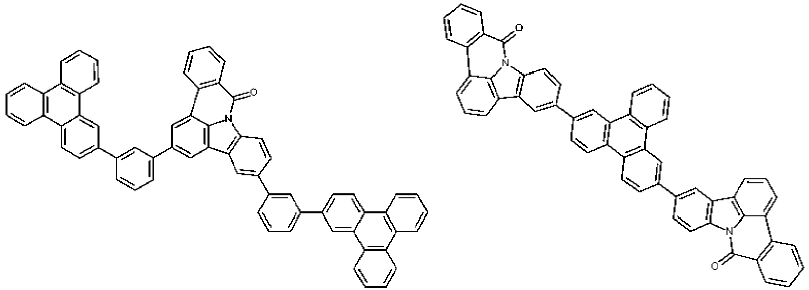

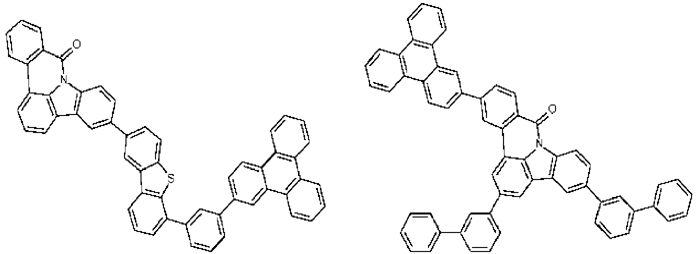

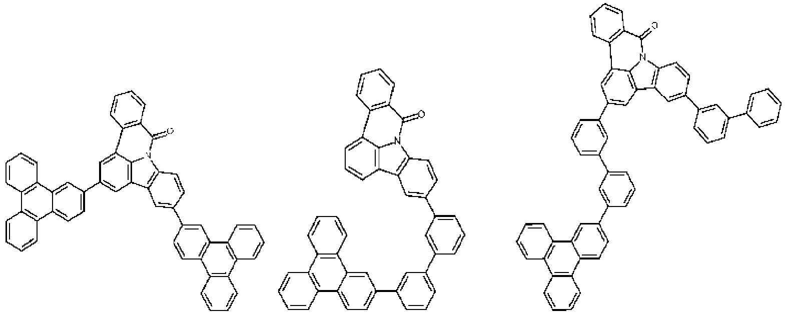

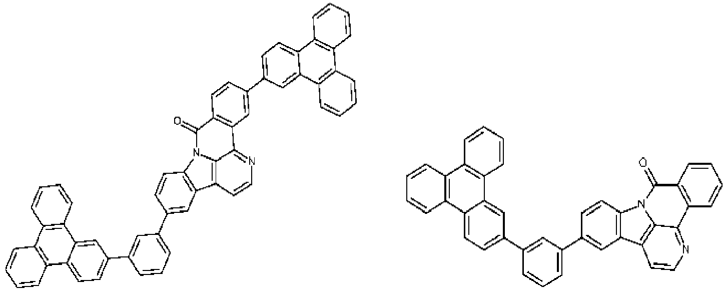

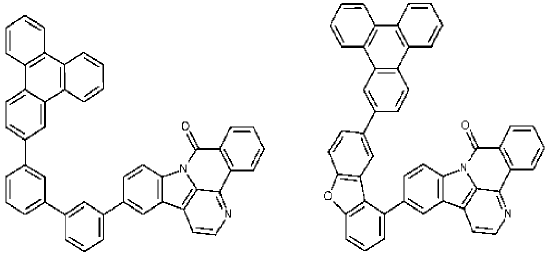

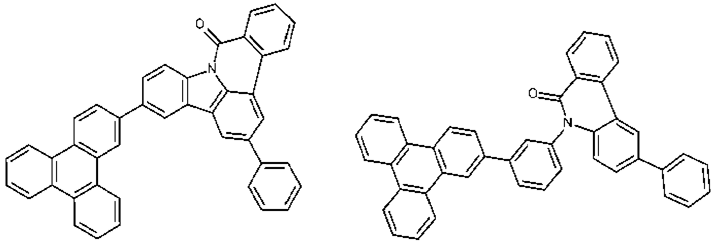

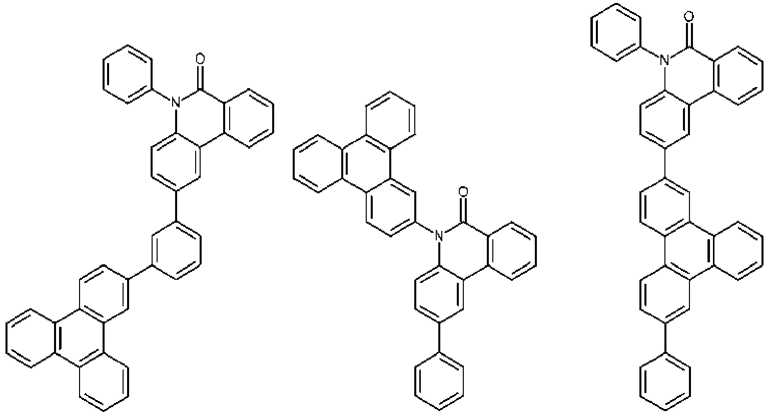

2. Dispositif électroluminescent organique selon la revendication 1, **caractérisé en ce que** le composé de formule (1) est choisi parmi les composés de formules (1A) ou(1B), 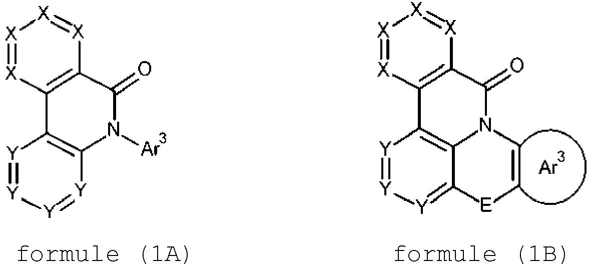 où
le symbole -E- a la même signification que dans la revendication 1 ;
X est CR¹ ou N ;
Y est CR² ou N ;
où les symboles R¹, R² ont la même signification que dans la revendication 1 ; et
Ar³ a la même signification que dans la revendication 1, à condition que Ar³, dans la formule (1B), soit relié au groupe -E- et à l'atome N par l'intermédiaire de deux atomes C adjacents comme représenté dans la formule (1B) ;
et où dans la formule (1A), au moins un groupe Ar³, R¹ ou R² désigne un dérivé de triphénylène de formule (T) et, dans la formule (1B), au moins un groupe R¹ ou R² désigne un dérivé de triphénylène de formule (T), où le dérivé de triphénylène de formule (T) est comme défini dans la revendication 1.

3. Dispositif électroluminescent organique selon la revendication 1 ou 2, **caractérisé en ce que** le composé de formule (1) est choisi parmi les composés de formules (2), (3), (4), (5), (6) ou (7), 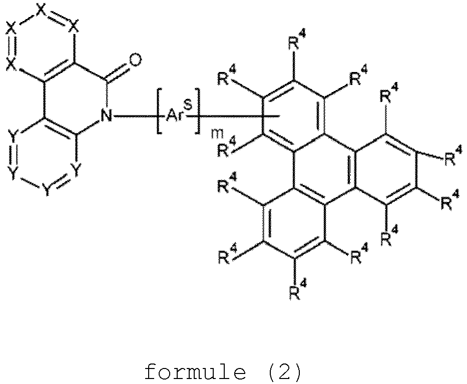 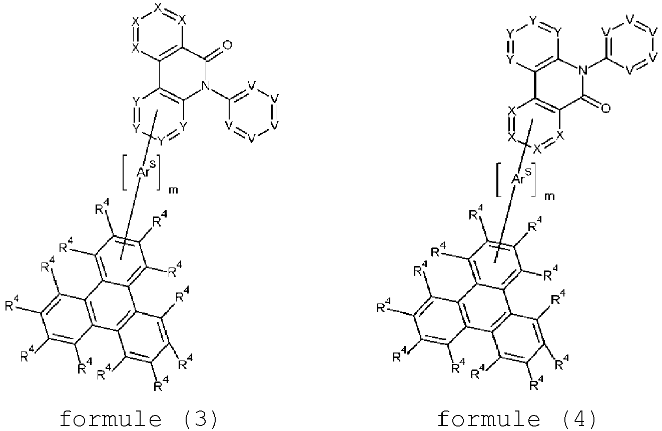 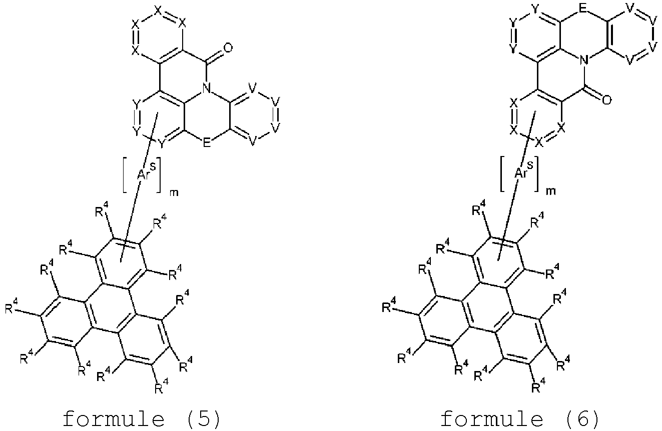 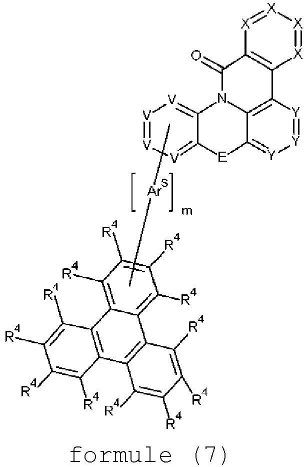
où les symboles et indices E, R⁴, Ar^{S} et m ont la même signification que dans la revendication 1 ; et où X est CR¹ ou N, à condition que X soit C à la position de la liaison à Ar^{S} ou, si m est 0 et Ar^{S} est absent, au groupe triphénylène dans les formules (4) et (6) ;
Y est CR² ou N, à condition que Y soit C à la position de la liaison à Ar^{S} ou, si m est 0 et Ar^{S} est absent, au groupe triphénylène dans les formules (3) et (5) ;
V est CR³ ou N, à condition que V soit C à la position de la liaison à Ar^{S} ou, si m est 0 et Ar^{S} est absent, au groupe triphénylène dans la formule (7) ; ou deux groupes V adjacents désignent un groupe de formule (V-1) ou (V-2), 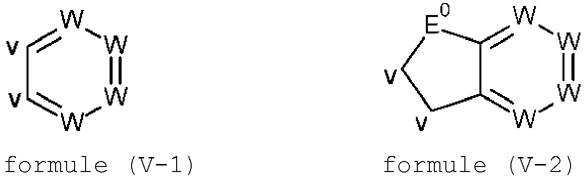
où les symboles V indiquent les groupes V adjacents correspondants dans les formules (2) à (7) ;
E⁰ désigne -C(R⁰)₂-, -Si(R⁰)₂-, -C(=O)-, -C(=NR⁰)-, - C(=C(R⁰)₂)-, -O-, -S-, -S(=O)-, -S(I₂)-, -N(R⁰)-, -P(R⁰)- ou -P((=O)R⁰)- ; où R⁰ a la même signification que dans la revendication 1 ; et
W désigne CR ou N.

4. Dispositif électroluminescent organique selon un ou plusieurs des revendications précédentes, **caractérisé en ce que** l'indice m est égal à 0 ou 1.

5. Dispositif électroluminescent organique selon une ou plusieurs des revendications précédentes, **caractérisé en ce que** le groupe Ar^{S} désigne à chaque occurrence, identique ou différent, phényle, biphényle, fluorène, spirobifluorène, naphtalène, phénanthrène, anthracène, dibenzofurane, dibenzothiophène, carbazole, pyridine, pyrimidine, pyrazine, pyridazine, triazine, benzopyridine, benzopyridazine, benzopyrimidine et quinazoline, dont chacun peut être substitué par un ou plusieurs radicaux R.

6. Dispositif électroluminescent organique selon une ou plusieurs des revendications précédentes, **caractérisé en ce que** le groupe Ar^{s} désigne à chaque occurrence, identique ou différent, phényle, biphényle, fluorène, naphtalène, dibenzofurane, dibenzothiophène et carbazole, dont chacun peut être substitué par un ou plusieurs radicaux R.

7. Dispositif électroluminescent organique selon une ou plusieurs des revendications 2 à 6, **caractérisé en ce que**, dans le composé de formule (1), il y a 0 ou 1 groupe X désignant N dans le cycle à six chaînons comprenant les groupes X, il y a 0 ou 1 groupe Y désignant N dans le cycle à six chaînons comprenant les groupes Y et il y a 0 ou 1 groupe V désignant V dans le cycle à six chaînons comprenant les groupes V.

8. Dispositif électroluminescent organique selon une ou plusieurs des revendications précédentes, **caractérisé en ce que** le composé de formule (1) est choisi parmi les composés de formules (2A) à (7B), 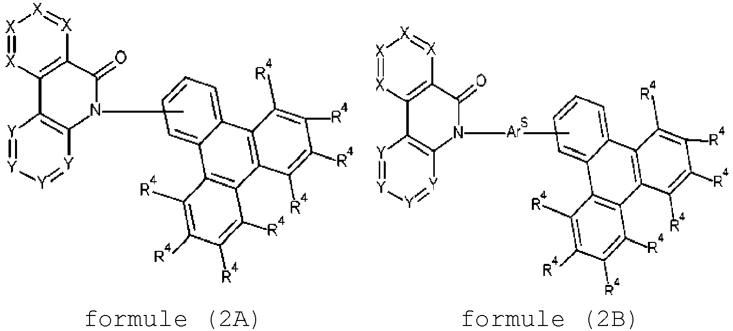 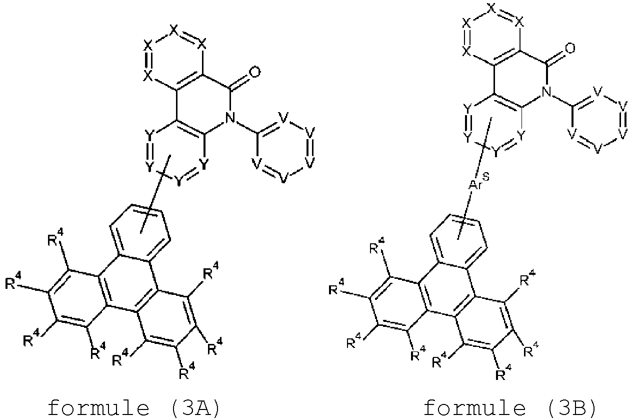 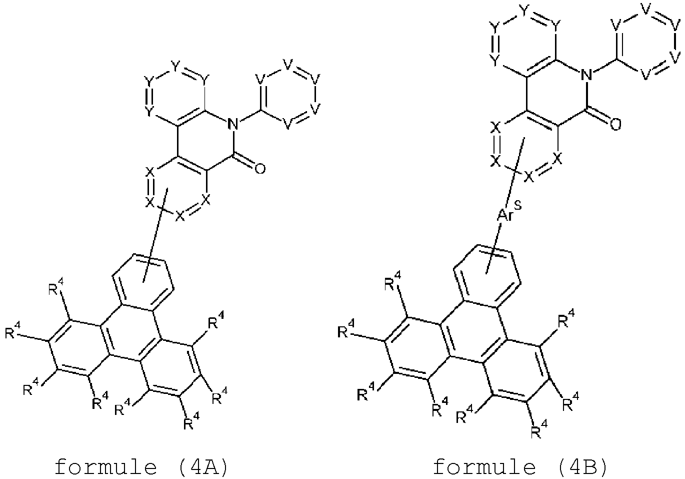 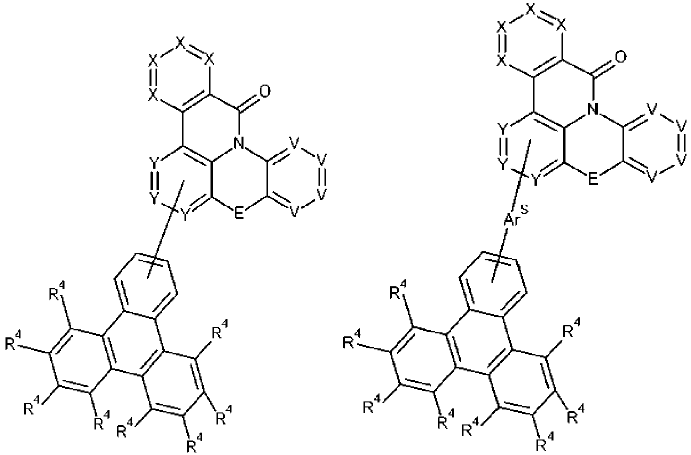 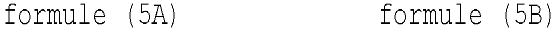 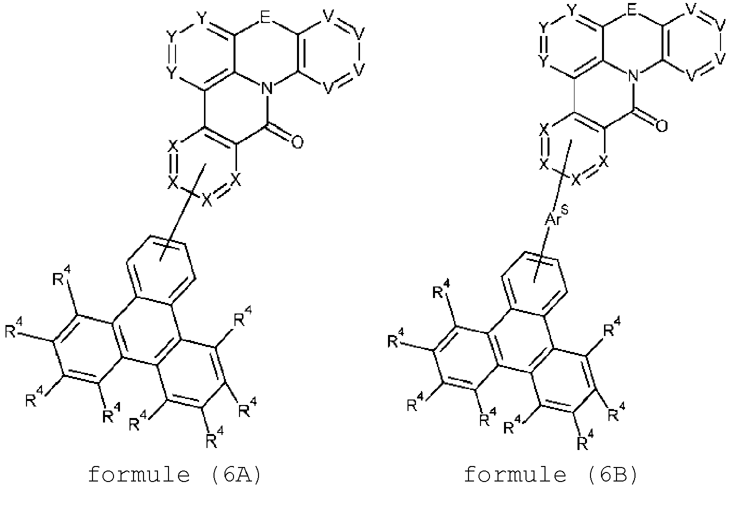 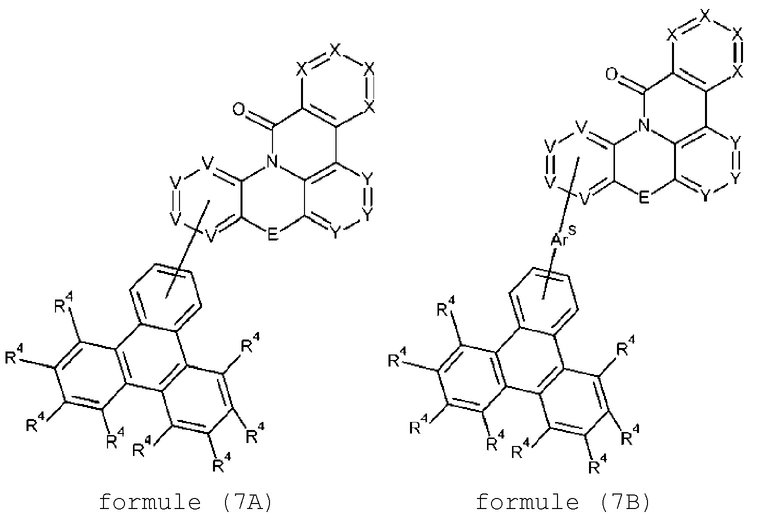 où les symboles E, Ar^{s} et R⁴ ont la même signification que dans la revendication 1, et les symboles X, Y et V ont la même signification que dans la revendication 3.

9. Dispositif électroluminescent organique selon une ou plusieurs des revendications précédentes, **caractérisé en ce que** le composé de formule (1) est choisi parmi les composés (2A-1) à (7B-1), 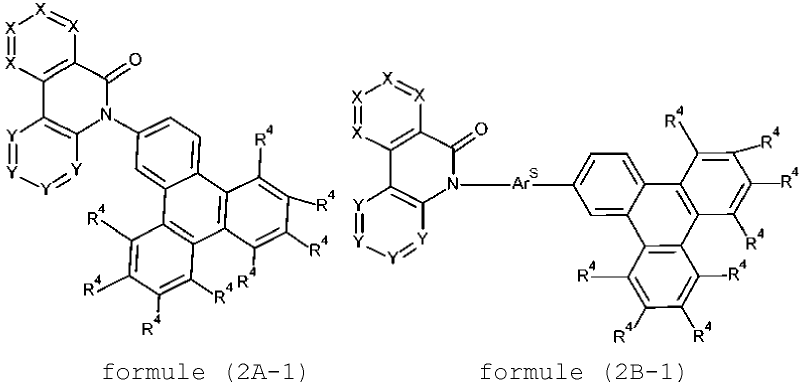 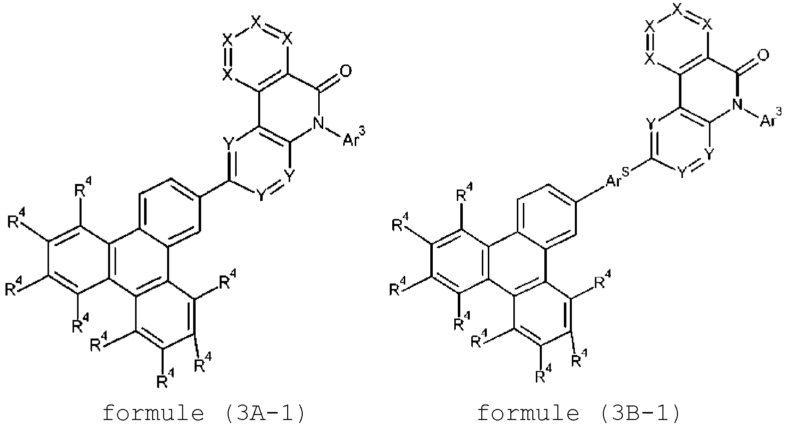 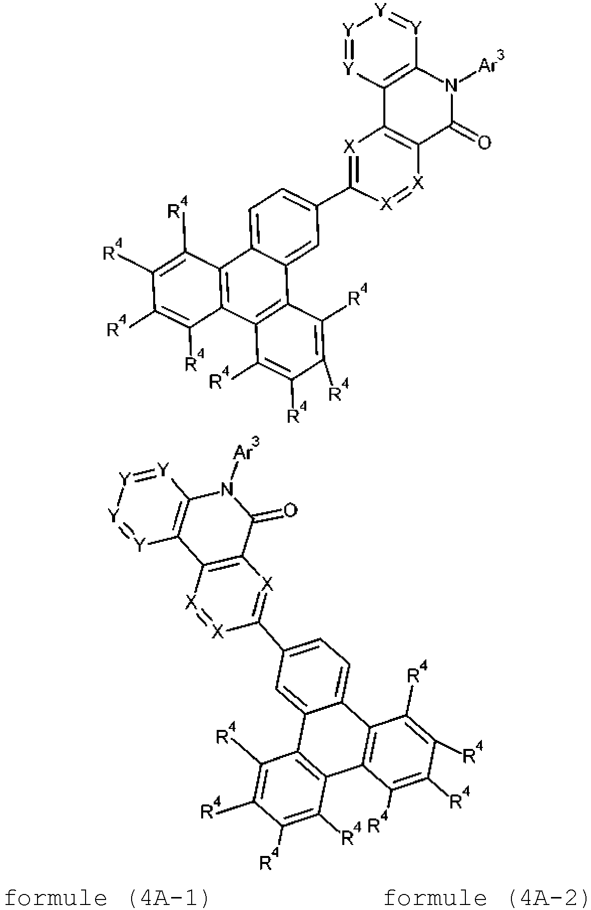 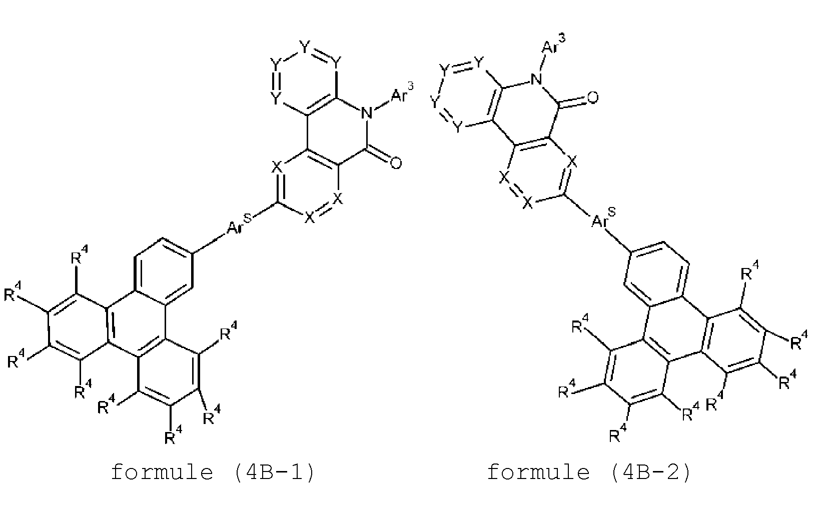 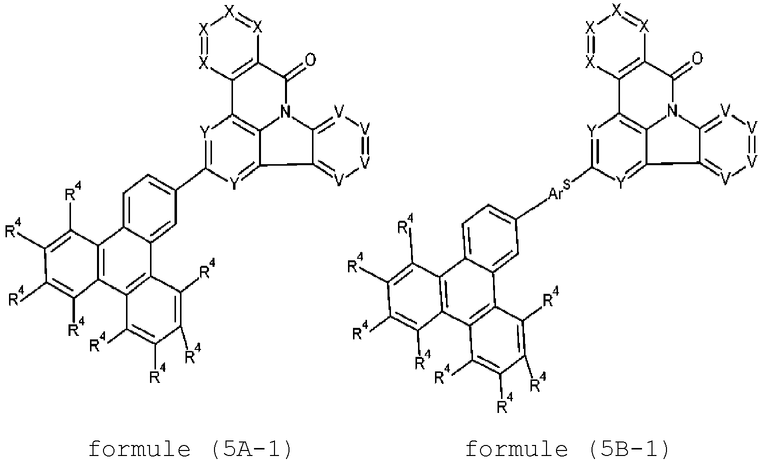 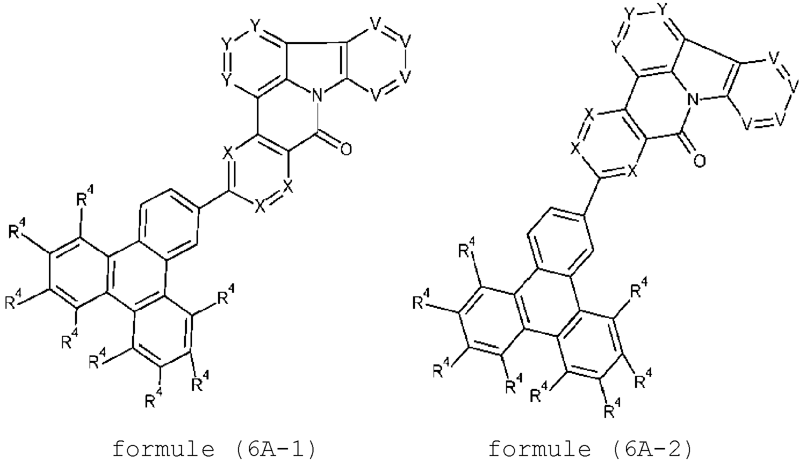 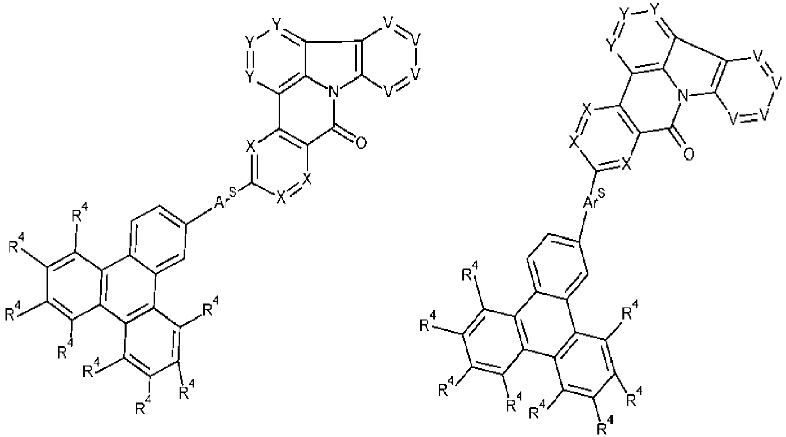 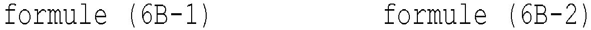 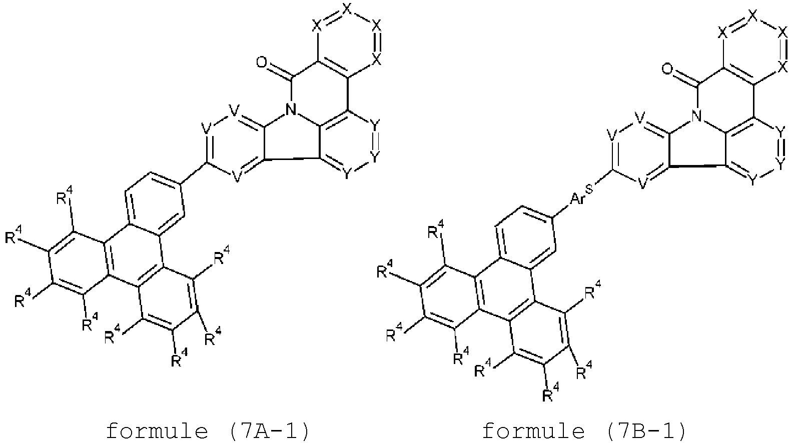 où le symbole R⁴ a la même signification que dans la revendication 1 et les symboles X, Y, V ont la même signification que dans la revendication 3.

10. Dispositif électroluminescent organique selon une ou plusieurs des revendications précédentes, **caractérisé en ce que** le composé de formule (1) comprend au moins un des substituants R¹ ou R², qui est choisi parmi des systèmes cycliques aromatiques et hétéroaromatiques.

11. Dispositif électroluminescent organique selon une ou plusieurs des revendications précédentes, **caractérisé en ce que** le deuxième composé est choisi parmi les composés de formules (11-1) à (12-4), 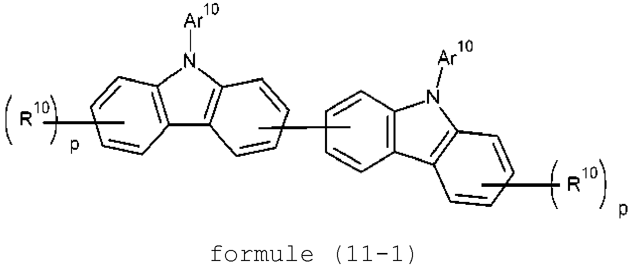 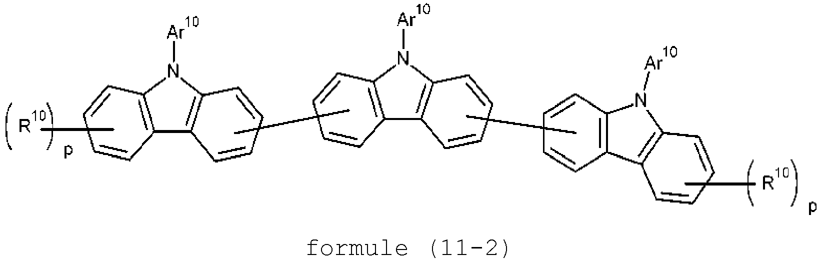 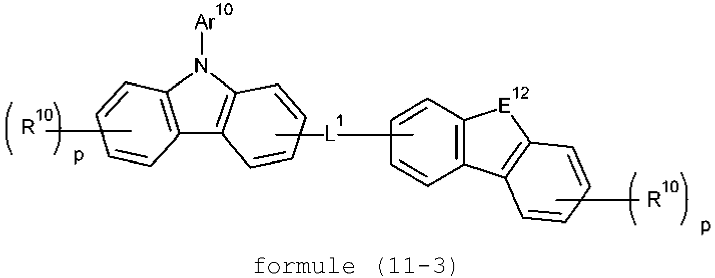 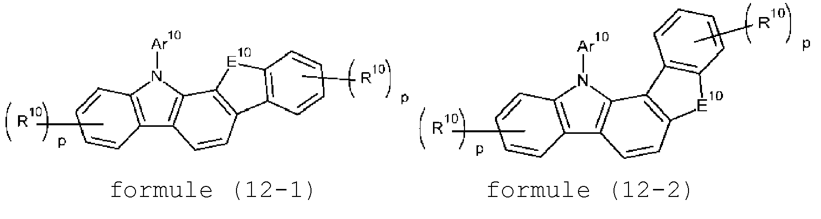 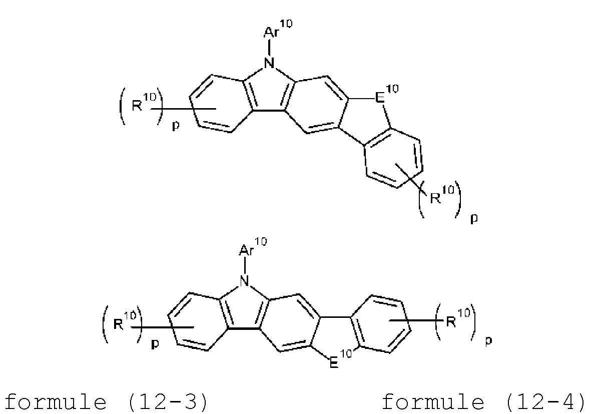
où les symboles et indices Ar¹⁰, R¹⁰, E¹⁰, L¹ et p ont la même signification que dans la revendication 1 ; et
E¹² désigne -C(R⁰)₂-, -O-, -S-, -N(Ar¹⁰)- ; où R⁰ a la même signification que dans la revendication 1.

12. Dispositif électronique organique selon une ou plusieurs des revendications précédentes, **caractérisé en ce que** le deuxième composant est choisi parmi les composés de formules (13-1) à (14-4),zzz 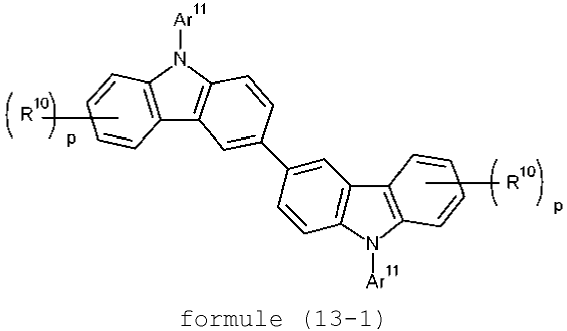 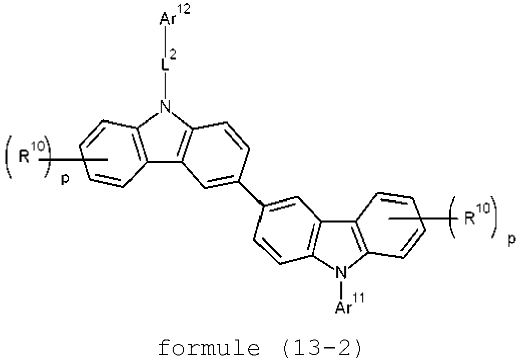 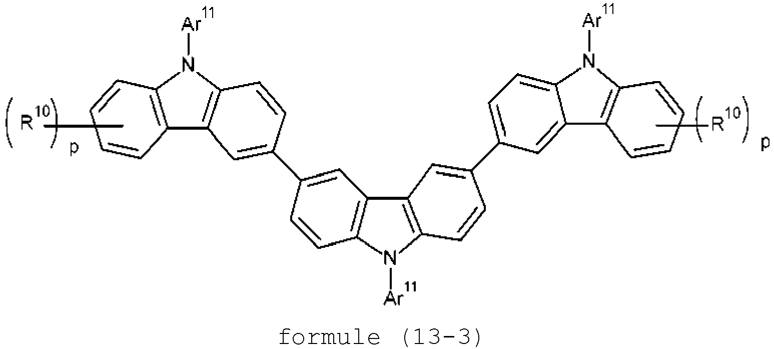 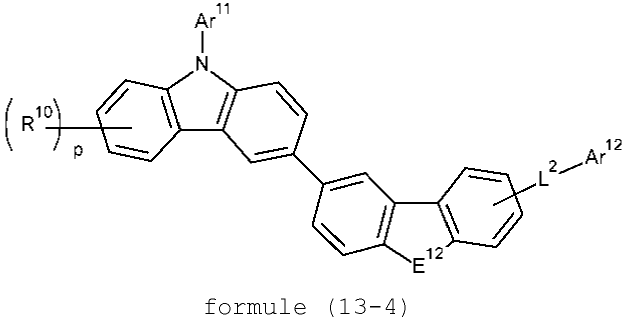 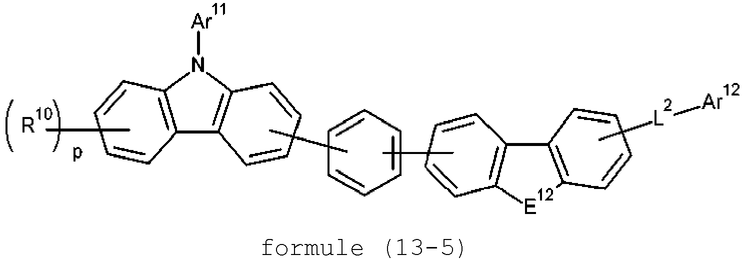 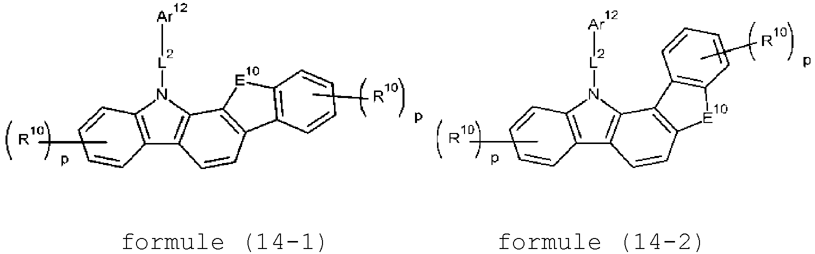 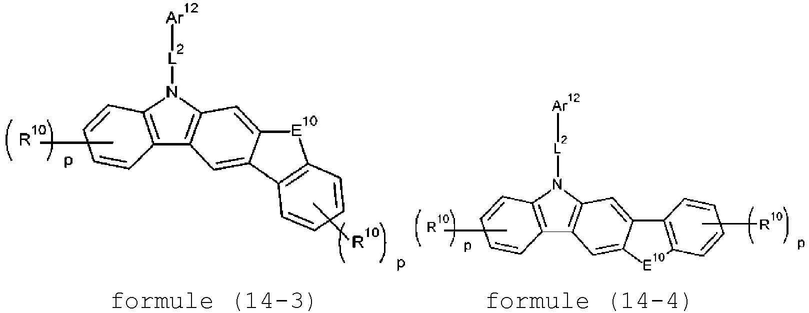
où les symboles et indices R¹⁰, E¹⁰ et p ont la même signification que dans la revendication 11, le symbole E¹² a la même signification que dans la revendication 13 ; et
Ar¹⁰ désigne un système cyclique aromatique ou hétéroaromatique choisi parmi benzène, naphtalène, pyrène, biphényle, terphényle, quaterphényle, fluorène, spirobifluorène, cis- ou trans-indénofluorène, dibenzofurane, dibenzothiophène, carbazole, indolocarbazole, indénocarbazole, qui peut être substitué par un ou plusieurs radicaux R ; ou des combinaisons de ces groupes ;
L² désigne une simple liaison ou un système cyclique aromatique ou hétéroaromatique ayant 6 à 18 atomes de cycle aromatique, qui peut être substitué par un ou plusieurs radicaux R ;
Ar¹² désigne un système cyclique hétéroaromatique choisi parmi pyridine, pyrazine, pyrimidine, triazine, qui peut être substitué par un ou plusieurs radicaux R ;
R a la même signification que dans la revendication 1.
